# EUROPÄISCHE PATENTANMELDUNG

(11) **EP 4 198 114 A1**
(43) Veröffentlichungstag der Anmeldung: **21.06.2023**
(21) Anmeldenummer: 22213334.0
(22) Anmeldetag: 14.12.2022
(51) Int. Cl.: C11D 1/74, C11D 3/00, C11D 17/00, A61K 8/11, C11D 3/50

(54) **MITTEL ENTHALTEND EMULGATOR UND MIKROKAPSELN**

(30) Priorität: 15.12.2021 EP 21214656; 15.12.2021 EP 21214696
(71) Anmelder: Henkel AG & Co. KGaA, 40589 Düsseldorf (DE)
(72) Erfinder: Bauer, Andreas, 41564 Kaarst (DE); Schiedel, Marc-Steffen, 40789 Monheim (DE); Urlichs, Stefan, 45307 Essen (DE); Biedenbach, Michael, 77704 Oberkirch (DE); Kind, Christian, 76532 Baden-Baden (DE); Gerigk, Andreas, 41812 Erkelenz (DE); Materne, Manuela, 41564 Kaarst (DE); Smyrek, Hubert, 47804 Krefeld (DE); Giesen, Christian, 41334 Nettetal (DE)

(57) **Zusammenfassung**

Die Erfindung betrifft gemäß einem ersten Aspekt ein Mittel ausgewählt aus Wasch- und Reinigungsmitteln sowie kosmetischen Mitteln, enthaltend einen Emulgator wie hierin definiert sowie bioabbaubare Mikrokapseln umfassend ein Kernmaterial und eine Schale, wobei die Schale aus mindestens einer Barriereschicht und einer Stabilitätsschicht besteht, wobei die Barriereschicht das Kernmaterial umgibt, wobei die Stabilitätsschicht mindestens ein Biopolymer umfasst, und auf der äußeren Oberfläche der Barriereschicht angeordnet ist. Ferner betrifft die Erfindung die Verwendung dieser Mittel.

## Beschreibung

### Gebiet der Erfindung

Die Erfindung betrifft Wasch- und Reinigungsmittel sowie Kosmetika, welche bioabbaubare Mikrokapseln mit umweltverträglichen Wandmaterialien sowie spezielle Emulgatoren enthalten.

### Hintergrund der Erfindung

Die Mikroverkapselung ist eine vielseitig nutzbare Technologie. Sie bietet Lösungen für zahlreiche Innovationen - von der Papierindustrie bis hin zu Haushaltsprodukten erhöht die Mikroverkapselung die Funktionalität unterschiedlichster aktiver Substanzen. Verkapselte Aktivstoffe können wirtschaftlicher eingesetzt werden und verbessern die Nachhaltigkeit und Umweltverträglichkeit vieler Produkte.

Allerdings sind die polymeren Wandmaterialien der Mikrokapseln selbst in sehr unterschiedlichem Grad umweltverträglich. In Selbstdurchschreibepapier werden schon lange auf dem Naturprodukt Gelatine basierende und somit vollständig bioabbaubare Mikrokapselwände eingesetzt. Ein schon in den 1950er Jahren entwickeltes Verfahren zur Gelatineverkapselung ist in US 2,800,457 offenbart. Seitdem wurde eine Vielzahl an Variationen in Bezug auf Materialien und Verfahrensschritte beschrieben. Außerdem werden bioabbaubare bzw. enzymatisch abbaubare Mikrokapselwände eingesetzt, um den enzymatischen Abbau als Verfahren zur Freisetzung des Kernmaterials zu verwenden. Solche Mikrokapseln sind beispielsweise in WO 2009/126742 A1 oder WO 2015/014628 A1 beschrieben.

Solche Mikrokapseln sind jedoch für viele industrielle Anwendungen und Haushaltsprodukte nicht geeignet. Denn Naturstoff-basierte Mikrokapseln erfüllen die für z.B. Wasch- und Reinigungsmittel, Klebstoffsystemen, Lacke und Dispersionen geforderte Diffusionsdichtigkeit, die chemische Resistenz und die Temperaturbeständigkeit und auch die geforderte Beladung mit Kernmaterial nicht.

In diesen sogenannten Hochanforderungsgebieten werden klassisch organische Polymere wie Melamin-Formaldehyd-Polymere (siehe z.B. EP 2 689 835 A1, WO 2018/114056 A1, WO 2014/016395 A1, WO 2011/075425 A1 oder WO 2011/120772 A1); Polyacrylate (siehe z.B. WO 2014/032920 A1, WO 2010/79466 A2); Polyamide; Polyurethan oder Polyharnstoffe (siehe z.B. WO 2014/036082 A2 oder WO 2017/143174 A1) eingesetzt. Die aus solchen organischen Polymeren aufgebauten Kapseln verfügen über die benötigte Diffusionsdichtigkeit, Stabilität und chemische Resistenz. Diese organischen Polymere sind allerdings nur in sehr geringem Maße enzymatisch bzw. biologisch abbaubar.

Im Stand der Technik sind verschiedene Ansätze beschrieben, bei denen Biopolymere als zusätzliche Komponente mit den organischen Polymeren der Mikrokapselschale zur Anwendung in Hochanforderungsgebieten kombiniert werden, allerdings nicht mit der Zielsetzung, bioabbaubare Mikrokapseln zu erzeugen, sondern vorrangig die Freisetzungs-, Stabilitäts-, oder Oberflächeneigenschaften der Mikrokapseln zu verändern. Beispielsweise wird in WO 2014/044840 A1 ein Verfahren zur Herstellung von zweischichtigen Mikrokapseln beschrieben mit einer inneren Polyharnstoff-Schicht und einer äußeren Gelatine-enthaltenden Schicht. Dabei wird die Polyharnstoff-Schicht durch Polyaddition an der Innenseite der durch Koazervation erhaltenden Gelatine-Schicht erzeugt. Die so erhaltenen Kapseln weisen gemäß Beschreibung aufgrund der Polyharnstoffschicht die nötige Stabilität und Dichtigkeit für den Einsatz in Wasch- und Reinigungsmitteln auf und zusätzlich durch die Gelatine eine Klebrigkeit, um sie an Oberflächen anzuheften. Konkrete Stabilitäten und Resistenzen werden nicht erwähnt. Nachteilig an Polyharnstoffkapseln ist jedoch die unvermeidliche Nebenreaktion der Kernmaterialien mit den zur Erzeugung des Harnstoffs verwendeten Diisocyanaten, die dem ölbasierten Kern beigemischt werden müssen.

Andererseits sind im Stand der Technik auch auf Biopolymeren basierende Mikrokapseln beschrieben, die durch Hinzufügung einer Schutzschicht eine verbesserte Dichtigkeit oder Stabilität gegenüber Umwelteinflüssen oder eine gezielte Einstellung eines verzögerten Freisetzungsverhaltens erreichen. Beispielsweise beschreibt die WO 2010/003762 A1 Partikel mit einem Kern-Schale-Schale Aufbau. Im Inneren eines jeden Partikels befindet sich als Kern ein schwer wasserlöslicher oder wasserunlöslicher organischer Wirkstoff. Die den Kern direkt umhüllende Schale enthält ein biologisch abbaubares Polymer und die äußere Schale mindestens ein Metall- oder Halbmetalloxid. Mit diesem Aufbau wird zwar eine bioabbaubare Schale erhalten. Die Mikrokapseln werden dennoch gemäß WO 2010/003762 A1 in Lebensmitteln, Kosmetika oder pharmazeutischen Mitteln eingesetzt, sind für die erfindungsgemäßen Hochanforderungsgebiete jedoch aufgrund mangelnder Dichtheit nicht verwendbar.

In der nicht veröffentlichten PCT/EP2020/085804 werden Mikrokapseln mit einem Mehrschichtaufbau der Schalen beschrieben, die im Wesentlichen bioabbaubar sind und dennoch ausreichende Stabilität und Dichtigkeit aufweisen, um in Hochanforderungsgebieten eingesetzt werden zu können. Dies wird dadurch erreicht, dass eine Stabilitätsschicht den Hauptanteil der Kapselschale ausmacht, die aus natürlich vorkommenden und gut bioabbaubaren Materialien, insbesondere wie Gelatine oder Alginat bzw. aus ubiquitär in der Natur vorhandenen Materialien besteht.

Diese Stabilitätsschicht wird mit einer Barriereschicht kombiniert, die aus bekannten für Mikroverkapselung eingesetzten Materialien, wie Melamin-Formaldehyd oder Meth(acrylat) bestehen kann. Dabei ist es gelungen, die Barriereschicht in einer bisher nicht darstellbaren geringen Wandstärke auszugestalten und dennoch eine ausreichende Dichtigkeit zu gewährleisten. Damit wird der Anteil der Barriereschicht an der Gesamtwand sehr geringgehalten, so dass die Mikrokapselwand eine Bioabbaubarkeit gemessen nach OECD 301 F von mindestens 40 % aufweist.

Derartige Mikrokapseln werden typischerweise in Form von wässrigen Dispersionen eingesetzt, auch als Aufschlämmungen oder Slurries bezeichnet, in welchen die Mikrokapseln als feste Phase in einem überwiegend wässrigen Medium als kontinuierlicher Phase dispergiert sind. Es ist wünschenswert, dass derartige Dispersionen eine ausreichende Phasenstabilität aufweisen, um auch nach längeren Lager- oder Transportzeiten ein stabiles Produkt ohne unerwünschte Sedimente oder Aufrahmungen bereitzustellen. Dazu sind oftmals in die kontinuierliche Phase verschiedene Additive oder Hilfsstoffe eingearbeitet, die diese Stabilität gewährleisten sollen. Diese müssen aber oftmals in Abhängigkeit der eingesetzten Kapseln speziell ausgewählt werden, da eine generelle Eignung typischerweise nicht gegeben ist. Die vorliegende Erfindung betrifft Mittel, in denen ein spezieller Emulgator eingesetzt wird, der für die beschriebenen Mikrokapseln die gewünschte Phasenstabilität in der Mikrokapsel-Dispersion und im die Mikrokapseln enthaltenen Endprodukt liefert.

### Zusammenfassung der Erfindung

Die Erfindung betrifft gemäß einem ersten Aspekt Mittel ausgewählt aus Wasch- und Reinigungsmitteln sowie kosmetischen Zubereitungen, die
(1) bioabbaubare Mikrokapseln, umfassend ein Kernmaterial und eine Schale, enthalten, wobei die Schale aus mindestens einer Barriereschicht und einer Stabilitätsschicht besteht, wobei die Barriereschicht das Kernmaterial umgibt, wobei die Stabilitätsschicht mindestens ein Biopolymer umfasst, und auf der äußeren Oberfläche der Barriereschicht angeordnet ist, und wobei am Übergang von Barriereschicht zu Stabilitätsschicht optional ein Emulsionsstabilisator angeordnet ist und
(2) mindestens einen Emulgator, wobei der Emulgator ausgewählt wird aus der Gruppe der ethoxylierten, hydrierten Rizinusöle, insbesondere solchen mit mittleren EO-Werten im Bereich von 20 bis 60, vorzugsweise 30 bis 50.

In bevorzugten Ausführungsformen ist der mindestens eine Emulgator, bezogen auf das Gesamtgewicht des Mittels, in 0,001 bis 0,25 Gew.-%, vorzugsweise 0,001 bis 0,15 Gew.-%, noch bevorzugter 0,001 bis 0,08 Gew.-% enthalten, wobei der Emulgator bevorzugt in vorformulierter Form mit den bioabbaubaren Mikrokapseln eingesetzt wird.

In verschiedenen Ausführungsformen der Erfindung wird der Emulgator als Bestandteil einer Mikrokapsel-Dispersion (Slurry) eingesetzt, wobei die Dispersion die Mikrokapseln als feste Phase und Wasser als Hauptbestandteil der kontinuierlichen Phase umfasst. In derartigen Ausführungsformen ist der Emulgator Teil der kontinuierlichen Phase.

In einer bevorzugten Ausführungsform enthält die Mikrokapsel-Dispersion den mindestens einen Emulgator in einer Menge von bis zu 50 Gew.-%, vorzugsweise bis zu 40 Gew.-%, bevorzugter bis zu 30 Gew.-% oder bis zu 20 Gew.-%, noch bevorzugter bis maximal 10 Gew.-%, besonders bevorzugt in einer Menge von 2 bis 10 Gew.-%.

Bevorzugt umfasst der Anteil des Emulgators in der Mikrokapsel-Dispersion 0,5 Gew.-% bis 50 Gew.-%, bevorzugt 1,0 Gew.-% bis 30 Gew.-%, noch bevorzugter 2 Gew.-% bis 20 Gew.-%, besonders bevorzugt 4 Gew.-% bis 8 Gew.-% beträgt, bezogen auf das Gesamtgewicht der Mikrokapsel-Dispersion.

In bevorzugten Ausführungsformen ist der Anteil der Mikrokapsel-Dispersion in dem Mittel mindestens 0,1 Gew.-%, bevorzugt mindestens 0,5 Gew.-%, bezogen auf das Gesamtgewicht des Mittels, und vorzugsweise ist der Anteil des Emulgators im Mittel, bezogen auf das Gesamtgewicht des Mittels, 0,001 bis 0,25 Gew.-%, vorzugsweise 0,001 bis 0,15 Gew.-%, noch bevorzugter 0,001 bis 0,08 Gew.-%, wobei das Mittel bevorzugt flüssig ist.

Ferner ist es möglich, die Mikrokapsel-Dispersion zu trocknen, wobei die getrocknete Mikrokapsel-Dispersion weniger als 5 Gew.-%, bevorzugt weniger als 1 Gew.-% Wasser enthalten kann und besonders bevorzugt bis auf unvermeidliche Spuren kein Wasser enthält.

In verschiedenen Ausführungsformen unterscheiden sich Barriereschicht und Stabilitätsschicht in ihrer chemischen Zusammensetzung bzw. ihrem chemischen Aufbau. Das Kernmaterial umfasst vorzugsweise mindestens einen Duftstoff und kann beispielsweise eine Parfümölzusammensetzung sein.

Wenn es sich bei dem Mittel um ein Wasch- oder Reinigungsmittel handelt, enthält dieses vorzugsweise mindestens einen weiteren Bestandteil ausgewählt aus Tensiden, Gerüststoffen, Enzymen und aufziehverstärkenden Mitteln. Wenn es sich bei dem Mittel um ein kosmetisches Mittel handelt, kann dieses ebenfalls mindestens einen weiteren Bestandteil enthalten, der beispielsweise aus Tensiden und hautpflegenden Stoffen ausgewählt sein kann.

Bei dem Emulsionsstabilisator handelt es sich um ein Polymer oder Copolymer, das aus bestimmten Acrylsäure-Derivaten, N-Vinylpyrrolidon, und/oder Styrol aufgebaut ist. In verschiedenen Ausführungsformen besteht das Polymer oder Copolymer aus einem oder mehreren Monomeren, die ausgewählt sind aus:
(1) Acrylsäure-Derivaten der allgemeinen Formel (I) 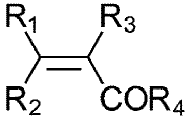 wobei
   R₁, R₂ und R₃ ausgewählt sind aus: Wasserstoff und einer Alkylgruppe mit 1 bis 4 Kohlenstoffatomen, wobei R₁ und R₂ insbesondere Wasserstoff und R₃ insbesondere Wasserstoff oder Methyl ist; und R₄ für -OX oder -NR₅R₆ steht, wobei X für Wasserstoff, ein Alkalimetall eine Ammonium-Gruppe oder ein gegebenenfalls durch -SO₃M oder -OH substituiertes C₁-C₁₈ Alkyl steht, wobei M für Wasserstoff, ein Alkalimetall oder Ammonium steht, wobei das gegebenenfalls durch -SO₃M oder -OH substituierte C₁-C₁₈ Alkyl vorzugsweise Methyl, Ethyl, n-Butyl, 2-Ethylhexyl, 2-Sulfoethyl oder 2-Sulfopropyl ist, wobei R₅ und R₆ unabhängig voneinander für Wasserstoff oder ein gegebenenfalls durch -SO₃M substituiertes C₁-C₁₀ Alkyl stehen, wobei mindestens eines von R₅ und R₆ nicht Wasserstoff ist, wobei vorzugsweise R₅ H ist und R₆ 2-Methyl-propan-2-yl-1-sulfonsäure;
(2) N-Vinylpyrrolidon; und
(3) Styrol.

Der Emulsionsstabilisator ist bevorzugt ein Acrylat-Copolymer enthaltend 2-Acrylamido-2-methyl-propansulfonsäure (AMPS). Ein geeignetes Copolymer ist beispielsweise unter dem Handelsnamen Dimension PA 140 erhältlich.

In verschiedenen Ausführungsformen ist die Barriereschicht aus einer oder mehreren Komponenten, ausgewählt aus der Gruppe bestehend aus einer aldehydischen Komponente, einem aromatischen Alkohol, einer Aminkomponente, einer Acrylat-Komponente und einer Isocyanat-Komponente aufgebaut, und die Stabilitätsschicht umfasst mindestens ein Biopolymer.

Vorteilhaft ist des Weiteren, dass die verbesserte strukturelle Aufnahme der Stabilitätgeberschicht durch die Barriereschicht durch Zugabe des Emulsionsstabilisators die strukturelle (kovalente) Verbindung aller wandbildenden Komponenten gewährleistet, so dass die einzelnen Schichten untrennbar verbunden und als Monopolymer angesehen werden können.

Aufgrund der Robustheit bzw. Dichtigkeit der bioabbaubaren Kapsel können diese in einer Vielzahl von Produkten aus dem Bereich der Wasch- und Reinigungsmittel sowie Kosmetika eingesetzt werden.

In bevorzugten Ausführungsformen weist das Mittel einen pH-Wert von weniger als 11, vorzugsweise weniger als 10, bevorzugter weniger als 9, noch bevorzugter weniger als 5 und besonders bevorzugt weniger als 4 und/oder eine Leitfähigkeit von mindestens 0,1 mS/cm, vorzugsweise mindestens 0,2 mS/cm, stärker bevorzugt mindestens 0,3 mS/cm, noch stärker bevorzugt mindestens 1,0 mS/cm, noch stärker bevorzugt mindestens 2,5 mS/cm und besonderes bevorzugt mindestens 5,0 mS/cm, und/oder eine Leitfähigkeit von höchstens 100 mS/cm, vorzugsweise bis zu 60 mS/cm, besonders bevorzugt bis zu 34 mS/cm auf.

Ferner betrifft die Erfindung in einem weiteren Aspekt die Verwendung von Wasch- und Reinigungsmitteln gemäß dem ersten Aspekt in einem Verfahren zur Konditionierung von Textilien oder zur Reinigung von Textilien und/oder harten Oberflächen.

Ferner betrifft die Erfindung in einem weiteren Aspekt die kosmetische Verwendung von Mitteln gemäß dem ersten Aspekt.

### Detaillierte Beschreibung der Erfindung

### Definitionen

"Barriereschicht" bezeichnet die Schicht einer Mikrokapselwand, die im Wesentlichen für die Dichtigkeit der Kapselschale verantwortlich ist, d. h. Austritt des Kernmaterials verhindert.

"Biologische Abbaubarkeit" bezeichnet das Vermögen organischer Chemikalien, biologisch, also durch Lebewesen oder deren Enzyme, zersetzt zu werden. Im Idealfall verläuft dieser chemische Metabolismus vollständig bis zur Mineralisierung, kann aber auch bei abbaustabilen Transformationsprodukten stehen bleiben. Allgemein anerkannt sind die Richtlinien zur Prüfung von Chemikalien der OECD, die auch im Rahmen der Chemikalienzulassung verwendet werden. Die Tests der OECD-Testserie 301 (A-F) weisen einen raschen und vollständigen biologischen Abbau (ready biodegradability) unter aeroben Bedingungen nach. Unterschiedliche Testmethoden stehen für gut oder schlecht lösliche sowie für flüchtige Substanzen zur Verfügung. Insbesondere wird im Rahmen der Anmeldung der manometrische Respirationstest (OECD 301 F) verwendet. Die grundsätzliche biologische Abbaubarkeit (inherent biodegradability) kann über die Messnorm OECD 302 bestimmt werden, beispielsweise den MITI-II-Test (OECD 302 C).

Als "bioabbaubar" oder "biologisch abbaubar" im Sinne der vorliegenden Erfindung werden Mikrokapselwände bezeichnet, die eine Bioabbaubarkeit gemessen nach OECD 301 F von mindestens 40 % innerhalb von 60 Tagen aufweisen. Ab einem Grenzwert von mindestens 60 % Abbau innerhalb von 60 Tagen gemessen nach OECD 301 F werden Mikrokapselwände vorliegend auch als rasch bioabbaubar bezeichnet.

Ein "Biopolymer" ist ein natürlich vorkommendes Polymer, beispielsweise ein in einer Pflanze, einem Pilz, einem Bakterium oder einem Tier vorkommendes Polymer. Zu den Biopolymeren zählen auch auf natürlich vorkommenden Polymeren basierende modifizierte Polymere. Das Biopolymer kann aus der natürlichen Quelle gewonnen oder künstlich hergestellt worden sein.

"Dichtheit" gegenüber einem Stoff, Gas, einer Flüssigkeit, Strahlung o. Ä., ist eine Eigenschaft von Materialstrukturen. Die Begriffe "Dichtheit" und "Dichtigkeit" werden erfindungsgemäß synonym verwendet. Dichtheit ist ein relativer Begriff und bezieht sich immer auf vorgegebene Rahmenbedingungen.

"Emulsionsstabilisator" sind Hilfsstoffe zur Stabilisierung von Emulsionen. Die Emulsionsstabilisatoren können in geringer Menge der wässrigen oder öligen Phase (von Emulsionen) zugesetzt werden, wobei sie in der Grenzfläche phasenorientiert angereichert werden und dabei zum einen die Zerteilung der inneren Phase durch Herabsetzen der Grenzflächenspannung erleichtern und zum anderen die Zerteilungsbeständigkeit der Emulsion erhöhen.

Der Begriff "(Meth)Acrylat" bezeichnet in dieser Erfindung sowohl Methacrylate wie Acrylate.

Unter dem Begriff "Mikrokapseln" werden erfindungsgemäß Partikel verstanden, die einen inneren Raum oder Kern enthalten, der mit einem festen, gelierten, flüssigen oder gasförmigen Medium gefüllt ist und von einer kontinuierlichen Hülle (Schale) aus filmbildenden Polymeren umschlossen (verkapselt) ist. Diese Teilchen besitzen vorzugsweise kleine Abmessungen. Die Begriffe "Mikrokapseln", "Kern-Schale-Kapseln" oder einfach "Kapseln" werden synonym verwendet.

Als "Mikroverkapselung" wird ein Herstellungsverfahren bezeichnet, bei dem kleine und kleinste Portionen fester, flüssiger oder gasförmiger Substanzen mit einer Hülle aus polymeren oder anorganischen Wandmaterialien umgeben werden. Die so erhaltenen Mikrokapseln können einen Durchmesser von einigen Millimetern bis unter 1 µm haben.

Die Mikrokapsel weist eine mehrschichtige "Schale" auf. Die das Kernmaterial der Mikrokapsel umhüllende Schale wird regelmäßig auch als "Wand" oder "Hülle" bezeichnet.

Die Mikrokapseln mit einer mehrschichtigen Schale können auch als mehrschalige Mikrokapseln bzw. mehrschaliges Mikrokapseln-System bezeichnet werden, da die einzelnen Schichten auch als individuelle Schalen angesehen werden können. "Mehrschichtig" und "mehrschalig" werden somit synonym verwendet.

"Stabilitätsschicht" bezeichnet die Schicht einer Kapselwand, die im Wesentlichen für die Stabilität der Kapselschale verantwortlich ist, d. h. in der Regel den Hauptteil der Schale ausmacht.

"Wandbildner" sind die Komponenten, die die Mikrokapselwand aufbauen.

"Hydriertes Rizinusöl" bezeichnet teilweise oder vollständig hydriertes Rizinusöl (engl. castor oil, hydrogenated). Rizinusöl (CAS-Nr. 8001-79-4) ist ein bekanntes Pflanzenöl, welches zu 80-85% aus dem Triglycerid der Ricinolsäure besteht (Triricinolein). Weitere Bestandteile sind Glyceride verschiedener anderer Fettsäuren sowie ein geringer Anteil von freien Fettsäuren. Durch die Hydrierung wird das Triricinolein in das Triglycerid von 12-Hydroxystearinsäure umgewandelt. Erfindungsgemäß eingesetzt werden ethoxylierte, hydrierte Rizinusöle, die üblicherweise durch die Umsetzung von hydriertem Rizinusöl mit Ethylenoxid erhältlich sind. Die so erhaltenen und erfindungsgemäß eingesetzten Verbindungen enthalten im Mittel 20-60 Ethylen-Einheiten, besonders bevorzugt sind 30 bis 50 EO, besonders bevorzugt 40 EO. PEG-40 hydrogenated Castor Oil (INCI) ist beispielsweise kommerziell als Eumulgin^{®} HRE 40 von BASF erhältlich. Derartige Verbindung sind als nichtionische Öl-in-Wasser Emulgatoren geeignet und werden als solche angeboten und eingesetzt.

### Mikrokapseln

Die bioabbaubaren Mikrokapseln, die gemäß dem ersten Aspekt der Erfindung in Wasch- und Reinigungsmitteln sowie kosmetischen Mitteln, eingesetzt werden, umfassen ein Kernmaterial und eine Schale, wobei die Schale aus mindestens einer Barriereschicht und einer Stabilitätsschicht besteht, wobei die Barriereschicht das Kernmaterial umgibt, wobei die Stabilitätsschicht mindestens ein Biopolymer umfasst, und auf der äußeren Oberfläche der Barriereschicht angeordnet ist, und wobei am Übergang von Barriereschicht zu Stabilitätsschicht vorzugsweise ein Emulsionsstabilisator angeordnet ist. Diese Anordnung kann in einer Zwischenschicht aus Emulsionsstabilisator bestehen, die kontinuierlich oder diskontinuierlich sein und Teile oder die gesamte innere Barriereschicht bedecken kann. Alternativ können auch nur einzelne Moleküle des Emulsionsstabilisators auf der Oberfläche der Barriereschicht derart angeordnet sein, dass sie eine Bindung zwischen Stabilitätsschicht und Barriereschicht vermitteln. Der Emulsionsstabilisator wirkt hier als Vermittleragens.

Die Mikrokapselschalen weisen aufgrund der Anwendung des Emulsionsstabilisators eine deutlich erhöhte Dicke der Stabilitätsschicht auf. Dadurch ist der Anteil an natürlichen Komponenten in der Kapsel weiter erhöht gegenüber zuvor beschriebenen Mehrschichtmikrokapseln.

Gemäß einer Ausführungsform der bioabbaubaren Mikrokapseln wird bei Herstellung der Mikrokapseln, die Oberfläche der Barriereschicht vor Ausbildung der Stabilitätsschicht mit dem Emulsionsstabilisator in Kontakt gebracht. Dadurch ist die Kapazität der Oberfläche zur strukturellen Anbindung der Stabilitätsschicht erhöht. Ohne darauf festgelegt werden zu wollen, gehen die Erfinder davon aus, dass sich der Emulsionsstabilisator an der unpolaren Oberfläche der Barriereschicht, insbesondere einer Melamin-Formaldehyd-Schicht, anlagert und somit den Biopolymeren der Stabilitätsschicht ein Gerüst für die Abscheidung auf der Oberfläche bietet. Dadurch wird nicht nur die mittlere Schichtdicke der mit dem Biopolymer erzeugten Stabilitätsschicht erhöht, sondern zusätzlich der Emulsionsstabilisator an der Grenzfläche zwischen Stabilitätsschicht und Barriereschicht eingebaut. Ausgehend von dieser Theorie kommt grundsätzlich jeder Emulsionsstabilisator als Vermittleragens zur Herstellung der Mikrokapseln in Frage.

In einer bevorzugten Ausführungsform ist der Emulsionsstabilisator ein Polymer oder Copolymer, bestehend aus einem oder mehreren Monomeren ausgewählt aus:
(1) Acrylsäure-Derivaten der allgemeinen Formel (I) 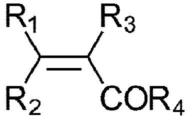 wobei
   R₁, R₂ und R₃ ausgewählt sind aus: Wasserstoff und einer Alkylgruppe mit 1 bis 4 Kohlenstoffatomen, wobei R₁ und R₂ insbesondere Wasserstoff und R₃ insbesondere Wasserstoff oder Methyl ist; und
   R₄ für -OX oder -NR₅R₆ steht, wobei X für Wasserstoff, ein Alkalimetall, eine Ammonium-Gruppe oder ein gegebenenfalls durch -SO₃M oder -OH substituiertes C₁-C₁₈ Alkyl steht, wobei M für Wasserstoff, ein Alkalimetall oder Ammonium steht, wobei das gegebenenfalls durch -SO₃M oder -OH substituierte C₁-C₁₈ Alkyl vorzugsweise Methyl, Ethyl, n-Butyl, 2-Ethylhexyl, 2-Sulfoethyl oder 2-Sulfopropyl ist, wobei R₅ und R₆ unabhängig voneinander für Wasserstoff oder ein gegebenenfalls durch -SO₃M substituiertes C₁-C₁₀ Alkyl stehen, wobei mindestens eines von R₅ und R₆ nicht Wasserstoff ist, wobei vorzugsweise R₅ H ist und R₆ 2-Methyl-propan-2-yl-1-sulfonsäure;
(2) N-Vinylpyrrolidon; und
(3) Styrol.

Die für R₁, R₂ und R₃ möglichen C₁₋₄-Hydroxyalkylgruppen können Ethyl-, n-Propyl-, i-Propyl und n-Butyl sein. In einigen Ausführungsformen der Acrylsäure-Derivate sind R₁ und R₂ Wasserstoff und R₃ Wasserstoff oder Methyl. Je nach Auswahl von R₃ handelt es sich um ein Acrylat (Wasserstoff) oder Methacrylat (Methyl).

Die für X möglichen gegebenenfalls durch -OH oder -SO₃M substituierten C₁-C₁₈ Alkylgruppen, sind vorzugsweise ausgewählt aus Methyl-, Ethyl-, C₂₋₄-Hydroxyalkyl-, C₂₋₄-Sulfoalkyl- und C₄-C₁₈ Alkylgruppen.

Die C₂₋₄-Hydroxyalkylgruppen können ausgewählt sein aus Ethyl-, n-Propyl-, i-Propyl und n-Butyl. Als unsubstituierte C₄₋₁₈-Alkylgruppen sind beispielsweise die n-Butyl-, sec.-Butyl-, tert.-Butyl-, Pentyl-, Hexyl-, Ethylhexyl-, Octyl-, Decyl-, Dodecyl- oder Stearyl-Gruppen zu nennen. Insbesondere geeignet sind die n-Butyl- und Ethylhexyl. Das Ethylhexyl ist insbesondere 2-Ethylhexyl. Als C₂₋₄-Sulfoalkylgruppen sind insbesondere 2-Sulfoethyl und 3-Sulfopropyl zu nennen.

Gemäß einer Ausführungsform der Acrylsäure-Derivate ist R₄ -NR₅R₆, wobei R₅ H ist und R₆ 2-Methyl-propan-2-yl-1-sulfonsäure. Dabei sind insbesondere R₁, R₂ und R₃ Wasserstoff.

Gemäß einer Ausführungsform ist R₄ -OX und X ist Wasserstoff. Dabei sind insbesondere R₁, R₂ und R₃ Wasserstoff (Acrylsäure). Alternativ ist dabei R₃ Methyl (Methacrylat). Gemäß einer Ausführungsform der Acrylsäure-Derivate ist R₄ -OX und X ist Methyl. Dabei sind insbesondere R₁, R₂ und R₃ Wasserstoff (Methylacrylat). Gemäß einer Ausführungsform ist R₄ -OX und X ist 2-Ethylhexyl. Dabei sind insbesondere R₁, R₂ und R₃ Wasserstoff (Ethylhexacrylat). Gemäß einer Ausführungsform ist R₄ -OX und X ist n-Butyl. Dabei sind insbesondere R₁, R₂ und R₃ Wasserstoff (n-Butylacrylat). Gemäß einer Ausführungsform der Acrylsäure-Derivate ist R₄ -OX und X ist 2-Sulfoethyl. Dabei sind insbesondere R₁, R₂ und R₃ Wasserstoff (Sulfoethylacrylat). Gemäß einer Ausführungsform der Acrylsäure-Derivate ist R₄ -OX und X ist 3-Sulfopropyl. Dabei sind insbesondere R₁, und R₂ Wasserstoff und R₃ ist Methyl (Sulfopropyl(meth)acrylat).

Die Polymere oder Copolymere, die aus Monomeren der Formel (I) aufgebaut sind, genügen typischerweise der Formel (II): 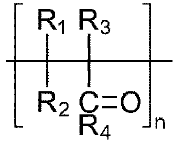 wobei R¹-R⁴ die oben angegebenen Bedeutungen haben und n eine ganze Zahl von mindestens 3 ist. n kann beispielsweise größer als 5, 10, 20, 30, 40, 50, 60, 70, 80, oder 100 sein. n kann beispielsweise kleiner als 10.000, 7.500, 5.000, 2.500, 1000, oder 500 sein. Gemäß einer Ausführungsform liegt n im Bereich von 5 bis 5000. In einer Ausführungsform liegt n im Bereich von 10 bis 1000

Die Gruppe dieser Polymere und Copolymere stellt eine sinnvolle Verallgemeinerung der in dem kommerziell erhältlichen Emulsionsstabilisator "Dimension PA 140" vorhandenen Copolymere dar.

Der Emulsionsstabilisator ist bevorzugt ein Acrylat-Copolymer, welches mindestens zwei unterschiedliche Monomere der Formel (I) enthält. In verschiedenen Ausführungsformen enthält das Copolymer AMPS, optional in Kombination mit (Meth)Acrylsäure und/oder mindestens einem Alkyl(meth)acrylat. Gemäß einer Ausführungsform enthält das Copolymer AMPS und ein oder mehrere Monomere ausgewählt aus Acrylat, Methacrylat, Methylacrylat, Ethylhexacrylat, n-Butylacrylat, N-Vinylpyrrolidon und Styrol.

Gemäß einer Ausführungsform enthält das Copolymer AMPS, Acrylat, Methylacrylat, und Styrol. Gemäß einer Ausführungsform enthält das Copolymer AMPS, Acrylat, Methylacrylat, und Ethylhexacrylat. Gemäß einer Ausführungsform enthält das Copolymer AMPS, Methylacrylat, N-Vinylpyrrolidon und Styrol. Gemäß einer Ausführungsform enthält das Copolymer AMPS, Acrylat, Methylacrylat, und Ethylhexacrylat. Gemäß einer Ausführungsform enthält das Copolymer AMPS, Methylacrylat, N-Vinylpyrrolidon und Styrol. Gemäß einer Ausführungsform enthält das Copolymer AMPS, Methylacrylat und Styrol. Gemäß einer Ausführungsform enthält das Copolymer AMPS, Methacrylat und Styrol. Gemäß einer Ausführungsform enthält das Copolymer AMPS, Acrylat, Methylacrylat, und n-Butylacrylat.

Gemäß einer Ausführungsform ist der Emulsionsstabilisator ein Copolymer wie in EP0562344B1 definiert, das durch Verweis hierin einbezogen ist. Gemäß einer Ausführungsform ist der Emulsionsstabilisator ein Copolymer, enthaltend a) AMPS, Sulfoethyl- oder Sulfopropyl(meth)acrylat oder Vinylsulfonsäure, insbesondere mit einem Anteil von 20 bis 90 %; b) einer vinylisch ungesättigten Säure, insbesondere mit einem Anteil von 0 bis 50 %; c) Methyl- oder Ethylacrylat oder -methacrylat, C₂₋₄-Hydroxyalkylacrylat oder N-Vinylpyrrolidon, insbesondere mit einem Anteil von 0 bis 70 % und d) Styrol oder C₄₋₁₈-Alkylacrylat oder C₄₋₁₈-Alkylmethacrylat, insbesondere mit einem Anteil von 0,1 bis 10 %.

Gemäß einer Ausführungsform ist der Emulsionsstabilisator ein Copolymer, enthaltend a) 2-Acrylamido-2-methylpropansulfonsäure, Sulfoethyl- oder Sulfopropyl(meth)acrylat oder Vinylsulfonsäure, insbesondere mit einem Anteil von 40 bis 75 % b) Acrylsäure oder Methacrylsäure, insbesondere mit einem Anteil von 10 bis 40 % c) Methyl- oder Ethyl-acrylat oder -methacrylat, C₂₋₄-Hydroxyalkylacrylat oder N-Vinylpyrrolidon, insbesondere mit einem Anteil von 10 bis 50 % und d) 0,5 bis 5 % Styrol oder C₄₋₁₈-Alkyl-acrylat oder -methacrylat, insbesondere mit einem Anteil von 0,5 bis 5 %.

Gemäß einer Ausführungsform ist der Emulsionsstabilisator ein Copolymer, enthaltend a) 40 bis 75 % 2-Acrylamido-2-methylpropansulfonsäure, Sulfoethyl- oder Sulfopropyl(meth)acrylat oder Vinylsulfonsäure, insbesondere mit einem Anteil von 40 bis 75 % b) Acrylsäure oder Methacrylsäure, 10 bis 30 % c) Methyl- oder Ethyl-acrylat oder -methacrylat oder N-Vinylpyrrolidon, insbesondere mit einem Anteil von 10 bis 50 % und d) Styrol oder C₄₋₁₈-Alkyl-acrylat oder -methacrylat, insbesondere mit einem Anteil von 0,5 bis 5 %.

Ein geeignetes Copolymer ist beispielsweise unter dem Handelsnamen Dimension PA 140 (Fa. Solenis) erhältlich.

Die exakte Bestimmung des Anteils des Emulsionsstabilisators an der Stabilisationsschicht ist technisch schwierig. Im Gegensatz zur Anwendung als Schutzkolloid bei der Verkapselung des Kernmaterials wird jedoch davon ausgegangen, dass ein wesentlicher Teil des Emulsionsstabilisators in der Mikrokapselschale verbaut wird.

Der Anteil des eingesetzten Emulsionsstabilisators an den für die Mikroverkapselung eingesetzten Komponenten kann im Bereich von 0,1 bis 15 Gew.-% liegen. Beispielsweise kann der Anteil des eingesetzten Emulsionsstabilisators 0,1 Gew.-%, 0,2 Gew.-%, 0,5 Gew.-%, 1 Gew.-%,2 Gew.-%,3 Gew.-%, 4 Gew.-%, 5 Gew.-%, 6 Gew.-%, 7 Gew.-%, 8 Gew.-%, 9 Gew.-%, 10 Gew.-%, 11 Gew.-%, 12 Gew.-% 13 Gew.-%, 14 Gew.-% oder 15 Gew.-% betragen. Unterhalb einer Konzentration von 0,1 Gew.-% besteht die Gefahr, dass die Oberfläche der Barriereschicht nicht ausreichend mit dem Emulsionsstabilisator bedeckt ist, um den gewünschten Effekt, nämlich die Erhöhung der Abscheidungsmenge der Stabilitätsschicht zu gewährleisten. Oberhalb von 15 Gew.-% kann die hohe Konzentration eines Emulsionsstabilisators die Ausbildungen der Stabilitätsschicht behindern. In einer bevorzugten Ausführungsform wird der Emulsionsstabilisator mit einem Anteil an den für die Mikroverkapselung eingesetzten Komponenten im Bereich von 0,25 Gew.-% bis 5 Gew.-% eingesetzt. In einer besonders bevorzugten Ausführungsform liegt der Anteil des eingesetzten Emulsionsstabilisators im Bereich von 0,5 Gew.-% bis 4 Gew.-%.

Davon ausgehend, dass zumindest ein Teil des Emulsionsstabilisators in die Mikrokapselwand eingebaut wird, liegt gemäß einer Ausführungsform der Anteil des Emulsionsstabilisators bezogen auf das Gesamtgewicht der Mikrokapselwand im Bereich von 0,5 bis 15,0 Gew.-%. Beispielsweise kann der Anteil des eingesetzten Emulsionsstabilisators 0,5 Gew.-%, 1,0 Gew.-%, 1,5 Gew.-%, 2,0 Gew.-%, 2,5 Gew.-%, 3 Gew.-%, 4 Gew.-%, 5 Gew.-%, 6 Gew.-%, 7 Gew.-%, 8 Gew.-%, 9 Gew.-%, 10 Gew.-%, 11 Gew.-%, 12 Gew.-% 13 Gew.-%, 14 Gew.-% oder 15 Gew.-% betragen In einer bevorzugten Ausführungsform liegt der Anteil an den wandbildenden Komponenten der Mikrokapselschale im Bereich von 1 Gew.-% bis 11 Gew.-%. In einer besonders bevorzugten Ausführungsform liegt der Anteil des eingesetzten Emulsionsstabilisators im Bereich von 2 Gew.-% bis 7 Gew.-%.

Die Barriereschicht enthält bevorzugt als Wandbildner eine oder mehrere Komponenten, ausgewählt aus der Gruppe bestehend aus einer aldehydischen Komponente, einem aromatischen Alkohol, einer Aminkomponente, einer Acrylat-Komponente. Herstellungsverfahren zur Erzeugung von Mikrokapseln mit diesen Wandmaterialien sind dem Fachmann bekannt. Zur Herstellung der Barriereschicht kann ein Polymer, ausgewählt aus einem Polykondensationsprodukt einer aldehydischen Komponente mit einem oder mehreren aromatischen Alkoholen, und/oder Aminkomponenten verwendet werden.

Die geringe Wandstärke der Barriereschicht kann insbesondere mit einer, aromatische Alkohole oder m-Aminophenol enthaltenden, Melamin-Formaldehyd-Schicht erreicht werden. Folglich umfasst die Barriereschicht bevorzugt eine aldehydische Komponente, eine Aminkomponente und einen aromatischen Alkohol.

Der Einsatz von Amin-Aldehyd-Verbindungen in der Barriereschicht, insbesondere Melamin-Formaldehyd, hat den Vorteil, dass diese Verbindungen eine hydrophile Oberfläche mit einem hohen Anteil an Hydroxyfunktionalität ausbilden, die damit eine grundsätzliche Kompatibilität mit den auf Wasserstoffbrücken ausgerichteten Komponenten der ersten Schicht (Stabilitätsschicht), wie bioabbaubare Proteine, Polysaccharide, Chitosan, Lignine und Phosphazene, aber auch anorganischen Wandmaterialien wie CaCO₃ und Polysiloxanen, aufweisen. Genauso können Polyacrylate, insbesondere aus den Komponenten Styrol, Vinylverbindungen, Methylmethacrylat, und 1,4-Butandiolacrylat, Methacrylsäure, durch Initiierung z.B. mit t-Butyl-hydroperoxid in einer radikalisch induzierten Polymerisation (Polyacrylate) als Mikrokapselwand erzeugt werden, die eine hydrophile Oberfläche mit einem hohen Anteil an Hydroxyfunktionalität ausbilden, die deshalb genauso kompatibel mit den Komponenten der Stabilitätsschicht sind.

In einer bevorzugten Ausführungsform ist somit ein Wandbildner der Barriereschicht eine aldehydische Komponente. Gemäß einer Ausführungsform ist die aldehydische Komponente der Barriereschicht ausgewählt aus der Gruppe bestehend aus Formaldehyd, Glutaraldehyd, Succinaldehyd, Furfural und Glyoxal. Mit all diesen Aldehyden wurden schon erfolgreich Mikrokapseln hergestellt (siehe WO 2013 037 575 A1), so dass davon ausgegangen werden kann, dass damit ähnlich dichte Kapseln wie mit Formaldehyd erhalten werden.

Der Anteil der aldehydischen Komponente kann für die Wandbildung bezogen auf das Gesamtgewicht der Barriereschicht im Bereich von 5 Gew.-% bis 50 Gew.-% liegen. Beispielsweise kann der Anteil der aldehydischen Komponente bei 5 Gew.-%, 6 Gew.-%, 7 Gew.-%, 8 Gew.-%, 9 Gew.-%, 10 Gew.-% oder 15 Gew.-% 20 Gew.-%, 25 Gew.-%, 30 Gew.-%, 35 Gew.-%, 40 Gew.-%, 45 Gew.-% oder 50 Gew.-%, liegen. Es wird davon ausgegangen, dass außerhalb dieser Grenzen keine ausreichend stabile und dichte, dünne Schicht erhalten werden kann. Bevorzugt liegt die Konzentration der aldehydischen Komponente in der Barriereschicht im Bereich von 10 Gew.-% bis 30 Gew.-%. Besonders bevorzugt liegt die Konzentration der aldehydischen Komponente in der Barriereschicht im Bereich von 15 Gew.-% bis 20 Gew.-%.

Als Aminkomponente in der Barriereschicht kommen insbesondere Melamin, Melaminderivate und Harnstoff oder Kombinationen davon in Frage. Geeignete Melaminderivate sind veretherte Melaminderivate sowie methylolierte Melaminderivate. Melamin in der methylolierten Form ist dabei bevorzugt. Die Aminkomponenten können beispielsweise in Form von alkylierten Mono- und Polymethylol-Harnstoff-Vorkondensationsprodukten oder partiell methylolierten Mono- und Polymethylol-1,3,5 triamono- 2,4,6 Triazin-Vorkondensationsprodukten wie Dimension SD^{®} (von Solenis) eingesetzt werden. Gemäß einer Ausführungsform ist die Aminkomponente Melamin. Gemäß einer alternativen Ausführungsform ist die Aminkomponente eine Kombination von Melamin und Harnstoff.

Die aldehydische Komponente und die Aminkomponente können in einem Molverhältnis im Bereich von 1:5 bis 3:1 vorliegen. Beispielsweise kann das Molverhältnis 1:5, 1:4,5, 1:4, 1:3,5, 1:3, 1:2,5, 1:2, 1:1,8, 1:1,6, 1:1,4, 1:1,35, 1;1,3, 1:1,2, 1:1, 1,5:1, 2:1, 2,5:1, oder 3:1 sein. Bevorzugt liegt das Molverhältnis im Bereich von 1:3 bis 2:1. Besonders bevorzugt kann das Molverhältnis der aldehydischen Komponente und der Aminkomponente im Bereich von 1:2 bis 1:1 liegen. Die aldehydische Komponente und die Aminkomponente werden in der Regel im Verhältnis von etwa 1:1,35 eingesetzt. Dieses Molverhältnis erlaubt eine vollständige Reaktion der beiden Reaktionspartner und führt zu einer hohen Dichtigkeit der Kapseln. Es sind beispielsweise auch Aldehyd-Amin-Kapselwände bekannt mit einem Molverhältnis von 1:2. Diese Kapseln haben den Vorteil, dass der Anteil des hochvernetzenden Aldehyds, insbesondere Formaldehyd sehr gering ist. Allerdings weisen diese Kapseln eine geringere Dichtigkeit auf als die Kapseln mit einem Verhältnis von 1:1,35. Kapseln mit einem Verhältnis von 2:1 weisen eine erhöhte Dichtigkeit auf, haben jedoch den Nachteil, dass die Aldehyd-Komponente teilweise unreagiert in der Kapselwand und der Slurry vorliegt.

In einer Ausführungsform liegt der Anteil der Aminkomponente(n) (bspw. Melamin und/oder Harnstoff) in der Barriereschicht bezogen auf das Gesamtgewicht der Barriereschicht im Bereich von 20 Gew.-% bis 85 Gew.-%. Beispielsweise kann der Anteil der Aminkomponente bei 20 Gew.-%, 25 Gew.-%, 30 Gew.-%, 35 Gew.-%, 40 Gew.-%, 45 Gew.-%, 50 Gew.-%, 55 Gew.-%, 60 Gew.-%, 65 Gew.-%, 70 Gew.-%, 75 Gew.-%, 80 Gew.-% oder 85 Gew.-% liegen. In einer bevorzugten Ausführungsform liegt der Anteil der Aminkomponente in der Barriereschicht bezogen auf das Gesamtgewicht der Barriereschicht im Bereich von 40 Gew.-% bis 80 Gew.-%. Besonders bevorzugt liegt der Anteil der Aminkomponente im Bereich von 55 bis 70 Gew.-%.

Mit dem aromatischen Alkohol ist es möglich die Wandstärke der aus der Amin-Komponente und der Aldehyd-Komponente aufgebauten Barriereschicht stark zu reduzieren, um dennoch eine Schicht zu erhalten, die die notwendige Dichtheit aufweist und zumindest in Kombination mit der Stabilitätsschicht stabil genug ist. Die aromatischen Alkohole verleihen der Wand eine erhöhte Dichtheit, da ihre stark hydrophobe Aromatenstruktur das Hindurchdiffundieren niedermolekularer Substanzen erschwert. Wie in den Beispielen dargestellt eignet sich als aromatischer Alkohol besonders Phloroglucin, Resorcin oder m-Aminophenol. Folglich ist der aromatische Alkohol in einer Ausführungsform ausgewählt aus der Gruppe bestehend aus Phloroglucin, Resorcin und Aminophenol. In Kombination mit der Amin- und der Aldehyd-Komponente wird der aromatische Alkohol in einem Molverhältnis zur Aldehyd-Komponente im Bereich von (Alkohol:Aldehyd) 1:1 bis 1:20, bevorzugt im Bereich von 1:2 bis 1:10 eingesetzt.

In einer Ausführungsform liegt der Anteil des aromatischen Alkohols in der Barriereschicht bezogen auf das Gesamtgewicht der Barriereschicht im Bereich von 1,0 Gew.-% bis 20 Gew.-%. Beispielsweise kann der Anteil des aromatischen Alkohols bei 1,5 Gew.-%, 2,0 Gew.-%, 2,5 Gew.-%, 3,0 Gew.-%, 4,0 Gew.-%, 5,0 Gew.-%, 6 Gew.-%, 7 Gew.-%, 8 Gew.-%, 9 Gew.-%, 10 Gew.-%, 11 Gew.-%, 12 Gew.-% 13 Gew.-%, 14 Gew.-%, 15 Gew.-%, 16 Gew.-%, 17 Gew.-%, 18 Gew.-%, 19 Gew.-% oder 20 Gew.-% liegen. Aufgrund ihrer aromatischen Struktur geben die aromatischen Alkohole der Kapselwand eine Färbung, die mit dem Anteil des aromatischen Alkohols zunimmt. Eine solche Färbung ist in einer Vielzahl von Anwendungen unerwünscht. Zudem sind die aromatischen Alkohole oxidationsanfällig, was zu einer Veränderung der Färbung im Laufe der Zeit führt. Dadurch kann die unerwünschte Färbung der Mikrokapseln schlecht mit einem Farbstoff ausgeglichen werden. Deshalb sollten die aromatischen Alkohole nicht oberhalb von 20,0 Gew.-% eingesetzt werden. Unterhalb von 1,0 Gew.-% ist kein Effekt bezüglich der Dichtigkeit nachweisbar. In einer bevorzugten Ausführungsform liegt der Anteil des aromatischen Alkohols in der Barriereschicht bezogen auf das Gesamtgewicht der Barriereschicht im Bereich von 5,0 Gew.-% bis 15,0 Gew.-%. Bis zu einem Prozentsatz von 15,0 Gew.-% ist die Färbung in den meisten Anwendungen tolerierbar. In einer besonders bevorzugten Ausführungsform liegt der Anteil des aromatischen Alkohols in der Barriereschicht bezogen auf das Gesamtgewicht der Barriereschicht im Bereich von 6 Gew.-% bis 16,0 Gew.-%. Insbesondere liegt der Anteil des aromatischen Alkohols in der Barriereschicht im Bereich von 10 Gew.-% bis 14,0 Gew.-%.

In einer weiteren Ausführungsform kann die Aldehydkomponente der Barriereschicht zusammen mit einem aromatischen Alkohol wie Resorcin, Phloroglucin oder m-Aminophenol als wandbildende Komponente(n) verwendet werden, d.h. unter Verzicht auf die Aminkomponente(n).

In einer Ausführungsform enthält die Barriereschicht Melamin, Formaldehyd und Resorcin. In einer Ausführungsform enthält die Barriereschicht der Mikrokapseln Melamin, Harnstoff, Formaldehyd und Resorcin. In einer bevorzugten Ausführungsform enthält die Barriereschicht Melamin im Bereich von 25 bis 40 Gew.-%, Formaldehyd im Bereich von 15 bis 20 Gew.-% und Resorcin im Bereich von 10 bis 14 Gew.-% und gegebenenfalls Harnstoff im Bereich von 25 bis 35 Gew.-%. Die Anteile beziehen sich auf die für die Wandbildung der Schicht eingesetzten Mengen und sind bezogen auf das Gesamtgewicht der Barriereschicht ohne Schutzkolloid.

Zur Verkapselung des Kernmaterials mit der Barriereschicht aus einer aldehydischen Komponente, einer Aminkomponente und einem aromatischen Alkohol wird wie oben erwähnt bevorzugt ein Emulsionsstabilisator als Schutzkolloid eingesetzt werden. Der als Schutzkolloid verwendete Emulsionsstabilisator kann ein wie oben als Vermittleragens definiertes Polymer oder Copolymer sein. Beispielsweise ist das Schutzkolloid ein Copolymer enthaltend AMPS (Dimension^{®}PA 140, Fa. Solenis) oder dessen Salze. In einer Ausführungsform wird als Schutzkolloid und als Vermittleragens dasselbe Copolymer verwendet.

Als Aminkomponente in der Barriereschicht kommen insbesondere Melamin, Melaminderivate und Harnstoff oder Kombinationen davon in Frage. Geeignete Melaminderivate sind veretherte Melaminderivate sowie methylolierte Melaminderivate. Melamin in der methylolierten Form ist dabei bevorzugt. Die Aminkomponenten können beispielsweise in Form von alkylierten Mono- und Polymethylol-Harnstoff-Vorkondensationsprodukten oder partiell methylolierten Mono- und Polymethylol-1,3,5 triamono- 2,4,6 Triazin-Vorkondensationsprodukten wie Dimension SD^{®} (von Solenis) eingesetzt werden. Gemäß einer Ausführungsform ist die Aminkomponente Melamin. Gemäß einer alternativen Ausführungsform ist die Aminkomponente eine Kombination von Melamin und Harnstoff.

Die Stabilitätsschicht bildet den Hauptbestandteil der Mikrokapselschale und gewährleistet so eine hohe Bioabbaubarkeit nach OECD 301 F von mindestens 40 % innerhalb von 60 Tagen. Als Wandbildner für die Stabilitätsschicht geeignete Biopolymere sind Proteine wie Gelatine, Molkeprotein, Pflanzenspeicherprotein; Polysaccharide wie Alginat, Gummi arabicum modifiziertes Gummi, Chitin, Dextran, Dextrin, Pektin, Cellulose, modifizierte Cellulose, Hemicellulose, Stärke oder modifizierte Stärke; phenolische Makromoleküle wie Lignin; Polyglucosamine wie Chitosan, Polyvinylester, wie Polyvinylalkohole und Polyvinylacetat; Phosphazene und Polyestern wie Polylactid oder Polyhydroxyalkanoat. Diese Aufzählung der konkreten Komponenten in den einzelnen Stoffklassen ist nur beispielhaft und soll nicht als limitierend verstanden werden. Dem Fachmann sind geeignete natürliche Wandbildner bekannt. Ferner sind dem Fachmann die verschiedenen Verfahren zur Wandbildung, beispielsweise Koazervation oder Grenzflächenpolymerisation bekannt.

Die Biopolymere können für die jeweilige Anwendung entsprechend ausgewählt werden, um mit dem Material der Stabilitätsschicht eine stabile Mehrschichtschale auszubilden. Zudem können die Biopolymere ausgewählt werden, um eine Kompatibilität mit den chemischen Gegebenheiten des Anwendungsgebiets zu erreichen. Die Biopolymere können beliebig kombiniert werden, um die Bioabbaubarkeit oder auch beispielsweise Stabilität und chemische Resistenz der Mikrokapsel zu beeinflussen.

In einer Ausführungsform des ersten Aspekts weist die Schale der Mikrokapseln eine Bioabbaubarkeit von 50 % nach OECD 301 F auf. In einer weiteren Ausführungsform weist die Schale der Mikrokapsel eine Bioabbaubarkeit von mindestens 60 % (OECD 301 F) auf. In einer weiteren Ausführungsform beträgt die Bioabbaubarkeit mindestens 70 % (OECD 301 F). Die Bioabbaubarkeit ist jeweils gemessen über einen Zeitraum von 60 Tagen. Im verlängerten Abbauverfahren ("enhanced ready biodegredation") wird die Bioabbaubarkeit über einen Zeitraum von 60 Tagen gemessen (siehe Opinion on an Annex XV dossier proposing restrictions on intentionally-added microplastics of June 11, 2020 ECHA/RAC/RES-O-0000006790-71-01/F). Bevorzugt werden die Mikrokapseln vor der Bestimmung der Bioabbaubarkeit mittels Waschen von Rückständen befreit. Besonders bevorzugt werden Kopien der Mikrokapseln für diesen Test mit einem inerten, nicht biologisch abbaubaren Kernmaterial wie Perfluoroctan (PFO) anstelle des Parfümöls hergestellt. In einer Ausführungsform wird die Kapseldispersion nach Herstellung durch dreimaliges Zentrifugieren und Redispergieren in dest. Wasser gewaschen. Dafür wird die Probe zentrifugiert (z.B. für 10 min bei 12.000 RPM). Nach Absaugen des Klarüberstandes wird mit Wasser aufgefüllt und der Bodensatz durch Schütteln redispergiert. Bei der Messung der Bioabbaubarkeit können verschiedene Referenzproben eingesetzt werden, wie das schnell abbaubare Ethylenglycol oder naturbasiertes Wallnussschalen-Mehl mit dem typischen stufenartigen Abbau eines komplexen Stoffgemisches. Die Mikrokapsel zeigt eine ähnliche, bevorzugt bessere Bioabbaubarkeit über einen Zeitraum von 28 oder 60 Tagen als das Wallnussschalen-Mehl.

Rückstände in den Mikrokapseldispersionen sind Stoffe, die bei der Herstellung der Mikrokapseln verwendet werden und in nicht-kovalenter Wechselwirkung mit der Schale stehen wie Ablagerungshilfsmittel, Konservierungsmittel, Emulgatoren/Schutzkolloide, überschüssige Einsatzstoffe. Diese Rückstände haben einen nachgewiesenen Einfluss auf die biologische Abbaubarkeit von Mikrokapseldispersionen. Aus diesem Grund ist das Waschen vor der Bestimmung der Bioabbaubarkeit notwendig.

Um einen Eindruck über den Anteil an kovalent gebundenen und nicht-kovalenten gebundenen Bestandteilen in der Mikrokapseldispersion zu gewinnen, wurden die Kapseln mittels der in Gasparini et al. 2020 beschriebenen Quantifizierungsmethode auf Basis von Py-GC-MS für polymerverkapselten Duftstoffe untersucht. Diese Methode beinhaltet ein mehrstufiges Reinigungsprotokoll für Polymere aus komplexen Proben wie Mikrokapseldispersionen und ermöglicht die Quantifizierung von flüchtigen Restbestandteilen, von denen vermutet wird, dass sie nicht kovalent in das 3D-Polymernetzwerk eingebunden sind und daher mit anderen Standardmethoden (z. B. SPME-GC-MS oder TGA) nicht quantifizierbar sind. Anhand dieses Verfahrens wurde bestätigt, dass einzelnen Schichten der Mikrokapsel, insbesondere die Barriere- und die Stabilitätsschicht, untrennbar verbunden und als Monopolymer angesehen werden können. Es ist davon auszugehen, dass durch Zugabe des Emulsionsstabilisators nicht nur die strukturelle Aufnahme der Stabilitätsschicht durch die Barriereschicht verbessert, sondern zusätzlich die strukturelle (kovalente) Verbindung aller wandbildenden Komponenten erhöht wird.

Ein hoher Wert der Bioabbaubarkeit wird zum einen durch die verwendeten Wandbildner zum anderen aber durch den Aufbau der Schale erreicht. Denn der Einsatz eines bestimmten Prozentsatzes an Biopolymeren führt nicht automatisch zu einem entsprechenden Wert der Bioabbaubarkeit. Dies ist abhängig davon, wie die Biopolymere in der Schale vorliegen.

Gemäß einer bevorzugten Ausführungsform enthält die Stabilitätsschicht Gelatine als Biopolymer. Gemäß einer weiteren bevorzugten Ausführungsform enthält die Stabilitätsschicht Alginat als Biopolymer. Gemäß einer weiteren bevorzugten Ausführungsform enthält die Stabilitätsschicht Gelatine und Alginat als Biopolymere. Sowohl Gelatine als auch Alginat sind geeignet für die Herstellung von Mikrokapseln mit hoher Bioabbaubarkeit und hoher Stabilität. Insbesondere bei einer Gelatine und Alginat enthaltenden Stabilitätsschicht kann die Behandlung der Oberfläche der Barriereschicht mit einem Emulsionsstabilisator, insbesondere einem AMPS enthaltenden Copolymer, zu einer starken Zunahme der Schichtdicke der Stabilitätsschicht führen. Weitere geeignete Kombinationen natürlicher Komponenten in der ersten Schicht (Stabilitätsschicht) sind Gelatine und Gummi arabicum.

Die Stabilitätsschicht enthält ein oder mehrere Aushärtungsmittel. Geeignete Aushärtungsmittel sind Aldehyde wie beispielsweise Glutaraldehyd, Formaldehyd und Glyoxal sowie Tannine, Enzyme wie Transglutaminase und organische Anhydride wie Maleinsäureanhydrid, Epoxyverbindungen mehrwertige Metallkationen, Amine, Polyphenole, Maleimide, Sulfide, Phenoloxide, Hydrazide, Isocyanate, Isothiocyanate, N-Hydroxysulfosuccinimid-Derivate, Carbodiimid-Derivate, und Polyole. Bevorzugt ist das Aushärtungsmittel Glutaraldehyd auf Grund seiner sehr guten Vernetzereigenschaft. Weiterhin bevorzugt ist das Aushärtungsmittel Glyoxal auf Grund seiner guten Vernetzereigenschaften und, im Vergleich zu Glutaraldehyd, niedrigeren toxikologischen Einstufung. Durch die Verwendung von Aushärtungsmitteln wird eine höhere Dichtigkeit der Stabilitätsschicht erreicht. Allerdings führen Aushärtungsmittel zu einer reduzierten Bioabbaubarkeit der natürlichen Polymere.

Aufgrund der Präsenz der Barriereschicht als Diffusionsbarriere, kann die Menge an Aushärtungsmittel in der Stabilitätsschicht geringgehalten werden, was wiederum zur leichten Bioabbaubarkeit der Schicht beiträgt. Gemäß einer Ausführungsform liegt der Anteil des Aushärtungsmittels an der Stabilitätsschicht unterhalb von 25 Gew.-%. Soweit nicht explizit anders definiert, beziehen sich die Anteile der Bestandteile der Schichten auf das Gesamtgewicht der Schicht, d.h. das Gesamttrockengewicht der zur Herstellung verwendeten Bestandteile, ohne Berücksichtigung der in der Herstellung verwendeten Bestandteile, die nicht bzw. nur geringfügig in die Schicht eingebaut werden, wie Tenside und Schutzkolloide. Oberhalb dieses Wertes kann die Bioabbaubarkeit nach OECD 301 F nicht gewährleistet werden. Der Anteil des Aushärtungsmittels an der Stabilitätsschicht kann beispielsweise 1,0 Gew.-%, 2,0 Gew.-%, 3,0 Gew.-%, 4,0 Gew.-%, 5,0 Gew.-%, 6 Gew.-%, 7 Gew.-%, 8 Gew.-%, 9 Gew.-%, 10 Gew.-%, 11 Gew.-%, 12 Gew.-% 13 Gew.-%, 14 Gew.-%, 15 Gew.-%, 16 Gew.-%, 17 Gew.-%, 18 Gew.-%, 19 Gew.-%, 20 Gew.-%, 21 Gew.-%, 22 Gew.-%, 23 Gew.-% oder 24 Gew.-% betragen. Bevorzugt liegt der Anteil des Aushärtungsmittels an der Stabilitätsschicht im Bereich von 1 bis 15 Gew.-%. Dieser Anteil führt zur effektiven Vernetzung der Gelatine und führt in einer quantitativen Reaktion dazu, dass möglichst wenig Restmonomer entsteht. Der Bereich 9 bis 12 Gew.-% ist besonders bevorzugt, er sorgt für den benötigten Vernetzungsgrad und für eine stabile Umhüllung der Barriereschicht, um die ansonsten empfindliche Barriereschicht abzupuffern und hat nur wenig Restaldehyd, der in einer nachgeschalteten alkalischen Einstellung der Slurry über eine Aldolreaktion abgebaut wird.

In einer Ausführungsform enthält die Stabilitätsschicht Gelatine und Glutaraldehyd. Nach einer weiteren Ausführungsform enthält die Stabilitätsschicht Gelatine, Alginat und Glutaraldehyd. In einer zusätzlichen Ausführungsform enthält die Stabilitätsschicht Gelatine und Glyoxal. Nach einer weiteren Ausführungsform enthält die Stabilitätsschicht Gelatine, Alginat und Glyoxal. Die genaue chemische Zusammensetzung der Stabilitätsschicht ist nicht entscheidend. Allerdings wird der gewünschte Effekt bevorzugt mit polaren Biopolymeren erreicht.

Gemäß einer bevorzugten Ausführungsform enthält die Mikrokapselschale kein Titandioxid. Gemäß einer weiteren bevorzugten Ausführungsform enthält die Mikrokapselschale kein Metalloxid. Gemäß einer ebenfalls bevorzugten Ausführungsform enthält die Mikrokapselschale kein Pigment. Gemäß einer zusätzlichen Ausführungsform enthält die Mikrokapselschale keinen Farbstoff.

Durch die Verwendung des Emulsionsstabilisators auf der Oberfläche der Barriereschicht wird die mittlere Dicke der Stabilitätsschicht signifikant erhöht. Die mittlere Dicke der Stabilitätsschicht beträgt mindestens 1 µm. Die mittlere Dicke der Stabilitätsschicht kann 1 µm, 1,2 µm, 1,4 µm, 1,6 µm, 1,8 µm 2 µm, 2,2 µm, 2,4 µm, 2,6 µm, 2,8 µm, 3 µm, 3,5 µm, 4 µm, 4,5 µm, 5 µm, 5,5 µm, 6 µm, 6,5 µm, 7 µm, 7,5 µm, 8 µm, 8,5 µm, 9 µm, 9,5 µm, oder 10 µm betragen. Die Stabilitätsschicht hat im Querschnitt häufig eine elliptische Form, damit variiert die Dicke der Stabilitätsschicht über die Mikrokapseloberfläche. Deshalb wird eine mittlere Dicke der Mikrokapseln berechnet. Darüber variiert die Abscheidung von Mikrokapsel zu Mikrokapsel. Dem wird dadurch Rechnung getragen, dass die mittleren Dicken mehrerer Mikrokapseln bestimmt werden und davon der Durchschnitt berechnet wird. Somit ist die hier genannte mittlere Dicke genau genommen eine durchschnittliche mittlere Dicke. Die Bestimmung der Schichtdicke der Stabilitätsschicht kann auf zwei Wegen erfolgen. Zunächst sei hier der lichtmikroskopische Ansatz erwähnt, also das direkte, optische Ausmessen der beobachteten Schichtdicke mittels eines Mikroskops und entsprechender Software. Dabei wird eine Vielzahl an Mikrokapseln einer Dispersion gemessen und aufgrund der Varianz innerhalb der Kapseln der Mindestdurchmesser jeder einzelnen Mikrokapsel bestimmt.

Eine zweite Möglichkeit stellt die Messung der Partikelgrößenverteilung mittels Laserbeugung dar. Hier kann der Modalwert einer Partikelgrößenverteilung des ohne die zu messende Schicht in Vergleich des Modalwerts einer Partikelgrößenverteilung mit der zu messenden Schicht gesetzt werden. Die Vergrößerung dieses Modalwerts gibt die Vergrößerung des hydrodynamischen Durchmessers der Hauptfraktion an vermessenen Mikrokapseln wieder. Die Bildung der Differenz aus den beiden gemessenen Modalwerten ergibt letztlich die zweifache Schichtdicke der Schicht.

Gemäß einer bevorzugten Ausführungsform beträgt die mittlere Dicke der Stabilitätsschicht mindestens 2 µm. Durch Wahl einer geeigneten Kombination von Emulsionsstabilisator und Wandbildner der Stabilitätsschicht, können Stabilitätsschichten mit einer mittleren Dicke von 6 µm oder mehr gebildet werden. In einer besonders bevorzugten Ausführungsform beträgt die mittlere Dicke der Stabilitätsschicht mindestens 3 µm.

Im Gegensatz zu anderen bioabbaubaren Mikrokapseln weisen die hierin beschriebenen Mikrokapseln eine hohe Dichtheit auf. Gemäß einer Ausführungsform weisen die Mikrokapseln eine Dichtheit auf, die einen Austritt von höchstens 50 Gew.-% des eingesetzten Kernmaterials nach Lagerung über einen Zeitraum von 4 Wochen bei einer Temperatur von 0 bis 40 °C gewährleistet.

Die Dichtigkeit der Kapselwand kann mit der Wahl der Schalenkomponenten beeinflusst werden. Gemäß einer Ausführungsform weisen die Mikrokapseln eine Dichtheit auf, die einen Austritt von höchstens 45 Gew.-%, höchstens 40 Gew.-%, höchstens 35 Gew.-%, höchstens 30 Gew.-%, höchstens 25 Gew.-%, höchstens 20 Gew.-% des eingesetzten Kernmaterials bei Lagerung über einen Zeitraum von 4 Wochen bei einer Temperatur von 0 bis 40 °C gewährleistet. Dabei werden die Mikrokapseln in einer der Zielanwendung entsprechenden Modellformulierung gelagert. Die Mikrokapseln sind darüber hinaus auch in dem Produkt, in dem sie verwendet werden, lagerstabil. Beispielsweise in Waschmitteln, Weichspülern oder Kosmetikprodukten. Dem Fachmann sind die Richtrezepturen dieser Produkte bekannt. Typischerweise liegt der pH-Wert in der Umgebung der Mikrokapseln bei der Lagerung im Bereich von 2 bis 12.

Die Mikrokapselschalen weisen mindestens zwei Schichten auf, d.h. sie können z.B. zweischichtig, dreischichtig, vierschichtig, oder fünfschichtig sein. Bevorzugt sind die Mikrokapseln zwei- oder dreischichtig.

Gemäß einer Ausführungsform weist die Mikrokapsel eine dritte Schicht auf, die an der Außenseite der Stabilitätsschicht angeordnet ist. Diese dritte Schicht kann eingesetzt werden, um die Oberflächeneigenschaften der Mikrokapsel für eine bestimmte Anwendung anzupassen. Zu nennen wären hier die Verbesserung der Haftung der Mikrokapseln auf verschiedensten Oberflächen und eine Reduzierung der Agglomeration. Die dritte Schicht bindet zudem Restaldehydmengen, verringert damit den Gehalt an freien Aldehyden in der Kapseldispersion. Ferner kann sie zusätzliche (mechanische) Stabilität erbringen oder die Dichtigkeit weiter erhöhen. Abhängig von der Anwendung kann die dritte Schicht eine Komponente ausgewählt aus Aminen, organischen Salzen, anorganischen Salzen, Alkoholen, Ethern, Polyphosphazenen und Edelmetallen enthalten.

Edelmetalle erhöhen die Dichtigkeit der Kapseln und können der Mikrokapseloberfläche zusätzliche katalytische Eigenschaften verleihen oder die antibakterielle Wirkung einer Silberschicht. Organische Salze, insbesondere Ammoniumsalze, führen zu einer Kationisierung der Mikrokapseloberfläche, die dazu führt, dass diese besser an z.B. Textilien haftet. Auch Alkohole führen bei Einbindung über freie Hydroxylgruppen zur Bildung von H-Brücken, die ebenfalls bessere Anhaftung an Substrate erlauben. Eine zusätzliche Polyphosphazen-Schicht oder die Beschichtung mit anorganischen Salzen, bspw. Silikaten, führt zu einer zusätzlichen Erhöhung der Dichtigkeit ohne die Bioabbaubarkeit zu beeinflussen. Gemäß einer bevorzugten Ausführungsform enthält die dritte Schicht aktiviertes Melamin. Das Melamin fängt zum einen mögliche freie Aldehydanteile der Stabilitäts- und/oder Barriereschicht auf, erhöht die Dichtheit und Stabilität der Kapsel und kann zudem die Oberflächeneigenschaften der Mikrokapseln und damit das Anhaftungs- und Agglomerationsverhalten beeinflussen.

Aufgrund der geringen Wandstärken beträgt der Anteil der Barriereschicht an der Schale bezogen auf das Gesamtgewicht der Schale höchstens 30 Gew.-%. Der Anteil der Barriereschicht an der Schale bezogen auf das Gesamtgewicht der Schale kann beispielsweise 30 Gew.-%, 28 Gew.-%, 25 Gew.-%, 23 Gew.-%, 20 Gew.-%. 18 Gew.-%, 15 Gew.-%. 13 Gew.-%, 10 Gew.-%, 8 Gew.-%, oder 5 Gew.-% betragen. Für eine hohe Bioabbaubarkeit beträgt der Anteil höchstens 25 Gew.-% bezogen auf das Gesamtgewicht der Schale. Besonders bevorzugt beträgt der Anteil der Barriereschicht höchstens 20 Gew.-%. Der Anteil der Stabilitätsschicht an der Schale bezogen auf das Gesamtgewicht der Schale beträgt mindestens 40 Gew.-%. Der Anteil der Stabilitätsschicht an der Schale bezogen auf das Gesamtgewicht der Schale kann beispielsweise 40 Gew.-%, 43 Gew.-%, 45 Gew.-%, 48 Gew.-%, 50 Gew.-%. 53 Gew.-%, 55 Gew.-%. 58 Gew.-%, 60 Gew.-%, 63 Gew.-%, 65 Gew.-%, 68 Gew.-%, 70 Gew.-% 75 Gew.-%, 80 Gew.-%, 85 Gew.-%, oder 90 Gew.-%, betragen. Für eine hohe Bioabbaubarkeit beträgt der Anteil der Stabilitätsschicht mindestens 50 Gew.-%, besonders bevorzugt mindestens 60 Gew.-%. Der Anteil der dritten Schicht an der Schale bezogen auf das Gesamtgewicht der Schale beträgt höchstens 35 Gew.-%. Der Anteil der dritten Schicht an der Schale bezogen auf das Gesamtgewicht der Schale kann beispielsweise 35 Gew.-%, 33 Gew.-%, 30 Gew.-%, 28 Gew.-%, 25 Gew.-%, 23 Gew.-%, 20 Gew.-%. 18 Gew.-%, 15 Gew.-%. 13 Gew.-%, 10 Gew.-%, 8 Gew.-%, oder 5 Gew.-% betragen. Für eine hohe Bioabbaubarkeit beträgt der Anteil der dritten Schicht bevorzugt höchstens 30 Gew.-%, besonders bevorzugt höchstens 25 Gew.-%.

Die Größe der Mikrokapseln liegt im für Mikrokapseln üblichen Bereich. Dabei kann der Durchmesser im Bereich von 100 nm bis 1 mm liegen. Der Durchmesser ist abhängig von der genauen Kapselzusammensetzung und dem Herstellungsverfahren. Als Kennwert für die Größe der Kapseln wird regelmäßig das Peak-Maximum der Partikelgrößenverteilung verwendet. Bevorzugt liegt das Peak-Maximum der Partikelgrößenverteilung im Bereich von 1 µm bis 500 µm. Das Peak-Maximum der Partikelgrößenverteilung kann beispielsweise bei 1 µm, 2 µm, 3 µm, 4 µm, 5 µm, 10 µm, 15 µm, 20 µm, 30 µm, 40 µm, 50 µm, 60 µm, 70 µm, 80 µm, 90 µm, 100 µm, 120 µm, 140 µm, 160 µm, 180 µm 200 µm, 250 µm, 300 µm 350 µm, 400 µm, 450 µm oder 500 µm liegen. Gemäß einer besonders bevorzugten Ausführungsform haben die Mikrokapseln ein Peak-Maximum der Partikelgrößenverteilung von 10 µm bis 100 µm. Insbesondere liegt das Peak-Maximum der Partikelgrößenverteilung im Bereich von 10 µm bis 50 µm.

Der Einsatz des Emulsionsstabilisators zur Beschichtung der Barriereschicht stellt eine neue vom üblichen Einsatz des Emulsionsstabilisators, nämlich der Stabilisierung der Kernmaterialtröpfchen, zu unterscheidenden Verwendung dar.

Neben dem Schalenmaterial ist die Dichtheit auch von der Art des Kernmaterials abhängig. Als Kernmaterial kommt eine Vielzahl unterschiedlicher Materialien in Frage, unter anderem Duftstoffe, und kosmetische Wirkstoffe. Das Kernmaterial ist gemäß einer bevorzugten Ausführungsform der Mikrokapseln hydrophob. Das Kernmaterial kann fest oder flüssig sein. Insbesondere ist es flüssig. Bevorzugt handelt es sich um ein flüssiges hydrophobes Kernmaterial. In einer bevorzugten Ausführungsform handelt es sich bei dem Kernmaterial um einen Duftstoff bzw. umfasst das Kernmaterial mindestens einen Duftstoff. Besonders bevorzugt handelt es sich um für die Mikroverkapselung optimierte Duft- oder Parfümöle für den Wasch und Reinigungsmittelbereich, wie beispielsweise die Duftformulierung Weiroclean (Fa. Kurt Kitzing GmbH). Die Duftstoffe können in Form einer festen oder flüssigen Formulierung eingesetzt werden, insbesondere aber in flüssiger Form. Duftstoffverbindungen vom Typ der Ester sind z.B. Benzylacetat, Phenoxyethylisobutyrat, *p-tert-*Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden die oben genannten z.B. die linearen Alkanale mit 8 bis 18 C-Atomen, Citral, Citronellal, Citronellyloxyacetaldehyd, Cyclamenaldehyd (3-(4-Propan-2-ylphenyl)butanal), Lilial und Bourgeonal, zu den Ketonen z.B. die Ionone, [alpha]-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Duftstoffe verwendet, die gemeinsam eine ansprechende Duftnote erzeugen.

Geeignete Duftstoffaldehyde können ausgewählt werden aus Adoxal (2,6,10-Trimethyl-9-undecenal), Anisaldehyd (4-Methoxybenzaldehyd), Cymal oder Cyclamenaldehyd (3-(4-Isopropylphenyl)-2-methylpropanal), Nympheal (3-(4-Isobutyl-2-methylphenyl)propanal), Ethylvanillin, Florhydral (3-(3-Isopropylphenyl)butanal]), Trifernal (3-Phenylbutyraldehyd), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), Triplal (2,4-Dimethyl-3-cyclohexen-1-carboxaldehyd), 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, Tetrahydrocitral (3,7-Dimethyloctanal), Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Floral (4,8-Dimethyl-4,9-decadienal), Aldehyd C12MNA (2-Methylundecanal), Liminal (beta-4-Dimethylcyclohex-3-ene-1-propan-1-al), Methylnonylacetaldehyd, Hexanal, trans-2-Hexenal und Mischungen davon.

Geeignete Duftstoffketone schließen ein, sind aber nicht beschränkt auf Methyl-beta-naphthylketon, Moschusindanon (1,2,3,5,6,7-Hexahydro-1,1,2,3,3-pentamethyl-4H-inden-4-on), Calone (Methylbenzodioxepinon), Tonalid (6-Acetyl-1,1,2,4,4,7-hexamethyltetralin), alpha-Damascone, beta-Damascone, delta-Damascone, iso-Damascone, Damascenone, Methyldihydrojasmonat (Hedion), Menthon, Carvon, Kampfer, Koavon (3,4,5,6,6-Pentamethylhept-3-en-2-on), Fenchon, alpha-Ionon, beta-Ionon, Dihydro-beta-Ionon, gamma-Methyl-lonon, Fleuramon (2-Heptylcyclopentanon), Frambinonmethylether (4-(4-Methoxyphenyl)butan-2-on), Dihydrojasmon, cis-Jasmon, 1-(1,2,3,4,5,6,7,8-Octahydro-2,3,8,8-tetramethyl-2-naphthalenyl)-ethan-1-on und Isomere davon, Methylcedrenylketon, Acetophenon, Methylacetophenon, para-Methoxyacetophenon, Methyl-betanaphtylketon, Benzylaceton, Benzophenon, para-Hydroxyphenylbutanon, Sellerie-Keton(3-Methyl-5-propyl-2-cyclohexenon), 6-Isopropyldeca-hydro-2-naphton, Dimethyloctenon, Frescomenthe (2-Butan-2-ylcyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl (4-(1,3-Benzodioxol-5-yl)butan-2-on), Hexalon (1-(2,6,6-Trimethyl-2-cyclohexen-1-yl)-1,6-heptadien-3-on), Isocyclemon E (2-Acetonaphthon-1 ,2,3,4,5,6,7,8-octahydro-2,3,8,8-tetramethyl), Methylnonylketon, Methylcyclocitron, Methyllavendelketon, Orivon (4-tert-Amylcyclohexanon), 4-tert-Butylcyclohexanon, Delphon (2-Pentylcyclopentanon), Muscon (CAS 541 -91 -3), Neobutenon (1-(5,5-dimethyl-1-cyclo-hexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on, Tetrameran (6,10-dimethylundecen-2-on) und Mischungen davon.

Die Kernmaterialien können ferner auch natürliche Duftstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Weitere herkömmliche Duftstoffe, die im Rahmen der vorliegenden Erfindung in den erfindungsgemäßen Mikrokapseln enthalten sein können, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Copaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wntergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α -Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iran, Isoeugenol, Isoeugenolmethyl-ether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-ßnaphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octyl-aldehyd, p-Oxy-Acetophenon, Pentadekanolid, β-Phenylethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanillin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Es ist erfindungsgemäß besonders bevorzugt, wenn in den beschriebenen Mikrokapseln Parfumzusammensetzungen verkapselt sind, die bezogen auf das Gesamtgewicht aller in der verkapselten Parfümzusammensetzung enthaltenen Riechstoffe umfassen:
a) ≤10 Gew.-%, vorzugsweise ≤8 Gew.-%, an Riechstoffen mit einem CLogP von ≤2,5 und einem Siedepunkt von ≥200°C;
b) ≥15 Gew.-%, vorzugsweise ≥20 Gew.-%, von mindestens einem Riechstoff mit einem CLogP von ≥ 4,0 und einem Siedepunkt von ≤275°C; und
c) ≥30 Gew.-%, vorzugsweise ≥40 Gew.-%, von mindestens einem Riechstoff mit einem Dampfdruck von ≥5 Pa bei 20°C

Der CLogP Wert ist der Flüssig-Flüssig-Verteilungskoeffizient für das System n-Octanol-Wasser und ein Maß für das Verhältnis zwischen Lipophilie und Hydrophilie einer Substanz. Ein Wert größer als 1 bezeichnet einen eher lipophilen Stoff, ein Wert unter 1 einen Stoff, der besser in Wasser als in n-Octanol löslich ist. Der ClogP-Wert kann mit geeigneten Programmen, die kommerziell erhältlich sind, für jede Substanz berechnet werden. Falls nicht anders angegeben, werden die hierin genannte Werte mit dem Programm EPI SUITE^{™} (v4.11) mit dem Modul KOWWIN^{™} v1.68 bestimmt.

Der Siedepunkt wurde, sofern nicht anders angegeben, mit dem Programm EPI SUITE^{™} (v4.11) mit dem Modul MPBPWIN v.1.43 (adapted Stein and Brown Method) bestimmt.

Der Dampfdruck bei 20°C wurde, sofern nicht anders angegeben, mit dem Programm EPI SUITE^{™} (v4.11) mit dem Modul MPBPWIN v.1.43 (modified grain method) bestimmt.

Beispiele für die Duftstoffe der Gruppe a) sind, ohne darauf beschränkt zu sein:

| **Riechstoff** | **CAS-Nummer** | **CLoqP** | **Siedepunkt [°C]** |
|---|---|---|---|
| Dulcinyl | 55418-52-5 | 2,03 | 295.74 |
| Furaneolmethylether | 4077-47-8 | 0,62 | 214.32 |
| Methylcorylone | 13494-06-9 | -0,12 | 219.34 |
| Tonkanyle ii | 874-90-8 | 1,62 | 230.92 |
| 2-Amino-1.3-propanediol serinol | 534-03-2 | -1,55 | 216.16 |
| 2.3-Diethylpyrazine | 15707-24-1 | 2,02 | 209.41 |
| 3-(Methylthio)-1-hexanol | 51755-66-9 | 1,84 | 225.06 |
| Acetanisol | 100-06-1 | 1,75 | 229.45 |
| Acetate pa | 7493-74-5 | 2,46 | 268.49 |
| Aldehyd c 18 sog | 104-61-0 | 2,08 | 265.5 |
| Allylamylglycolat | 67634-00-8 | 2,34 | 217.66 |
| Anisaldehyd | 123-11-5 | 1,79 | 221.63 |
| Anisylacetat | 104-21-2 | 2,16 | 252.81 |
| Benzylacetat | 140-11-4 | 2,08 | 215.57 |
| Benzylaceton | 2550-26-7 | 1,96 | 228.74 |
| Benzylalkohol | 100-51-6 | 1,08 | 205.65 |
| Bicyclononalactone | 4430-31-3 | 1,55 | 271.33 |
| Birolan | 25322-68-3 | -2,3 | 353.51 |
| Buccoxime | 75147-23-8 | 2,2 | 264.34 |
| Calone | 28940-11-6 | 2,43 | 296.5 |
| Corps eglantine | 64988-06-3 | 2,35 | 231.03 |
| Corylon | 80-71-7 | 1,29 | 241.48 |
| Cumarin | 91-64-5 | 1,51 | 290.74 |
| Dmbc | 100-86-7 | 2,44 | 228.78 |
| Ethylfruitat | 6413-10-1 | 1,3 | 217.74 |
| Ethylmaltol | 4940-11-8 | 0,3 | 283.37 |
| Ethylvanillin | 121-32-4 | 1,55 | 290.04 |
| Florol | 63500-71-0 | 2,16 | 229.55 |
| Frambinonmethylether | 104-20-1 | 2,04 | 264.7 |
| Guaiacol | 90-05-1 | 1,34 | 211.43 |
| Heliotropin | 120-57-0 | 1,77 | 256.96 |
| Homofuronol | 27538-09-6 | 1,31 | 275.21 |
| Hydratropaald.dim.acetal | 90-87-9 | 2,35 | 227.23 |
| Hydratropaaldehyd | 93-53-8 | 1,96 | 209.32 |
| Hydroxycitronellal rein | 107-75-5 | 2,11 | 241.19 |
| Hydrozimtalkohol | 122-97-4 | 2,06 | 243.15 |
| Indocolore | 2206-94-2 | 2,49 | 227.9 |
| Indoflor | 18096-62-3 | 1,84 | 264.36 |
| Indol | 120-72-9 | 2,05 | 250.04 |
| Isobutavan | 20665-85-4 | 2,3 | 314.04 |
| Koumalactone | 92015-65-1 | 1,89 | 276.66 |
| Kresylacetat (para) | 140-39-6 | 1,14 | 215.56 |
| Liffarome | 67633-96-9 | 2,47 | 213 |
| Linalool oxyde | 1365-19-1 | 2,08 | 232.9 |
| Maltol | 118-71-8 | -0,19 | 267.24 |
| Maltyl isobutyrat | 65416-14-0 | 1,7 | 286.06 |
| Menoxaline | 13708-12-8 | 1,66 | 265.62 |
| Methoxymelonal | 62439-41-2 | 2,32 | 205.16 |
| Methyl cinnamic alcohol | 1504-55-8 | 2,39 | 261.14 |
| Methyl diantilis | 5595-79-9 | 1,61 | 282.35 |
| Methyl octalactone | 39212-23-2 | 2 | 260.63 |
| Methylacetophenon para | 122-00-9 | 2,22 | 209.72 |
| Methylanthranilat | 134-20-3 | 2,26 | 263.57 |
| Methylcinnamat | 103-26-4 | 2,36 | 239.9 |
| Methylphenylacetat | 101-41-7 | 2,08 | 215.57 |
| Methylserinol | 115-69-5 | -1,1 | 227.56 |
| Methylzimtaldehyd (alpha) | 101-39-3 | 2,37 | 240.02 |
| Octalacton delta | 698-76-0 | 1,59 | 249.98 |
| Octalactone gamma | 104-50-7 | 1,59 | 248.37 |
| Orcinyl 3 | 3209-13-0 | 2,14 | 230.36 |
| Oxane 969380 | 59323-76-1 | 2,35 | 209.28 |
| Oxyphenylon | 5471-51-2 | 1,48 | 280.39 |
| Phenoxyethylalkohol | 122-99-6 | 1,1 | 243.84 |
| Phenylacetaldehyd | 122-78-1 | 1,54 | 201.51 |
| Phenylacetaldehyd-glycerinacetal | 29895-73-6 | 0,77 | 312.53 |
| Phenylacetaldehyddimethylacetal | 101-48-4 | 1,93 | 219.76 |
| Phenylessigsäure | 103-82-2 | 1,43 | 266.58 |
| Phenylethylalkohol | 60-12-8 | 1,57 | 224.85 |
| Phenylethylformiat | 104-62-1 | 2,02 | 217.34 |
| Plicatone | 41724-19-0 | 2,45 | 259.49 |
| Propylfruitat | 6290-17-1 | 1,72 | 231.07 |
| Styrolylacetat | 93-92-5 | 2,5 | 223.12 |
| Sulfurol (4-methyl-5-thiazol ethanol) | 137-00-8 | 1,11 | 259.88 |
| Sulfurol milkv | 137-00-8 | 1,11 | 259.88 |
| Toluylaldehyd (para) | 104-87-0 | 2,26 | 201.5 |
| Trifernal | 16251-77-7 | 2,45 | 228.35 |
| Vanillin | 121-33-5 | 1,05 | 274.3 |
| Vetikolacetat | 68083-58-9 | 1,68 | 275.57 |
| Zimtaldehyd | 104-55-2 | 1,82 | 226.69 |
| Zimtalkohol | 104-54-1 | 1,84 | 248.6 |
| Diantheme | 4125-43-3 | 2,48 | 229.65 |
| Hydratropaalkohol | 1123-85-9 | 1,98 | 232.23 |
| Hydroxicitronellal synth. | 107-75-5 | 2,11 | 241.19 |
| Salicylaldehyd | 90-02-8 | 2,01 | 239.42 |
| Trivalon | 71566-51-3 | 1,39 | 241.44 |
| Anisalkohol | 105-13-5 | 1,16 | 243.83 |

Beispiele für Duftstoffe der Gruppe b) sind, ohne darauf beschränkt zu sein:

| **Riechstoff** | **CAS-Nummer** | **CLoqP** | **Siedepunkt [°C]** |
|---|---|---|---|
| aldehyd c 11 | 112-44-7 | 4,25 | 234.81 |
| aldehvd c 11.(en) | 112-45-8 | 4,12 | 233.44 |
| aldehyd c 12 | 112-54-9 | 4,75 | 252.62 |
| aldehyd c 12.mna | 110-41-8 | 4,67 | 241.99 |
| aldehyd supra | 143-14-6 | 4,04 | 240.39 |
| alkohol c 12 | 112-53-8 | 4,77 | 272.96 |
| allylcyclohexylpropionat | 2705-87-5 | 4,47 | 254.19 |
| azarbre | 68845-36-3 | 4,11 | 259.5 |
| boisiris | 68845-00-1 | 5,44 | 256.67 |
| caryophyllene beta | 87-44-5 | 6,3 | 256.8 |
| cedramber | 19870-74-7 | 5,03 | 265.3 |
| cetonal | 73398-85-3 | 5,2 | 270.68 |
| cis-3-hexenyl-2-methylbutyrat | 53398-85-9 | 4,01 | 224.17 |
| citronellyl butyrate | 141-16-2 | 5,54 | 272.03 |
| citronellyl formate | 105-85-1 | 4,01 | 220.77 |
| citronellyl isobutyrate | 97-89-2 | 5,47 | 262.03 |
| citronellylacetat | 150-84-5 | 4,56 | 237.59 |
| citronellylpropionat | 141-14-0 | 5,05 | 255.26 |
| coniferan pure | 67874-72-0 | 4,91 | 250.47 |
| cyclomyral | 68991-96-8 | 4,38 | 271.8 |
| cymol (para) =cymene | 99-87-6 | 4 | 178.34 |
| damarose alpha | 43052-87-5 | 4,29 | 259.84 |
| damascenon bm | 23696-85-7 | 4,21 | 265.13 |
| damascenone | 23696-85-7 | 4,21 | 265.13 |
| damascone alpha | 43052-87-5 | 4,29 | 259.84 |
| damascone beta | 35044-68-9 | 4,42 | 262.93 |
| decylacetat | 112-17-4 | 4,79 | 247.73 |
| dihydro-beta-jonon | 17283-81-7 | 4,35 | 257.7 |
| dihydrofloriffone td | 57378-68-4 | 4,16 | 255.99 |
| dihydroterpinylacetat=menthanylacetat | 58985-18-5 | 4,42 | 232.55 |
| diphenylether | 101-84-8 | 4,05 | 269.66 |
| dynascone pur | 56973-85-4 | 4,45 | 257.9 |
| ethyl damascenate | 35044-59-8 | 4,25 | 247.88 |
| ethyl-2-4-decadienoate | 3025-30-7 | 4,36 | 258.41 |
| ethyllinalylacetat | 61931-80-4 | 4,88 | 247.05 |
| ethylsafranat | 35044-59-8 | 4,25 | 247.88 |
| fleuramon | 137-03-1 | 4 | 263.53 |
| floral super | 71077-31-1 | 4,38 | 241.28 |
| floramat | 67801-64-3 | 4,77 | 228.23 |
| folenox | 26619-69-2 | 4,38 | 247.81 |
| frutonile | 69300-15-8 | 4,2 | 250.43 |
| galbanolene super | 16356-11-9 | 5,18 | 196.6 |
| galbascone bht captiv | 56973-85-4 | 4,45 | 257.9 |
| geraldehyde | 762-26-5 | 4,43 | 253.84 |
| geranylisobutyrat | 2345-26-8 | 5,38 | 272.6 |
| geranylpropionat | 105-90-8 | 4,97 | 266.06 |
| givescone | 57934-97-1 | 4,83 | 259.38 |
| gyrane | 24237-00-1 | 4,07 | 214.86 |
| herbanate | 116044-44-1 | 4,13 | 254.37 |
| herbavert | 67583-77-1 | 4,12 | 193.28 |
| hexvlhexanoat | 6378-65-0 | 4,79 | 247.73 |
| irone alpha | 79-69-6 | 4,71 | 271.32 |
| isoamylhexanoate | 2198-61-0 | 4,23 | 218.34 |
| isobornylisobutyrat | 85586-67-0 | 4,77 | 251.13 |
| isodamascone | 33673-71-1 | 4,29 | 259.84 |
| isononylacetat (neononylacetat) | 58430-94-7 | 4,12 | 198.85 |
| jonon beta synth. | 14901-07-6 | 4,42 | 262.93 |
| jonon rein 100 | 127-41-3 | 4,29 | 259.48 |
| koavone | 86115-11-9 | 4,19 | 207.93 |
| linalylacetat | 115-95-7 | 4,39 | 228.95 |
| mandarinenaldehyd | 20407-84-5 | 4,53 | 257.92 |
| melusat | 67707-75-9 | 4,12 | 198.85 |
| menthylacetat-rf | 89-48-5 | 4,39 | 234.5 |
| mercapto-8-menthene-1 para | 71159-90-5 | 4,74 | 224.11 |
| methvl ionone qamma coeur #1 | 127-51-5 | 4,84 | 271.6 |
| methyloctylacetaldehyd (moa) | 19009-56-4 | 4,18 | 223.64 |
| myrcenylacetat | 1118-39-4 | 4,47 | 221.78 |
| neobutenone alpha | 56973-85-4 | 4,45 | 257.9 |
| nerylacetat | 141-12-8 | 4,48 | 248.97 |
| nerylpropionat | 105-91-9 | 4,97 | 266.06 |
| nopylacetat | 128-51-8 | 4,3 | 259.16 |
| ocimenylacetat | 72214-23-4 | 4,39 | 228.95 |
| otbca | 88-41-5 | 4,42 | 232.55 |
| ozofleur | 181258-89-9 | 5,1 | 228.54 |
| peranat | 90397-38-9 | 4,65 | 225.84 |
| phellandrene alpha extra | 99-83-2 | 4,62 | 165.01 |
| phenylethylisoamylether | 56011-02-0 | 4,16 | 255.27 |
| pinane | 473-55-2 | 4,35 | 149.9 |
| pinen beta | 127-91-3 | 4,35 | 150.8 |
| pinene alpha | 80-56-8 | 4,27 | 157.25 |
| poirenate | 2511-00-4 | 4,04 | 226.74 |
| poivrol | 68966-86-9 | 4,62 | 259.95 |
| ptbca 25 cis | 32210-23-4 | 4,42 | 232.55 |
| ptbca 40 cis | 32210-23-4 | 4,42 | 232.55 |
| sandalore | 65113-99-7 | 5,15 | 273.81 |
| tangerinal 10 dipg semi-captive | 21944-98-9 | 4,53 | 257.92 |
| terpinen gamma | 99-85-4 | 4,75 | 169.36 |
| terpinene alpha p&f | 99-86-5 | 4,75 | 169.36 |
| terpinolen | 586-62-9 | 4,88 | 178.17 |
| terpinvl methyl ether 90 | 14576-08-0 | 4,03 | 196.38 |
| tetrameran | 1322-58-3 | 4,44 | 250.01 |
| thesaron | 22471-55-2 | 4,42 | 235.87 |
| trithioacetone | 828-26-2 | 7,6 | 269.84 |
| troenan | 80480-24-6 | 4,35 | 253.14 |
| undecavertol | 81782-77-6 | 4,05 | 240.58 |
| undecvlenalkohol alkohol c 11 en | 112-43-6 | 4,14 | 254.95 |
| veloutone | 65443-14-3 | 4,34 | 257.68 |
| violette feuilles non dec. | 06.08.8024 | 4,29 | 259.48 |
| violettyne mip 991805 | 166432-52-6 | 4,68 | 204.87 |

Beispiele für Duftstoffe der Gruppe c) sind, ohne darauf beschränkt zu sein:

| **Riechstoff** | **CAS-Nummer** | **Dampfdruck[Pa] 20°C** |
|---|---|---|
| linalool | 78-70-6 | 6.946 |
| peelessenz | 78-70-6 | 6.946 |
| dihydromvrcenol | 53219-21-9 | 6.986 |
| indocolore semi-captive | 2206-94-2 | 7.586 |
| ethylphenylacetat | 101-97-3 | 7.946 |
| citral ar | 5392-40-5 | 7.946 |
| citral n | 5392-40-5 | 7.946 |
| menthylacetat-rf | 89-48-5 | 7.946 |
| poirenate | 2511-00-4 | 8.066 |
| ethylacetat | 141-78-6 | 10265 |
| livescone | 3720-16-9 | 10.292 |
| decenal-4-trans | 65405-70-1 | 10.385 |
| myrcenylacetat | 1118-39-4 | 10.492 |
| orivone | 16587-71-6 | 10.732 |
| methylheptincarbonat | 111-12-6 | 11.052 |
| citronellyl formate | 105-85-1 | 11.065 |
| ozofleur | 181258-89-9 | 11.479 |
| carvon I | 6485-40-1 | 11.505 |
| linalylacetat | 115-95-7 | 11.519 |
| benzylpropionat | 122-63-4 | 11.519 |
| montaverdi | 188570-78-7 | 11.585 |
| phenylacetaldehyddimethylacetal | 101-48-4 | 11.665 |
| firascone | 815580-59-7 | 11.692 |
| delphone | 4819-67-4 | 11.705 |
| cyclohexylethylacetat | 21722-83-8 | 11.985 |
| sclareolate | 319002-92-1 | 12.079 |
| centifolether | 120811-92-9 | 12.132 |
| bergamal | 22418-66-2 | 12.998 |
| allylamylglycolat | 67634-00-8 | 13.025 |
| phenylethylformiat | 104-62-1 | 13.252 |
| mintonat | 67859-96-5 | 13.332 |
| neofolione | 111-79-5 | 13.732 |
| cis-3-hexenyl butyrate | 16491-36-4 | 13.732 |
| methylphenylacetat | 101-41-7 | 13.865 |
| 9-decenal | 39770-05-3 | 15.198 |
| isocyclocitral | 1335-66-6 | 15.465 |
| benzylacetat | 140-11-4 | 16.665 |
| aphermate | 25225-08-5 | 16.665 |
| liffarome | 67633-96-9 | 16.665 |
| kresylacetat (para) | 140-39-6 | 17.065 |
| linalylformiat | 115-99-1 | 17.731 |
| dihydro anethol | 104-45-0 | 17.998 |
| freskomenthe | 14765-30-1 | 19.065 |
| octylacetat | 112-14-1 | 19.465 |
| allylheptanoat - allyloenanthat | 142-19-8 | 20.265 |
| amyl vinyl carbinol | 3391-86-4 | 20.398 |
| nonadienal | 557-48-2 | 20.798 |
| ethylcaprylat | 106-32-1 | 21.064 |
| aldehyd c 10 | 112-31-2 | 21.064 |
| hexylbutyrat | 2639-63-6 | 21.598 |
| citronellal | 106-23-0 | 22.664 |
| gyrane | 24237-00-1 | 22.931 |
| claritone | 74338-72-0 | 24.797 |
| methoxvmelonal | 62439-41-2 | 24.931 |
| hexenyl-cis-3-isobutyrat | 41519-23-7 | 25.331 |
| toluylaldehyd (para) | 104-87-0 | 25.864 |
| hydratropaaldehyd | 93-53-8 | 27.197 |
| acetophenon | 98-86-2 | 29.464 |
| 2-isopropyl-4-methylthiazole | 15679-13-7 | 29.864 |
| dimetol | 13254-34-7 | 31.597 |
| sultanene captive | 15848-49-4 | 31.597 |
| koavone | 86115-11-9 | 32.264 |
| methylbenzoat | 93-58-3 | 34.263 |
| damascia (frutinat) | 35206-51-0 | 46.796 |
| tetrahydrocitral | 5988-91-0 | 48.129 |
| phenylethylmethylether | 3558-60-9 | 50.129 |
| cuminaldehyd | 122-03-2 | 50.529 |
| aldehyd c 09 | 124-19-6 | 51.462 |
| dimethylsulfid | 75-18-3 | 52129 |
| aldehyd c 11 | 112-44-7 | 52.395 |
| amylvalerianat, iso-iso- | 659-70-1 | 53.062 |
| tetrahydrolinalool | 78-69-3 | 53.728 |
| lilessenz | 78-69-3 | 53.728 |
| octalactone gamma | 104-50-7 | 54.662 |
| galbanolene super | 16356-11-9 | 56.128 |
| benzylaceton | 2550-26-7 | 56.128 |
| aldehyd c 11.(en) | 112-45-8 | 56.395 |
| toscanol | 16510-27-3 | 56.395 |
| otbca | 88-41-5 | 56.662 |
| agrunitril | 51566-62-2 | 57.195 |
| methylpamplemousse | 67674-46-8 | 57.861 |
| amarocit | 67674-46-8 | 57.861 |
| geranylformiat | 105-86-2 | 58.795 |
| phenylethylacetat | 103-45-7 | 58.928 |
| methyloctincarbonat | 111-80-8 | 59.061 |
| ptbca 40 cis | 32210-23-4 | 59.195 |
| ptbca 25 cis | 32210-23-4 | 59.195 |
| dihydroterpinylacetat=menthanylacetat | 58985-18-5 | 59.195 |
| fenchylacetat | 13851-11-1 | 59.328 |
| velberry | 13431-57-7 | 59.999 |
| rosenoxid r | 16409-43-1 | 60.795 |
| rosenoxid I | 3033-23-6 | 60.795 |
| propylfruitat | 6290-17-1 | 61.061 |
| ethylheptanoat | 106-30-9 | 63.061 |
| corps eglantine | 64988-06-3 | 64.128 |
| isoamyl-2-methylbutyrat | 27625-35-0 | 64.261 |
| 2.3 diethylpyrazine | 15707-24-1 | 64.528 |
| irolene p | 29127-83-1 | 64.661 |
| dimethyloctenon | 2550-11-0 | 65.327 |
| cyclopidene | 40203-73-4 | 65.327 |
| allylcapronat | 123-68-2 | 66.261 |
| methylacetophenon para | 122-00-9 | 67.194 |
| hydrochinondimethylaether | 150-78-7 | 67.194 |
| 2-cyclopentyl cyclopentanone | 4884-24-6 | 68.394 |
| dibutyl sulphide | 544-40-1 | 69.727 |
| ocimenylacetat | 72214-23-4 | 71.727 |
| trifernal | 16251-77-7 | 73.993 |
| 4-methylthio-4-methyl-2-pentanone | 23550-40-5 | 81.326 |
| citrathal | 147060-73-9 | 82.659 |
| citral dimethylacetal | 7549-37-3 | 82.659 |
| alkohol c 08 | 111-87-5 | 83.059 |
| hexenol (beta gamma) | 928-96-1 | 83.459 |
| octanol-3 | 589-98-0 | 83.593 |
| isobornylacetat | 125-12-2 | 85.059 |
| acetessigester | 141-97-9 | 85.992 |
| givescone | 57934-97-1 | 87.326 |
| guaiacol | 90-05-1 | 88.526 |
| isopentyrat | 80118-06-5 | 91.992 |
| terpinolen | 586-62-9 | 93.592 |
| benzaldehyd rein | 100-52-7 | 93.992 |
| methyloctylacetaldehyd | 19009-56-4 | 95.058 |
| vivaldie | 292605-05-1 | 95.458 |
| tetrahydromyrcenol | 18479-57-7 | 95.725 |
| styrolylacetat | 93-92-5 | 97.725 |
| methylnonylketon | 112-12-9 | 98.791 |
| hexenylacetat | 3681-71-8 | 106.12 |
| terpinen gamma | 99-85-4 | 108.12 |
| kresvlmethvlether (para) | 104-93-8 | 111.85 |
| orrisate | 816-19-3 | 133.32 |
| hexvlacetat | 142-92-7 | 135.98 |
| aldehyd c 12.mna | 110-41-8 | 139.98 |
| aldehyd c 08 | 124-13-0 | 139.98 |
| myroxide | 69103-20-4 | 143.98 |
| ethylbutyrat | 105-54-4 | 1453.2 |
| 2.6-dimethylpyrazin | 108-50-9 | 147.98 |
| amylpropionat | 624-54-4 | 157.32 |
| butylbutyrate | 109-21-7 | 165.31 |
| methylheptenon | 110-93-0 | 169.31 |
| ethylcapronat | 123-66-0 | 169.31 |
| methylhexylketon | 111-13-7 | 177.31 |
| acetoin | 513-86-0 | 181.31 |
| phellandrene alpha extra | 99-83-2 | 181.31 |
| isobutylacetate | 110-19-0 | 1826.5 |
| limetol | 7392-19-0 | 191.98 |
| cerezoate | 1617-23-8 | 198.65 |
| neoproxen | 31996-78-8 | 198.65 |
| cyclamenaldehyd extra l.g. | 103-95-7 | 2053.1 |
| pinane | 473-55-2 | 209.31 |
| isoamylpropionat | 105-68-0 | 235.98 |
| pinen beta p&f | 127-91-3 | 239.98 |
| ethylisobutyrat | 97-62-1 | 2439.7 |
| hexenyl-3-formiat | 33467-73-1 | 273.31 |
| methylbutyl-2 propionate | 2438-20-2 | 274.64 |
| manzanate | 39255-32-8 | 274.64 |
| phenylacetaldehyd | 122-78-1 | 28.93 |
| ethyl tiglate | 5837-78-5 | 289.3 |
| triplal | 27939-60-2 | 31.73 |
| diethylmalonat | 105-53-3 | 32.53 |
| menthon | 14073-97-3 | 33.73 |
| isomenthone | 491-07-6 | 33.73 |
| ethylamylketon | 541-85-5 | 331.97 |
| aldehyd c 07 | 111-71-7 | 337.3 |
| isononylacetat (neononylacetat) | 58430-94-7 | 34.53 |
| rumacetal | 1670-47-9 | 36.13 |
| 2-isobutylthiazole | 18640-74-9 | 36.13 |
| pinene alpha | 80-56-8 | 390.63 |
| prenylacetate | 1191-16-8 | 401.3 |
| isobutyl isobutyrate | 97-85-8 | 411.96 |
| hexenal-2-trans | 6728-26-3 | 455.96 |
| ethylvalerat | 539-82-2 | 463.96 |
| isopropyl methyl-2-butyrate | 66576-71-4 | 517.29 |
| isovaleraldehyd | 590-86-3 | 5306.2 |
| melonal | 106-72-9 | 69.86 |
| hexanol | 111-27-3 | 78.26 |
| ethyl-2-methylbutyrat | 7452-79-1 | 786.6 |
| aldehyd c 06 | 66-25-1 | 941.25 |

Vorzugsweise werden Mischungen von Riechstoffen eingesetzt, zum Beispiel mindestens zwei oder mehr verschiedene Riechstoffe der Gruppen b) und/oder mindestens zwei oder mehr verschiedene Riechstoffe der Gruppe c).

In verschiedenen Ausführungsformen der Erfindung kann der Anteil der Riechstoffe der Gruppe a) auch kleiner als 6, kleiner als 5, kleiner als 4, kleiner als 3, kleiner als 2 oder kleiner als 1 Gew.-% sein. In verschiedenen Ausführungsformen kann die Parfümzusammensetzung keine Riechstoffe der Gruppe a) enthalten. In alternativen Ausführungsformen enthält die Parfümzusammensetzung Riechstoffe der Gruppe a), allerdings in Mengen unter den hier angegebenen Obergrenzen.

In verschiedenen Ausführungsformen können die Riechstoffe der Gruppen b) und c) gemeinsam mindestens 60, vorzugsweise mindestens 65, mindestens 70, mindestens 75, mindestens 80, mindestens 85 oder mindestens 90 Gew.-% der Gesamtriechstoffe der Parfümzusammensetzung ausmachen.

Die hierin angegebenen Mengenangaben beziehen sich auf die Summe aller Riechstoffe in der verkapselten Parfümzusammensetzung, sofern nicht anders angegeben. Alternativ können sie sich auch auf das Gesamtgewicht der verkapselten Parfümzusammensetzung beziehen, beispielsweise wenn die Formulierungshilfsmittel enthält.

Zusätzlich zu den oben genannten Duftstoffen der Gruppen a)-c) können weitere Duftstoffe als Bestandteile der verkapselten Parfümzusammensetzung eingesetzt werden, solange die Merkmale a)-c) erfüllt sind. Diese zusätzlichen Duftstoffen sind keinen besonderen Beschränkungen unterworfen.

Hierin wurde die Dichtheit der Mikrokapseln für das Duftöl Weiroclean der Fa. Kitzing bestimmt. Weiroclean weist die folgenden Komponenten auf (mit Anteil bezogen auf das Gesamtgewicht):

| | |
|---|---|
| 1-(1,2,3,4,5,6,7,8-Octahydro-2,3,8,8-tetramethyl-2-naphthalenyl)ethanon | 25-50 % |
| Benzoesäure, 2-hydroxy-, 2-hexyl ester | 10-25 % |
| Phenylmethylbenzoat | 5-10% |
| 3-Methyl-4-(2,6,6-trimethyl-2-cyclohexenyl)-3-buten-2-on | 1-5 % |
| 3,7-Dimethyl-6-octen-1-ol | 1-5 % |
| 3-Methyl-5-phenylpentanol | 1-5 % |
| 2,6-Dimethyloct-7-en-2-ol | 1-5 % |
| 4-(2,6,6-Trimethylcyclohex-1-eneyl)-but-3-ene-2-on | 1-5 % |
| 3a,4,5,6,7,7a-Hexahydro-4,7-methano-1H-inden-6-yl-propanoat | 1-5 % |
| 2-tert-Butylcyclohexylacetat | 1-5 % |
| 2-Heptylcyclopentanon | 1-5 % |
| Pentadecan-15-olid | 1-5 % |
| 2H-1-Benzopyran-2-on | 0,1-1 % |
| 2,6-Di-tert-butyl-p-cresol | 0,1-1 % |
| 4-Methyl-3-decen-5-ol | 0,1-1 % |
| 2,4-Dimethyl-3-cyclohexen-1-carboxaldehyd | 0,1-1 % |
| [(2E)-3,7-Dimethylocta-2,6-dienyl] acetat | 0,1-1 % |
| Allylhexanoat | 0,1-1 % |
| 2-Methylundecanal | 0,1-1 % |
| 10-Undecenal | 0,1-1 % |
| cis-3,7-Dimethyl-2,6-octadienylethanoat | 0,1-1 % |
| 3,7,11-Trimethyldodeca-1,6,10-trien-3-ol | 0,1-1 % |
| Undecan-2-on | 0,1-1 % |

Gemäß einer bevorzugten Ausführungsform enthält das Kernmaterial kein Monosaccharid-Polyol, insbesondere kein Mannitol, Erythritol, Xylitol, Sorbitol oder deren Mischungen.

### Mikrokapsel-Slurrv

Die hierin beschriebenen Mikrokapseln werden typischerweise in Form einer Dispersion, die auch als Slurry bezeichnet wird, vorformuliert. Dazu werden die Kapseln in ein wässriges Medium dispergiert, um eine Aufschlämmung der Kapseln in dem flüssigen Medium zu bewirken.

Erfindungsgemäß umfasst der Begriff "Dispersion" bzw. "Mikrokapsel-Dispersion" auch Suspensionen und Slurries wie hierin beschrieben.

Der Begriff "Slurry" bezeichnet im Kontext der vorliegenden Erfindung eine, typischerweise wässrige, Aufschlämmung der Parfümmikrokapseln, wie voranstehend definiert. Das flüssige Medium (kontinuierliche Phase) besteht vorzugsweise zum überwiegenden Anteil, d.h. zu mehr als 50 Gew.-% aus Wasser, beispielsweise zu mehr als 60, mehr als 70 oder mehr als 80 Gew.-%, kann aber auch nahezu oder vollständig, d.h. zu 90 Gew.-%, 95 Gew.-% oder mehr aus Wasser bestehen. Der Slurry ist vorzugsweise gießbar, d.h. er lässt sich aus einem Gefäß durch Neigen des Gefäßes ausgießen. Unter einem gießbaren Slurry wird insbesondere ein Kapsel-Flüssigkeitsgemisch verstanden, welches insbesondere bei der Verarbeitungstemperatur, vorzugsweise bei maximal 40 °C, insbesondere bei maximal 20 °C eine Viskosität unterhalb von 10⁴ mPas, vorzugsweise unterhalb von 10³ mPas (Brookfield- Rotationsviskosimeter; Spindel 2, 20 U/min.) aufweist.

Der Slurry kann neben dem erfindungsgemäß eingesetzten Emulgator weitere Hilfsstoffe enthalten, beispielsweise solche, die eine bestimmte Haltbarkeit oder Stabilität sicherstellen. Häufig verwendete Hilfsstoffe schließen beispielsweise Tenside, insbesondere anionische und/oder nichtionische Tenside ein, die von dem erfindungsgemäß eingesetzten Emulgator verschieden sind.

Wie bereits oben beschrieben werden in den Slurries der hierin beschriebenen Mikrokapseln Emulgatoren/Tenside aus der Klasse der ethoxylierten, hydrierten Rizinusöle als Additive eingesetzt (INCI: ethoxylated hydrogenated castor oil), insbesondere solche mit 20 bis 60, 30 bis 50 oder etwa 40 EO. Letztere sind auch als PEG40-hydrogenated castor oil bekannt und kommerziell beispielsweise als Eumulgin^{®} HRE40 von BASF erhältlich. Diese Emulgatoren werden in den Slurries in Mengen von bis zu 50 Gew.-%, vorzugsweise bis zu 40 Gew.-%, bis zu 30 Gew.-% oder bis zu 20 Gew.-%, besonders bevorzugt mit maximal 10 Gew.-% eingesetzt, wobei typische Mengen im Bereich von mindestens 0,5 Gew.-%, mindestens 1 Gew.-% oder mindestens 2 Gew.-% liegen, insbesondere in Bereichen von 2 bis 10 Gew.-%, 3 bis 9 Gew.-% oder 4 bis 8 Gew.-% oder ungefähr 4, 5, 6 oder 7 Gew.-%. Wenn der Emulgator Bestandteil der kontinuierlichen Phase ist, enthält diese vorzugsweise mehr als 50 Gew.-% Wasser und die Summe aus Wasser und Emulgator macht vorzugsweise mindestens 70 Gew.-%, bevorzugter mindestens 80 Gew.-%, noch bevorzugter mindestens 90 Gew.-% der kontinuierlichen Phase aus. In verschiedenen Ausführungsformen besteht die kontinuierliche Phase aus Wasser und dem mindestens einen Emulgator.

In verschiedenen Ausführungsformen enthält die kontinuierliche Phase 60 bis 95 Gew.-%, vorzugsweise 70 bis 95 Gew.-% Wasser und 2 bis 40 Gew.-%, vorzugsweise 2 bis 20 Gew.-% des mindestens eines Emulgators.

Es wurde überraschenderweise gefunden, dass diese Emulgatoren die Slurries zu stabilisieren vermögen, wohingegen andere übliche Emulgatoren, wie beispielsweise Hydroxypropyl Guar (CAS 39421-75-5, beispielsweise Jaguar HP105), ethoxylierte C₁₂₋₁₈ Fettalkohole (wie z.B. Dehydol^{®} LT5) und ethoxylierte Sorbitanmonoester, wie beispielsweise Polyoxyethylen Sorbitanmonopalmitat (CAS 9005-66-7), keine ausreichende Stabilisierung bewirken konnten.

Obwohl der Einsatz weiterer als der erfindungsgemäßen Emulgatoren möglich ist, ist dieser erfindungsgemäß nicht bevorzugt. Insbesondere ist es bevorzugt, wenn die Mikrokapseldispersion kein Kaliumcetylphosphat enthält. Neben den Emulgatoren gemäß der Erfindung können allerdings andere Hilfsstoffe und Additive eingesetzt werden bei denen es sich nicht um Emulgatoren oder Tenside handelt. Gemäß einer bevorzugten Ausführungsform enthält die Zusammensetzung kein hydroxyliertes Diphenylmethanderivat.

In verschiedenen Ausführungsformen sind die vorangehend beschriebenen Kapseln in einer Menge von 1 bis 60 Gew.-% bezogen auf das Gesamtgewicht der Mikrokapseldispersion enthalten. Die Mikrokapseln können beispielsweise in einer Menge von 2 Gew.-%, 4 Gew.-%, 6 Gew.-%, 8 Gew.-%, 10 Gew.-%, 12 Gew.-%, 14 Gew.-%, 16 Gew.-%, 18 Gew.-%, 20 Gew.-%, 22 Gew.-%, 24 Gew.-%, 26 Gew.-%, 28 Gew.-%, 30 Gew.-%, 32 Gew.-%, 34 Gew.-%, 36 Gew.-%, 38 Gew.-%, 40 Gew.-%, 42 Gew.-%, 44 Gew.-%, 46 Gew.-%, 48 Gew.-%, 50 Gew.-%, 52 Gew.-%, 54 Gew.-%, 56 Gew.-%, 58 Gew.-%, oder 60 Gew.-% enthalten sein. Gemäß einer Ausführungsform liegt der Anteil der Mikrokapseln im Bereich von 15 bis 50 Gew.-%. Gemäß einer bevorzugten Ausführungsform liegt der Anteil der Mikrokapseln im Bereich von 20 bis 35 Gew.-% in dem Slurry. Diese Dispersionen sind stabil, d.h. es kommt auch nach längeren Lagerungszeiträumen von beispielsweise mehreren Tagen bis Wochen bei üblichen Temperaturen im Bereich bis 40 °C, beispielsweise 4 Wochen bei einer Temperatur zwischen >0 und 40 °C, nicht zu einer Agglomeration, Sedimentation und/oder Aufschwimmen der Kapseln oder einer sonstigen Phasenseparation, wobei diese ausgeprägte Phasenstabilität auf den Einsatz des speziellen Emulgators zurückzuführen ist.

Bei der Herstellung dieser Slurries werden die Mikrokapseln typischerweise mit geeigneten Mitteln in eine wässrige kontinuierliche Phase, die bereits den erfindungsgemäß eingesetzten Emulgator enthält, dispergiert.

Die phasenstabilisierende Wirkung tritt in einem breiten pH-Bereich auf. Insbesondere kommt die phasenstabilisierende Wirkung des Emulgators bei einem nicht stark basischen pH-Wert zum Tragen. Gemäß einer Ausführungsform liegt der pH der Mikrokapsel-Dispersion nach Zugabe des Emulgators bei weniger als 11, beispielsweise kann der pH-Wert der Mikrokapsel-Dispersion bei 10,8, 10,5, 10,3, 10,0, 9,8, 9,5, 9,3, 9,0, 8,8, 8,5, 8,3, 8,0, 7,8, 7,5, 7,3, 7,0, 6,8, 6,5, 6,3, oder 6,0 liegen. Die Mikrokapseldispersion ist in der Regel basisch. Der pH-Wert der Mikrokapsel-Dispersion kann bei weniger als 10,8, bevorzugt bei höchstens 10,5 liegen. Der pH-Wert der Mikrokapsel-Dispersion ist in einer Ausführungsform bei mindestens 6, bevorzugt mindestens 7 und besonders bevorzugt bei mindestens 8.

Die phasenstabilisierende Wirkung tritt in einem breiten Leitfähigkeitsbereich auf. Die Leitfähigkeit der Mikrokapsel-Dispersion kann bei mindestens 6,0 mS/cm liegen. Beispielsweise kann die Leitfähigkeit bei 6,0 mS/cm, 6,5 mS/cm, 7,0 mS/cm, 7,5 mS/cm, 8,0 mS/cm, 8,5 mS/cm, 9,0 mS/cm, oder 10 mS/cm, 10,5 mS/cm, 11 mS/cm, 11,5 mS/cm, 12 mS/cm, 12,5 mS/cm, 13,0 mS/cm, 13,5 mS/cm, 14 mS/cm, 14,5 mS/cm oder 15,0 mS/cm liegen. Gemäß einer Ausführungsform liegt die Leitfähigkeit der Mikrokapsel-Dispersion im Bereich von 6,0 mS/cm bis 15,0 mS/cm, bevorzugt im Bereich von 8 mS/cm bis 12 mS/cm, besonders bevorzugt im Bereich von 9 mS/cm bis 11 mS/cm.

Für die Messungen des pH-Werts sowie der elektrischen Leitfähigkeit des Mittels oder der Mikrokapsel-Dispersion kann das Kombigerät pH/Cond 3320 der Fa. WTW verwendet werden. Dieses wird mit einer pH-Elektrode Modell "Inlab Expert" (Order Nummer: 5343103) der Fa. Mettler Toledo sowie einer Leitfähigkeitselektrode Modell "Tetra Con 325" der Fa. WTW ausgerüstet. Die Glasmembran der pH-Elektrode lagert im ungenutzten Zustand in 3M KCl Lösung. Durch die regelmäßige Kalibration der beiden Elektroden ist eine Messunsicherheit von ca. +/- 0,01 sowie +/- 0,05 mS/cm sichergestellt. Sowohl pH- als auch Leitfähigkeitselektrode sind mit einem Temperatursensor versehen, sodass eine Temperaturkompensation der Messwerte möglich ist.

Zur Aufnahme des pH-Wertes kann die pH-Elektrode aus der entsprechenden 3M KCl Lagerlösung entnommen und mittels Leitungswasser gereinigt werden. Anschließend wird die Elektrode in die entsprechende Mikrokapsel-Dispersion eingetaucht, wobei sichergestellt ist, dass die vollständige Glasmembran der Elektrode eingetaucht wurde. Nach Stabilisierung des Messwerts wird nach ca. 5 min der Messwert abgelesen. Der angezeigte Messwert ist dimensionslos. Die Messungen werden in unverdünnten Mikrokapsel-Dispersionen durchgeführt. Zur Messung der elektrischen Leitfähigkeit wird die gereinigte Leitfähigkeitselektrode in die entsprechende Mikrokapsel-Dispersion eingetaucht. Hierbei wird sichergestellt, dass der eigentliche Messspalt der Elektrode vollständig eingetaucht wurde. Nach Stabilisierung des Messwerts wird nach ca. 5 min der temperaturkompensierte Messwert abgelesen. Der angezeigte Messwert besitzt die Einheit mS/cm.

### Getrocknete Mikrokapsel-Zusammensetzunq

In einer weiteren Ausführungsform kann die Mikrokapsel-Dispersion enthaltend den Emulgator auch als getrocknete Zusammensetzung, also als Pulvermischung vorliegen. Die getrocknete Mikrokapselzusammensetzung kann insbesondere durch Trocknung einer zuvor beschriebenen (flüssigen) Mikrokapsel-Dispersion erhalten werden. Für die Trocknung der flüssigen Mikrokapsel-Dispersion sind dem Fachmann verschiedene Verfahren bekannt, unter anderem Sprühtrocknung, Wirbelschichttrocknung, Sprühgranulation, Sprühagglomeration, oder Verdunstung.

Nach der Trocknung liegt der Wassergehalt in der getrockneten Mikrokapsel-Dispersion bei weniger als 5 Gew.-% Der Wassergehalt kann bei 5 Gew.-%, 1 Gew.-%, 0,8 Gew.-%, 0,5 Gew.-%, 0,1 Gew.-%, 0,05 Gew.-%, 0,01 Gew.-%, 0,001 Gew.-%, oder 0,0001 Gew.-% liegen. Gemäß einer Ausführungsform der getrockneten Mikrokapsel-Dispersion liegt der Wassergehalt bei weniger als 1 Gew.-%, bevorzugt bei weniger als 0,01 Gew.-% noch bevorzugter weniger als 0,001 Gew.-%. Besonders bevorzugt enthält die getrocknete Mikrokapsel-Dispersion bis auf unvermeidliche Spuren kein Wasser.

Dabei können dem flüssigen Gemisch Trocknungs- und Sprühhilfsmittel zugesetzt sein, wie beispielsweise feinverteiltes Siliziumdioxid (Aerosil^{®} von Evonik Industries).

Die getrocknete Mikrokapsel-Dispersion (Pulvermischung) enthaltend den Emulgator kann wiederrum in Mittel, Zwischenerzeugnisse oder Formulierungen eingearbeitet werden, beispielsweise durch Redispergieren in einem flüssigen Medium, vorzugsweise in einer wässrigen Phase. Über diesen Weg kann der Trockengehalt der Formulierung direkt durch das Mischungsverhältnis von Pulvermischung und flüssigem Medium eingestellt werden.

In verschiedenen Ausführungsformen kann ein erfindungsgemäßes Mittel die beschriebene Mikrokapsel-Dispersion enthalten.

Dabei kommt die phasenstabilisierende Wirkung des Emulgators auf die Mikrokapseln besonders zur Geltung, nämlich wenn die Mikrokapsel-Dispersion in der Herstellung eines Mittels mit einer anderen Lösung oder Dispersion zur Ausbildung eines Zwischenprodukts oder des finalen Mittels in Kontakt gebracht bzw. vermischt wird. Das Mittel kann fest oder flüssig sein. Gemäß einer Ausführungsform ist das Mittel flüssig.

Die phasenstabilisierende Wirkung tritt in einem breiten pH-Bereich auf. Insbesondere kommt die phasenstabilisierende Wirkung des Emulgators bei einem nicht stark basischen pH-Wert zum Tragen. Gemäß einer Ausführungsform liegt der pH des Mittels bei weniger als 10. Beispielsweise kann der pH-Wert des Mittels bei 9,5, 9,0, 8,5, 8,0, 7,5, 7,0, 6,5, 6,0, 5,5, 5,0, 4,5, 4,0, 3,5, 3,0, 2,5, 2,0, oder 1,5 liegen. Der pH-Wert kann bei weniger als 9, weniger als 8 oder weniger als 7 liegen. Insbesondere in saurer Umgebung ist die phasenstabilisierende Wirkung des Emulgators wesentlich. Folglich liegt der pH-Wert des Mittels in einer Ausführungsform bei weniger als 6, bevorzugt weniger als 5, noch bevorzugter bei weniger als 4 und besonders bevorzugt bei weniger als 3.

Die phasenstabilisierende Wirkung tritt in einem breiten Leitfähigkeitsbereich auf. Gemäß einer Ausführungsform liegt die Leitfähigkeit des Mittels bei bis zu 100 mS/cm, bevorzugt bis zu 60 mS/cm, bis zu 50 mS/cm oder bis zu 40 mS/cm, besonders bevorzugt werden 34 mS/cm eingesetzt, wobei typische Leitfähigkeiten im Bereich von mindestens 0,1 mS/cm, mindestens 0,2 mS/cm, mindestens 0,3 mS/cm, mindestens 2,0 mS/cm oder mindestens 8,0 mS/cm liegen. Beispielsweise kann die Leitfähigkeit bei 0,4 mS/cm, 0,5 mS/cm, 0,6 mS/cm, 0,7 mS/cm, 0,8 mS/cm, 0,9 mS/cm, 1,0 mS/cm, 1,5 mS/cm, 2,0 mS/cm, 3,0 mS/cm, 4,0 mS/cm, 5,0 mS/cm, 6,0 mS/cm, 7,0 mS/cm, 8,0 mS/cm, 9,0 mS/cm, 10,0 mS/cm, 12,0 mS/cm, 14,0 mS/cm, 16,0 mS/cm, 18,0 mS/cm, 20,0 mS/cm, 22,0 mS/cm, 24,0 mS/cm, 26,0 mS/cm, 28,0 mS/cm, 30,0 mS/cm, 32,0 mS/cm, oder 34,0 mS/cm liegen. Gemäß einer Ausführungsform des Mittels liegt die Leitfähigkeit im Bereich von 0,2 mS/cm bis 6,0 mS/cm, bevorzugt im Bereich von 0,3 mS/cm bis 5,0 mS/cm, noch bevorzugter in Bereich von 0,4 mS/cm bis 4,0 mS/cm. In diesem Fall liegt der pH bevorzugt im sauren Bereich, insbesondere unterhalb von pH 4,0. Gemäß einer weiteren Ausführungsform liegt die Leitfähigkeit im Bereich von 7,0 mS/cm bis 40,0 mS/cm, bevorzugt im Bereich von 8,0 mS/cm bis 34,0 mS/cm. In diesem Fall liegt der pH bevorzugt im neutralen oder basischen Bereich, insbesondere im Bereich von 7,5 bis 9,0.

Die Mikrokapsel-Dispersion kann zur Bildung des Mittels oder dessen Zwischenprodukts eingesetzt werden. Gemäß einer Ausführungsform hat das Mittel oder das Zwischenprodukt, das durch Zugabe der Mikrokapsel-Dispersion gebildet wird, einen pH-Wert und/oder eine Leitfähigkeit wie für das Mittel definiert. Gemäß einer Ausführungsform hat das Mittel oder das Zwischenprodukt, das durch Zugabe der Mikrokapsel-Dispersion gebildet wird, einen pH-Wert von weniger als 10, vorzugsweise weniger als 9, bevorzugter weniger als 5 und besonders bevorzugt weniger als 4 und/oder eine Leitfähigkeit mehr als 0,3 mS/cm, vorzugsweise mehr als 1,0 mS/cm, bevorzugter mehr als 2,5 mS/cm und besonderes bevorzugt mehr als 5,0 mS/cm.

### Wasch- oder Reinigungsmittel enthaltend Mikrokapseln

Aufgrund der Robustheit bzw. Dichtigkeit dieser bioabbaubaren Kapsel können diese in vorteilhafter Weise in einem Wasch- und Reinigungsmittel oder in kosmetischen Mitteln eingesetzt werden, wobei diese Mittel Weichspüler, Textilpflegemittel, feste Waschmittel, beispielsweise Granulate oder Pulver, Flüssigwaschmittel, Haushaltsreiniger, Bad- und WC-Reiniger, Handgeschirrspülmittel, Maschinengeschirrspülmittel, Handseifen, Shampoos, Duschgele, Cremes und ähnliche umfassen, aber nicht auf diese beschränkt sind. Die hierin beschriebenen Mittel können in fester oder flüssiger Form vorliegen, wobei flüssige Formulierungen bevorzugt sein können.

Unabhängig von der Art der Mittel umfassen diese die hierin beschriebenen Mikrokapseln sowie den hierin beschriebenen Emulgator, wobei diese beiden Bestandteile vorformuliert sind, d.h. bereits vor Zugabe zu dem erfindungsgemäßen Mittel in Kontakt gebracht wurden, typischerweise durch Vorformulierung der Kapseln in einer den Emulgator enthaltenden Slurry. Abhängig von der eingesetzten Menge der Mikrokapseln im Endprodukt variiert auch die Menge des co-formulierten Emulgators. Übliche Mengen liegen allerdings im Bereich von 0,001 bis 0,25 Gew.-% bezogen auf das Gesamtgewicht des Mittels. Zunehmend bevorzugte Bereiche sind bis 0,20, bis zu 0,15, bis zu 0,12, bis zu 0,10 oder bis zu 0,08 Gew.-%. Die Untergrenze liegt typischerweise im Bereich von 0,001 oder 0,005 oder 0,01 Gew.-%. Es wurde gefunden, dass sich der stabilisierende Effekt der eingesetzten Emulgatoren nicht nur auf die vorformulierte Slurry, sondern auch auf das (flüssige) Endprodukt erstreckt, so dass die phasenstabilisierende Wirkung auf die Mikrokapseln auch in dem Endprodukt zu beobachten ist. Dieser Effekt ist aber von der Vorformulierung der Mikrokapseln mit dem Emulgator abhängig und tritt nicht auf, wenn Mikrokapseln und Emulgator separat in das Mittel einformuliert werden.

Die Wasch- oder Reinigungsmittel der Erfindung umfassen vorzugsweise mindestens einen Inhaltsstoff ausgewählt aus der Gruppe der Tenside, Enzyme, Gerüststoffe und aufziehverstärkenden Mittel.

Die Wasch- und Reinigungsmittel können anionische, nichtionische, kationische, amphotere oder zwitterionische Tenside oder Mischungen davon enthalten. Bei diesen Tensiden handelt es sich typischerweise um von den Emulgatoren, die bei der Formulierung der Mikrokapseln eingesetzt werden, unterschiedliche Tenside. In verschiedenen Ausführungsformen umfassen die Tenside insbesondere mindestens ein anionisches Tensid und/oder mindestens ein nichtionisches Tensid, insbesondere wenn es sich bei dem Mittel um ein Wasch- oder Reinigungsmittel im engeren Sinne, d.h. beispielsweise Textilwaschmittel und Geschirrspülmittel sowie Haushalts- und Badreiniger, handelt.

Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder Sulfonat-Gruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden z.B. mit Dimethylsulfat quaterniert. In Frage kommende QAV sind beispielweise Benzalkoniumchlorid (N-Alkyl-N,N-dimethylbenzylammoniumchlorid), Benzalkon B (m,p-Dichlorbenzyldimethyl-C₁₂-alkylammoniumchlorid, Benzoxoniumchlorid (Benzyldodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzyl-ammoniumchlorid), Dialkyldimethylammoniumchloride wie Di-n-decyl-dimethyl-ammoniumchlorid, Didecyldimethylammoniumbromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkylbenzyl-dimethylammoniumchlorid.

Bevorzugte Esterquats sind Methyl-N-(2-hydroxyethyl)-N,N-di(talgacyl-oxyethyl)ammonium-metho-sulfat, Bis-(palmitoyl)-ethyl-hydroxyethyl-methyl-ammonium-methosulfat oder Methyl-N,N-bis(acyl-oxyethyl)-N-(2-hydroxyethyl)ammonium-methosulfat. Handelsübliche Beispiele sind die von der Firma Stepan unter dem Warenzeichen Stepantex^{®} vertriebenen Methylhydroxyalkyldialkoyloxyalkylammoniummethosulfate oder die unter dem Handelsnamen Dehyquart^{®} bekannten Produkte der Firma BASF SE beziehungsweise die unter der Bezeichnung Rewoquat^{®} bekannten Produkte des Herstellers Evonik. Kationische Tenside wie die vorstehend beschriebenen werden vor allem in Weichspülern eingesetzt.

Die Mengen der einzelnen Inhaltsstoffe in den Wasch- und Reinigungsmitteln orientieren sich jeweils am Einsatzzweck der betreffenden Zusammensetzung und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der Zusammensetzungen wird man beispielsweise den Tensidgehalt höher oder niedriger wählen. Üblicherweise kann z.B. der Tensidgehalt beispielsweise von Waschmitteln von 10 bis 50 Gew.-%, bevorzugt von 12,5 bis 30 Gew.-% und stärker bevorzugt von 15 bis 25 Gew.-% betragen.

Die Wasch- und Reinigungsmittel können beispielsweise mindestens einen wasserlöslichen und/oder wasserunlöslichen, organischen und/oder anorganischen Builder enthalten. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Die organischen Buildersubstanzen können, insbesondere zur Herstellung flüssiger Wasch- und Reinigungsmittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Organische Buildersubstanzen können, falls gewünscht, in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, falls gewünscht, in Mengen von bis zu 50 Gew.-%, vorzugsweise nicht über 40 Gew.-% und in flüssigen Zusammensetzungen insbesondere von 1 Gew.-% bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in Wasch- oder Reinigungsmitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2.8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁·yH₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl beta- als auch delta-Natriumdisilikate (Na₂Si₂O₅·yH₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können eingesetzt werden. In einer weiteren bevorzugten Ausführungsform wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform der Textilbehandlungs- oder Reinigungsmittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Zusammensetzungen, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind, falls gewünscht, bevorzugt in Mengen bis zu 60 Gew.-%, insbesondere von 5 Gew.-% bis 40 Gew.-%, enthalten. Wäschenachbehandlungsmittel, wie z.B. Weichspüler, sind bevorzugt frei von anorganischem Builder.

In verschiedenen Ausführungsformen umfasst ein erfindungsgemäßes Mittel weiterhin mindestens ein Enzym.

Das Enzym kann ein hydrolytisches Enzym oder ein anderes Enzym in einer für die Wirksamkeit des Mittels zweckmäßigen Konzentration sein. Eine Ausführungsform der Erfindung stellen somit Mittel dar, die ein oder mehrere Enzyme umfassen. Als Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder eine Lipase, sowie deren Gemische. Enzyme sind in dem Mittel vorteilhafterweise jeweils in einer Menge von 1 × 10⁻⁸ bis 5 Gew.-% bezogen auf aktives Protein enthalten. Zunehmend bevorzugt ist jedes Enzym in einer Menge von 1 × 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Bei der/den Amylase(n) handelt es sich vorzugsweise um eine α-Amylase. Bei der Hemicellulase handelt es sich vorzugsweise um eine Pektinase, eine Pullulanase und/oder eine Mannanase. Bei der Cellulase handelt es sich vorzugsweise um ein Cellulase-Gemisch oder eine Einkomponenten-Cellulase, vorzugsweise bzw. überwiegend um eine Endoglucanase und/oder eine Cellobiohydrolase.

Bei der Oxidoreduktase handelt es sich vorzugsweise um eine Oxidase, insbesondere eine Cholin-Oxidase, oder um eine Perhydrolase.

Die eingesetzten Proteasen sind vorzugsweise alkalische Serin-Proteasen. Sie wirken als unspezifische Endopeptidasen, das heißt, sie hydrolysieren beliebige Säureamidbindungen, die im Inneren von Peptiden oder Proteinen liegen und bewirken dadurch den Abbau proteinhaltiger Anschmutzungen auf dem Reinigungsgut. Ihr pH-Optimum liegt meist im deutlich alkalischen Bereich. In bevorzugten Ausführungsformen ist das im erfindungsgemäßen Mittel enthaltene Enzym eine Protease.

Die vorliegend eingesetzten Enzyme können natürlicherweise vorkommende Enzyme sein oder Enzyme, die auf Basis natürlich vorkommender Enzyme durch eine oder mehrere Mutationen verändert wurden, um gewünschte Eigenschaften, wie katalytische Aktivität, Stabilität oder desinfizierende Leistung, positiv zu beeinflussen.

In bevorzugten Ausführungsformen der Erfindung ist das Enzym in Form eines Enzymprodukts in einer Menge von 0,01 bis 10 Gew.-%, vorzugsweise 0,01 bis 5 Gew.-% im erfindungsgemäßen Mittel bezogen auf das Gesamtgewicht des Mittels enthalten. Der Aktivproteingehalt liegt vorzugsweise im Bereich von 0,00001 bis 1 Gew.-%, insbesondere 0,0001 bis 0,2 Gew.-% bezogen auf das Gesamtgewicht des Mittels.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

In den hierin beschriebenen Mitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

In verschiedenen Ausführungsformen kann das erfindungsgemäße Mittel einen oder mehrere Enzymstabilisatoren aufweisen.

Aufziehverstärkende Mittel sind Mittel, die das Aufziehen der Mikrokapseln auf Oberflächen, insbesondere Textiloberflächen, verbessern. Unter diese Kategorie von Mitteln fallen beispielsweise die bereits oben erwähnten Esterquats. Weitere Beispiele sind sogenannte SRPs (soil repellent polymers), die nichtionisch oder kationisch sein können, wobei hier insbesondere Polyethylenimine (PEI) sowie ethoxylierte Varianten davon und Polyester, insbesondere Ester der Terephthalsäure, vor allem solche von Ethylenglykol und Terephthalsäure oder Polyester/Polyether von Polyethyleneterephthalat und Polyethylenglycol, zu nennen sind. Schließlich fallen auch anionische bzw. nichtionische Silikone unter diese Gruppe. Beispielhafte Verbindungen werden auch in der Patentschrift EP 2 638 139 A1 offenbart.

Ferner können die Wasch- und Reinigungsmittel weitere Inhaltsstoffe enthalten, die die anwendungstechnischen und/oder ästhetischen Eigenschaften der Zusammensetzung abhängig von dem beabsichtigten Verwendungszweck weiter verbessern. Im Rahmen der vorliegenden Erfindung können sie Bleichmittel, Bleichaktivatoren, Bleichkatalysatoren, Esterquats, Silikonöle, Emulgatoren, Verdicker, Elektrolyte, pH-Stellmittel, Fluoreszenzmittel, Farbstoffe, Hydrotope, Schauminhibitoren, Antiredepositionsmittel, Lösungsmittel, optische Aufheller, Vergrauungsinhibitoren, Einlaufverhinderer, Knitterschutzmittel, Farbübertragungsinhibitoren, Farbschutzmittel, Benetzungsverbesserer, antimikrobielle Wirkstoffe, Germizide, Fungizide, Antioxidantien, Korrosionsinhibitoren, Klarspüler, Konservierungsmittel, Antistatika, Bügelhilfsmittel, Phobier- und Imprägniermittel, Perlglanzmittel, Polymere, Quell- und Schiebefestmittel sowie UV-Absorber enthalten, ohne darauf beschränkt zu sein.

Geeignete Inhaltsstoffe und Rahmenzusammensetzungen für Wasch- und Reinigungsmittelzusammensetzungen (beispielsweise für Waschmittel und Weichspüler) sind beispielsweise in der EP 3 110 393 B1 offenbart. In verschiedenen Ausführungsformen handelt es sich bei dem Mittel gemäß der Erfindung um einen Weichspüler oder ein Flüssig(textil)waschmittel.

### Herstellungsverfahren

Verfahren zur Herstellung von Kern/Schale-Mikrokapseln sind dem Fachmann bekannt. In der Regel wird ein ölbasiertes nicht bzw. wenig wasserlösliches Kernmaterial in einer die Wandbildner enthaltenden wässrigen Phase emulgiert oder dispergiert. In Abhängigkeit von der Viskosität flüssiger Kernmaterialien kommen vom einfachen Rührer bis zum Hochleistungsdispergierer verschiedenste Aggregate zum Einsatz, die das Kernmaterial in feine Öltröpfchen verteilt. Dabei scheiden sich die Wandbildner aus der kontinuierlichen Wasserphase auf der Öltröpfchen-Oberfläche ab und können anschließend vernetzt werden.

Dieser Mechanismus wird genutzt bei der In-situ-Polymerisation von Amino- und Phenoplast-Mikrokapseln und bei der Koazervation wasserlöslicher Hydrokolloide.

Im Gegensatz dazu kommen bei der radikalischen Polymerisation öllösliche Acrylat-Monomere für die Wandbildung zum Einsatz. Darüber hinaus kommen Verfahren zum Einsatz, bei denen wasserlösliche und öllösliche Ausgangsstoffe an der Phasengrenze der Emulsionstropfen zur Reaktion gebracht werden, die die feste Schale bilden.

Beispiele hierfür sind die Reaktion von Isocyanaten und Aminen bzw. Alkoholen zu Polyharnstoff- bzw. Polyurethanwänden (Grenzflächenpolymerisation), aber auch die Hydrolyse von Silikat-Präkursoren mit anschließender Kondensation unter Ausbildung einer anorganischen Kapselwand (Sol-Gel-Verfahren).

In geeigneten Verfahren zur Herstellung von Mikrokapseln, umfassend einen Duftstoff als Kernmaterial und einer Schale, die aus drei Schichten besteht, wird die als Diffusionsbarriere dienende Barriereschicht als Templat vorgelegt. Zum Aufbau dieser Barriereschicht werden sehr geringe Anteile an Wandbildnern der genannten Art benötigt. Bevorzugt sind die empfindlichen Template nach der Tröpfchenbildung bei hohen Rührgeschwindigkeiten durch geeignete Schutzkolloide (z.B. Poly-AMPS) so mit einer elektrisch negativen Ladung ausgerüstet, dass weder Ostwaldreifung noch Koaleszenz auftreten können. Nach Herstellung dieser stabilen Emulsion kann bei nunmehr stark verminderter Rührgeschwindigkeit der Wandbildner, beispielsweise ein geeignetes Vorkondensat auf Aminoplastharzbasis eine sehr dünne Schale (Schicht) ausbilden. Die Dicke der Schale kann insbesondere durch Zusatz eines aromatischen Alkohols, z.B. m-Aminophenol, noch weiter reduziert werden. Es folgt die Ausbildung einer produktionsfähigen Schalenstruktur, die unerwarteter Weise bei Zugabe von Biopolymeren wie Gelatine oder Alginat eine gute Affinität zu diesen aufzeigt und eine Abscheidung auf den Templaten ohne die erwarteten Probleme wie Gelierung des Ansatzes, Agglomerationsbildung und Unverträglichkeit des Strukturgebers aufzeigt.

Durch den Einsatz des Emulsionsstabilisators kann die Abscheidung der Biopolymere noch weiter erhöht werden.

Die erfindungsgemäßen Mikrokapsel-Dispersion werden vorzugsweise nach einem Verfahren hergestellt, das zumindest die folgenden Schritte umfasst:
a) Herstellen einer Öl-in-Wasser-Emulsion durch Emulgierung eines Kernmaterials in einer wässrigen Phase in Anwesenheit der wandbildenden Komponente(n) der inneren Barriereschicht unter Zugabe von Schutzkolloiden;
b) Abscheidung und Aushärtung der wandbildenden Komponente(n) der Barriereschicht, wobei die wandbildende(n) Komponente(n) der Barriereschicht bevorzugt eine aldehydische Komponente, eine Aminkomponente und ein aromatischer Alkohol, besonders bevorzugt Formaldehyd, Melamin, und Resorcin sind;
c) optionale Zugabe eines Emulsionsstabilisators, wobei der Emulsionsstabilisator wie hierin definiert ist;
d) Zugabe der wandbildenden Komponente(n) der Stabilitätsschicht, gefolgt von Abscheidung und Aushärtung, wobei die wandbildende(n) Komponenten der Stabilitätsschicht mindestens ein Biopolymer, bevorzugt ein Protein und/oder ein Polysaccharid, besonders bevorzugt Gelatine und Alginat, sowie ein Aushärtungsmittel, bevorzugt Glutaraldehyd oder Glyoxal sind; und
e) gegebenenfalls Zugabe der wandbildenden Komponente(n) der äußeren, dritten Schalenschicht, gefolgt von Abscheidung und Aushärtung, wobei die wandbildende(n) Komponente(n) der äußeren, dritten Schalenschicht bevorzugt eine Aminkomponente, insbesondere Melamin ist.

Die Zugabe des Emulsionsstabilisators erfolgt bevorzugt langsam über mindestens zwei Minuten. Gemäß einer Ausführungsform wird die Mikrokapsel-Dispersion gerührt. Zum Rühren kann beispielsweise ein Flügelrührer eingesetzt werden. Die Rührgeschwindigkeit liegt bevorzugt im Bereich von 150 bis 250 U/min. Oberhalb von 250 U/min besteht die Gefahr eines Lufteintrags in die Mikrokapsel-Dispersion. Unterhalb von 150 U/min könnte die Durchmischung nicht ausreichend sein.

Die Temperatur liegt bevorzugt im Bereich von 15 °C bis 35 °C. Die Temperatur kann bei 15 °C, 18 °C, 20 °C, 23 °C, 25 °C, 28 °C, 30 °C, 33 °C, oder 35 °C liegen. Besonders bevorzugt liegt die Temperatur bei 25 °C. Nach Zugabe wird die Mikrokapsel-Dispersion gerührt bis eine homogene Mischung entsteht. In einer Ausführungsform wird die Mikrokapsel-Dispersion nach Zugabe für mindestens 5 min gerührt. In einer bevorzugten Ausführungsform wird die Mikrokapsel-Dispersion nach Zugabe für mindestens 10 min gerührt.

Alternativ können die Schritte a) und b) wie folgt durchgeführt werden:
a) Herstellen einer Öl-in-Wasser-Emulsion durch Emulgierung eines Kernmaterials in einer wässrigen Phase in Anwesenheit der wandbildenden Komponente(n) der inneren Barriereschicht, gegebenenfalls unter Zugabe von Schutzkolloiden;
b) Abscheidung und Aushärtung der wandbildenden Komponente(n) der inneren Barriereschicht, wobei die wandbildende(n) Komponente(n) der inneren Barriereschicht insbesondere eine aldehydische Komponente, eine Aminkomponente und ein aromatischer Alkohol sind.

Dieses Verfahren kann entweder sequentiell oder als sogenanntes Eintopfverfahren durchgeführt werden. Beim sequentiellen Verfahren werden in einem ersten Verfahren nur die Schritte a) und b) bis zum Erhalt von Mikrokapseln mit nur der inneren Barriereschicht als Schale (Intermediatsmikrokapseln) durchgeführt. Im Folgenden wird dann eine Teilmenge oder die Gesamtmenge dieser Intermediatsmikrokapseln in einen weiteren Reaktor überführt. In diesem werden dann die weiteren Reaktionsschritte durchgeführt. Beim Eintopf-Verfahren werden sämtliche Verfahrensschritte in einem Batch-Reaktor durchgeführt. Die Durchführung ohne Reaktorwechsel ist besonders zeitsparend.

Dazu sollte das Gesamtsystem auf das Eintopfverfahren abgestimmt sein. Die richtige Wahl der Feststoffanteile, die richtige Temperaturführung, die abgestimmte Zugabe an Formulierungsbestandteilen und die sequentielle Zugabe der Wandbildner ist auf diese Art möglich.

In einer Ausführungsform des Verfahrens umfasst das Verfahren die Herstellung einer Wasserphase durch Lösung eines Schutzkolloids, insbesondere eines Polymers auf Basis von Acrylamidosulfonat und einem methylierten Prä-Polymer in Wasser. Dabei wird das Prä-Polymer bevorzugt durch Umsetzung eines Aldehyds mit entweder Melamin oder Harnstoff erzeugt. Optional kann dabei Methanol zum Einsatz kommen.

Ferner kann in dem Verfahren eine Durchmischung der Wasserphase mittels Rühren und Einstellen einer ersten Temperatur erfolgen, wobei die erste Temperatur im Bereich von 30 °C bis 40 °C liegt. Im Anschluss kann ein aromatischer Alkohol, insbesondere Phloroglucin, Resorcin oder Aminophenol zur Wasserphase hinzugefügt und darin gelöst werden.

Alternativ kann in dem Verfahren die Herstellung einer Ölphase durch Mischung einer Duftstoffzusammensetzung oder eines Phasenwechselmaterials (PCM) mit aromatischen Alkoholen geschehen, insbesondere Phloroglucin, Resorcin oder Aminophenol. Alternativ können auch reaktive Monomere oder Diisocyanatderivate in die Duftstoffzusammensetzung eingebracht werden. Anschließend kann die Einstellung der ersten Temperatur erfolgen.

Ein weiterer Schritt kann die Herstellung eines Zwei-Phasen-Gemischs durch Zugabe der Ölphase zur Wasserphase und anschließender Erhöhung der Drehzahl sein.

Im Anschluss kann die Emulgierung durch Zugabe von Ameisensäure gestartet werden. Dabei bietet sich eine regelmäßige Bestimmung der Teilchengröße an. Ist die gewünschte Teilchengröße erreicht, kann die Zwei-Phasen-Mischung weiter gerührt werden und dabei eine zweite Temperatur zur Aushärtung der Kapselwände eingestellt werden. Die zweite Temperatur kann dabei im Bereich von 55 °C bis 65 °C liegen.

Im Anschluss kann die Zugabe einer Melamin-Dispersion zur Mikrokapsel-Dispersion und Einstellung einer dritten Temperatur erfolgen, wobei die dritte Temperatur bevorzugt im Bereich von 75 °C bis 85 °C liegt.

Ein weiterer geeigneter Schritt ist die Zugabe einer wässrigen Harnstoff-Lösung zur Mikrokapsel-Dispersion. Im Anschluss wird der Emulsionsstabilisator zur Mikrokapsel-Dispersion gegeben bevor diese zur Herstellung der Stabilisationsschicht zu einer Lösung von Gelatine und Alginat gegeben wird.

In diesem Fall würde im Anschluss eine Abkühlung auf 45 °C bis 55 °C erfolgen sowie ein Einstellen des pH der Mikrokapsel-Dispersion auf einen Wert im Bereich von 3,5 bis 4,1, insbesondere 3,7.

Die Mikrokapsel-Dispersion kann dann auf eine vierte Temperatur abgekühlt werden, wobei die vierte Temperatur im Bereich von 20°C bis 30°C liegt. Es kann im Folgenden auf eine fünfte Temperatur abgekühlt werden, wobei die fünfte Temperatur in einem Bereich von 4 °C bis 17 °C liegt, insbesondere bei 8°C.

Im Anschluss würde der pH der Mikrokapsel-Dispersion auf einen Wert im Bereich 4,3 bis 5,1 eingestellt und Glutaraldehyd oder Glyoxal zugegeben werden. Die Reaktionsbedingungen, insbesondere Temperatur und pH-Wert, können je nach Vernetzer unterschiedlich gewählt werden. Die jeweils geeigneten Bedingungen kann der Fachmann beispielsweise aus der Reaktivität des Vernetzers ableiten. Durch die zugegebene Menge an Glutaraldehyd oder Glyoxal wird die Vernetzungsdichte der ersten Schicht (Stabilitätsschicht) beeinflusst und damit beispielsweise die Dichtigkeit und Abbaubarkeit der Mikrokapselschale. Entsprechend kann der Fachmann die Menge gezielt variieren, um das Eigenschaftsprofil der Mikrokapsel anzupassen. Zur Erzeugung der zusätzlichen dritten Schicht kann eine Melamin-Suspension, bestehend aus Melamin, Ameisensäure und Wasser hergestellt werden. Es folgt die Zugabe der Melamin-Suspension zu der Mikrokapsel-Dispersion. Schließlich würde der pH der Mikrokapsel-Dispersion auf einen Wert im Bereich von 9 bis 12 eingestellt werden, insbesondere 10 bis 11.

Darüber hinaus kann die Mikrokapsel-Dispersion zur Aushärtung im Schritt e) auf eine Temperatur im Bereich von 20 °C bis 80 °C aufgeheizt werden. Diese Temperatur kann einen Einfluss auf die Farbbeständigkeit der Mikrokapseln haben. Die Temperatur kann bei 20 °C, 25 °C, 30 °C, 35 °C, 40 °C, 45 °C, 50 °C, 55 °C, 60 °C, 65 °C, 70 °C, 75 °C, oder 80 °C liegen. Unterhalb einer Temperatur von 20 °C ist mit keinem Einfluss auf die Farbbeständigkeit zu rechnen. Eine Temperatur von über 80 °C könnte sich negativ auf die Mikrokapsel-Eigenschaften auswirken. Gemäß einer Ausführungsform liegt die Temperatur im Bereich von 30 °C bis 60 °C. Gemäß einer bevorzugten Ausführungsform liegt die Temperatur im Bereich von 35 °C bis 50 °C.

Gemäß einer Ausführungsform, wird die Mikrokapsel-Dispersion bei der Aufheiztemperatur für einen Zeitraum von wenigstens 5 Minuten gehalten. Der Zeitraum kann beispielsweise 10 Minuten, 15 Minuten, 20 Minuten, 25 Minuten, 30 Minuten, 35 Minuten, 40 Minuten, 45 Minuten, 50 Minuten, 55 Minuten, 60 Minuten, 70 Minuten, 80 Minuten, 90 Minuten, 100 Minuten, 110 Minuten, 120 Minuten betragen. Gemäß einer Ausführungsform, wird die Mikrokapsel-Dispersion bei der Aufheiztemperatur für einen Zeitraum von wenigstens 30 Minuten gehalten. Gemäß einer Ausführungsform wird die Mikrokapsel-Dispersion bei der Aufheiztemperatur für einen Zeitraum von wenigstens 60 Minuten gehalten.

In einem Verfahrensschritt der Herstellung der Mikrokapseldispersion wird der Emulgator, ausgewählt aus der aus der Gruppe der ethoxylierten, hydrierten Rizinusöle, zugegeben. Bevorzugt wird der Emulgator, beispielsweise Eumulgin^{®} HRE 40, nach dem Aushärtungsschritt e) zugegeben. Dabei werden die Mikrokapseln typischerweise mit geeigneten Mitteln in eine wässrige kontinuierliche Phase, die bereits den erfindungsgemäß eingesetzten Emulgator enthält, dispergiert und damit die erfindungsgemäßen Slurries erzeugt. Die eingesetzten Mengen/Konzentrationen von Emulgator, Mikrokapseln und Wasser sind wie oben definiert.

Alternativ zum Einsatz des Emulgators, also des hydrierten, ethoxylierten Rizinusöls, kann auch eine Verbesserung der Phasenstabilität von Dispersionen der erfindungsgemäßen eingesetzten Mikrokapseln in einer Weichspülerbase durch eine Vorverdünnung der Mikrokapseldispersion erreicht werden.

Bei der Verdünnung einer Mikrokapsel-Dispersion enthaltend bioabbaubare Mikrokapseln umfassend ein Kernmaterial und eine Schale, wobei die Schale aus mindestens einer Barriereschicht und einer Stabilitätsschicht besteht, wobei die Barriereschicht das Kernmaterial umgibt, wobei die Stabilitätsschicht mindestens ein Biopolymer umfasst, und auf der äußeren Oberfläche der Barriereschicht angeordnet ist, und wobei optional am Übergang von Barriereschicht zu Stabilitätsschicht ein Emulsionsstabilisator angeordnet ist; in einer wässrigen Lösung ist der Mindestanteil der wässrigen Lösung im Verhältnis zur Mikrokapseldispersion 1:99.

Die Mikrokapsel-Dispersion kann mit einem Verhältnis von wässriger Lösung zu Mikrokapsel-Dispersion von 1:19, 1:15, 1:13, 1:11, 1:9, 1:7, 1:5, 1:4, 1:3, 1:2, 1:1, 2:1, 3:1, 4:1, 5:1, 7:1, 9:1, 11:1, 13:1, 15:1 oder 19:1 mit der wässrigen Lösung verdünnt werden. Gemäß einer Ausführungsform wird die Mikrokapsel-Dispersion auf ein Verhältnis von wässriger Lösung zu Mikrokapsel-Dispersion im Bereich von 1:15 bis 9:1 mit der wässrigen Lösung verdünnt werden. Gemäß einer Ausführungsform wird die Mikrokapsel-Dispersion auf ein Verhältnis von wässriger Lösung zu Mikrokapsel-Dispersion im Bereich von 1:9 bis 5:1 mit der wässrigen Lösung verdünnt werden. Gemäß einer Ausführungsform wird die Mikrokapsel-Dispersion auf ein Verhältnis von wässriger Lösung zu Mikrokapsel-Dispersion im Bereich von 1:3 bis 2:1 mit der wässrigen Lösung verdünnt werden.

Alternativ ist es bevorzugt, wenn die Mikrokapsel-Dispersion nach einem Verfahren hergestellt wird, das die folgenden Schritte umfasst:
a) Herstellen einer Öl-in-Wasser-Emulsion durch Emulgierung eines Kernmaterials in einer wässrigen Phase in Anwesenheit der wandbildenden Komponente(n) der inneren Barriereschicht unter Zugabe von Schutzkolloiden;
b) Abscheidung und Aushärtung der wandbildenden Komponente(n) der Barriereschicht, wobei die wandbildende(n) Komponente(n) der Barriereschicht bevorzugt eine aldehydische Komponente, eine Aminkomponente und ein aromatischer Alkohol, besonders bevorzugt Formaldehyd, Melamin, und Resorcin sind;
c) optionale Zugabe eines Emulsionsstabilisators, wobei der Emulsionsstabilisator wie hierin definiert ist;
d) Zugabe der wandbildenden Komponente(n) der Stabilitätsschicht, gefolgt von Abscheidung und Aushärtung, wobei die wandbildende(n) Komponenten der Stabilitätsschicht mindestens ein Biopolymer, bevorzugt ein Protein und/oder ein Polysaccharid, besonders bevorzugt Gelatine und Alginat, sowie ein Aushärtungsmittel, bevorzugt Glutaraldehyd oder Glyoxal sind; und
e) gegebenenfalls Zugabe der wandbildenden Komponente(n) der äußeren, dritten Schalenschicht, gefolgt von Abscheidung und Aushärtung, wobei die wandbildende(n) Komponente(n) der äußeren, dritten Schalenschicht bevorzugt eine Aminkomponente, insbesondere Melamin ist.
f) Verdünnung der Mikrokapsel-Dispersion auf eine Mikrokapsel-Konzentration im Bereich von 0,1 Gew.-% bis 50 Gew.-% in einer wässrigen Lösung.

Gemäß einer Ausführungsform besteht die wässrige Lösung aus Wasser. Die Temperatur der wässrigen Lösung liegt im Bereich von 1 °C bis 100 °C. Die Temperatur bei Einbringen in das Erzeugnis kann beispielsweise 1 °C, 5 °C, 10 °C, 15 °C, 20 °C, 25 °C, 30 °C, 35 °C, 40 °C, 45 °C, 50 °C, 55 °C, 60 °C, 65 °C, 70 °C, 75 °C, 80 °C, 85 °C, 90 °C, 95 °C oder 100 °C betragen. Gemäß einer Ausführungsform liegt die Temperatur der wässrigen Lösung im Bereich von 15 °C bis 90 °C. Gemäß einer Ausführungsform liegt die Temperatur der wässrigen Lösung im Bereich von 30 °C bis 80 °C. Gemäß einer Ausführungsform liegt die Temperatur der wässrigen Lösung im Bereich von 40 °C bis 80 °C. Gemäß einer Ausführungsform liegt die Temperatur der wässrigen Lösung im Bereich von 50 °C bis 70 °C. Gemäß einer Ausführungsform liegt die Temperatur der wässrigen Lösung bei etwa 60 °C.

Bevorzugt wird die Mikrokapsel-Dispersion kurz nach Herstellung also Abschluss von Schritt d) oder e) verdünnt. Die Zeit zwischen Schritt d) oder e) und Verdünnung f) beträgt höchstens sechs Monaten. Die Zeit zwischen Schritt d) oder e) und Verdünnung f) kann beispielsweise 5 min, 30 min, 1 h, 2 h, 4 h, 6 h, 8 h, 10 h, 12 h, 18 h, 24 h, 2 d, 4 d, 7 d, 2 Wochen, 3 Wochen, 4 Wochen, 2 Monate, 3 Monate, 4 Monate, 5 Monate oder 6 Monate betragen. Gemäß einer Ausführungsform beträgt die Zeit zwischen Schritt d) oder e) und Verdünnung f) höchstens 1 Woche. Gemäß einer Ausführungsform beträgt die Zeit zwischen Schritt d) oder e) und Verdünnung f) höchstens 2 Tage.

Die verdünnte Mikrokapsel-Konzentration kann bei 0,1 Gew.-%, 0,2 Gew.-%, 0,5 Gew.-%, 0,8 Gew.-%, 1,0 Gew.-%, 1,5 Gew.-%, 2,0 Gew.-%, 2,5 Gew.-%, 3,0 Gew.-%, 3,5 Gew.-%, 4,0 Gew.-%, 4,5 Gew.-%, 5,0 Gew.-%, 5,5 Gew.-%, 6,0 Gew.-%, 6,5 Gew.-%, 7,0 Gew.-%, 7,5 Gew.-%, 8,0 Gew.-%, 8,5 Gew.-%, 9,0 Gew.-%, 9,5 Gew.-%, 10 Gew.-%, 11 Gew.-%, 12 Gew.-%, 13 Gew.-%, 14 Gew.-%, 15 Gew.-%, 16 Gew.-%, 17 Gew.-%, 18 Gew.-%, 19 Gew.-%, 20 Gew.-%, 25 Gew.-%, 30 Gew.-%, 35 Gew.-%, 40 Gew.-%, 45 Gew.-% oder 50 Gew.-%. liegen. Gemäß einer Ausführungsform liegt die Mikrokapsel-Konzentration im Bereich von 5 Gew.-% bis 30 Gew.-%. Gemäß einer Ausführungsform liegt die Mikrokapsel-Konzentration im Bereich von 8 Gew.-% bis 20 Gew.-%. Gemäß einer Ausführungsform liegt die Mikrokapsel-Konzentration im Bereich von 10 Gew.-% bis 15 Gew.-%.

Mit der Verdünnung der Mikrokapseldisperion vor der Zudosierung in Weichspülerbase (Vorverdünnung) wird zwar nicht ganz die Phasenstabilität erreicht, die mit dem hydrierten, ethoxylierten Rizinusöl erreicht wird. Die Phasenstabilität ist dennoch signifikant besser als bei einer Dosierung ohne Vorbehandlung.

Vorzugsweise wird die verdünnte Mikrokapsel-Dispersion noch im warmen Zustand in das Erzeugnis gegeben, für das sie vorgesehen ist. Folglich betrifft die Erfindung ebenfalls ein Verfahren zur Herstellung eines Erzeugnisses umfassend die Schritte zur Herstellung der Mikrokapseldispersion und den zusätzlichen Schritt g) Einbringen der Mikrokapseldispersion in das Erzeugnis.

Methoden zur Durchmischung von Mikrokapsel-Dispersionen in Enderzeugnissen sind dem Fachmann bekannt. Bevorzugt wird die Mikrokapsel-Dispersion kurz nach Verdünnung in das Erzeugnis eingebracht. Die Zeit zwischen Verdünnung und Einbringen in das Erzeugnis beträgt höchstens 6 Monate. Die Zeit zwischen Verdünnung und Einbringen in das Erzeugnis kann beispielsweise 5 min, 30 min, 1 h, 2 h, 4 h, 6 h, 8 h, 10 h, 12 h, 18 h, 24 h, 36 h, 2 d, 4 d, 7 d, 2 Wochen, 3 Wochen, 4 Wochen, 6 Wochen, 2 Monate, 3 Monate, 4 Monate, 5 Monate, oder 6 Monate betragen betragen. Gemäß einer Ausführungsform beträgt die Zeit zwischen Verdünnung und Einbringen in das Erzeugnis höchstens 1 Woche. Gemäß einer Ausführungsform beträgt die Zeit zwischen Verdünnung und Einbringen in das Erzeugnis höchstens 2 d.

Bevorzugt wird die Mikrokapsel-Dispersion noch im warmen Zustand in das Erzeugnis eingebracht. Die Temperatur bei Einbringen in das Erzeugnis beträgt mindestens 20 °C. Die Temperatur bei Einbringen in das Erzeugnis kann beispielsweise 20 °C, 25 °C, 30 °C, 35 °C, 40 °C, 45 °C, 50 °C, 55 °C, 60 °C, 65 °C, 70 °C, 75 °C, 80 °C, 85 °C, 90 °C, 95 °C oder 100 °C betragen. Gemäß einer Ausführungsform liegt die Temperatur bei Einbringen in das Erzeugnis im Bereich von 20 °C bis 90 °C. Gemäß einer Ausführungsform liegt die Temperatur der wässrigen Lösung im Bereich von 25 °C bis 70 °C. Gemäß einer Ausführungsform liegt die Temperatur der wässrigen Lösung im Bereich von 30 °C bis 60 °C. Gemäß einer Ausführungsform liegt die Temperatur der wässrigen Lösung im Bereich von 40 °C bis 50 °C. Gemäß einer Ausführungsform liegt die Temperatur der wässrigen Lösung bei etwa 45°C.

Es ist zudem möglich, nach Vorverdünnung den erfindungsgemäßen Emulgator (hydriert, ethoxyliertes Rizinusöl) zu der Mikrokapsel-Dispersion zuzugeben, bevor diese dann in das Enderzeugnis, beispielsweise einen Weichspüler gegeben wird. Dadurch lässt sich die Phasenstabilität noch weiter verbessern. Gemäß einer Ausführungsform wird der Emulgator in die vorverdünnte Mikrokapseldispersion in einer Konzentration im Bereich von von 0,1 Gew.-% bis 50 Gew.-% zugegeben.

Folglich sind gemäß der vorliegenden Erfindung auch Mikrokapseldispersion bevorzugt, die bioabbaubare Mikrokapseln enthalten, umfassend ein Kernmaterial und eine Schale, wobei die Schale aus mindestens einer Barriereschicht und einer Stabilitätsschicht besteht, wobei die Barriereschicht das Kernmaterial umgibt, wobei die Stabilitätsschicht mindestens ein Biopolymer umfasst, und auf der äußeren Oberfläche der Barriereschicht angeordnet ist, und wobei optional am Übergang von Barriereschicht zu Stabilitätsschicht ein Emulsionsstabilisator angeordnet ist;
hergestellt durch ein Verfahren nach dem zweiten Aspekt, wobei die Mikrokapsel-Dispersion nach dem Schritt d) oder e) auf eine Mikrokapsel-Konzentration im Bereich von von 0,1 Gew.-% bis 50 Gew.-% in einer wässrigen Lösung verdünnt wird.

Gemäß einer Ausführungsform der Mikrokapsel-Dispersion wird im Herstellungsverfahren der Mikrokapsel-Dispersion kein Emulgator zugegeben. Gemäß einer Ausführungsform der Mikrokapsel-Dispersion wird im Herstellungsverfahren der Mikrokapsel-Dispersion nach der Verdünnung f) der erfindungsgemäße Emulgator zugegeben.

### Mikrokapseldispersion und Farbigkeit

Mikrokapseln liegen in der Regel in Form von Mikrokapsel-Dispersionen vor. Trotz der Verwendung von aromatischem Alkohol in der Barriereschicht der Mikrokapselschale weisen die Mikrokapsel-Dispersionen mit den hierin beschriebenen Mikrokapseln nur eine geringe Färbung auf.

Zur Qualifizierung der Verfärbung wurde für die hierin beschriebenen Mikrokapseln der Farbort im L*a*b*-Farbraum bestimmt. Das L*a*b*-Farbmodell ist in der EN ISO 11664-4 "Colorimetry -- Part 4: CIE 1976 L*a*b* Colour space" genormt. Der L*a*b*-Farbraum (auch: CIELAB, CIEL*a*b*, Lab-Farben) beschreibt alle wahrnehmbaren Farben. Er nutzt einen dreidimensionalen Farbenraum, bei dem der Helligkeitswert L* senkrecht auf der Farbebene (a*,b*) steht. Die a-Koordinate gibt die Farbart und Farbintensität zwischen Grün und Rot an und die b-Koordinate die Farbart und die Farbintensität zwischen Blau und Gelb. Je größer positive a- und b- und je kleiner negative a- und b-Werte, umso intensiver der Farbton. Falls a=0 und b=0, liegt ein unbunter Farbton auf der Helligkeitsachse vor. Zu den Eigenschaften des L*a*b*-Farbmodells zählen die Geräteunabhängigkeit und die Wahrnehmungsbezogenheit, das heißt: Farben werden unabhängig von der Art ihrer Erzeugung oder Wiedergabetechnik so definiert, wie sie von einem Normalbeobachter bei einer Standard-Lichtbedingung wahrgenommen werden.

Die beschriebenen Mikrokapsel-Dispersionen weisen im L*a*b*-Farbraum einen Farbort mit einem L*-Wert von mindestens 50 auf. Der L*-Wert kann beispielsweise 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, oder 80 betragen. Gemäß einer bevorzugten Ausführungsform weisen die Mikrokapsel-Dispersionen im L*a*b*-Farbraum einen Farbort mit einem L*-Wert von mindestens 50 auf. Besonders bevorzugt liegt der Farbort bei mindestens 60.

Darüber hinaus sind die mit den beschriebenen Herstellungsverfahren hergestellten Mikrokapsel-Dispersionen besonders farbstabil. Der Farbort der Mikrokapsel-Dispersion weist im L*a*b*-Farbraum nach Lagerung einen L*-Wert von mindestens 50 auf. Der L*-Wert nach Lagerung kann beispielsweise 51, 52, 53, 54, 55, 55, 56, 57, 58, 59, 60, 61, 62, 63, 64, 65, 66, 67, 68, 69, 70, 71, 72, 73, 74, 75, 76, 77, 78, 79 oder 80 betragen. Gemäß einer bevorzugten Ausführungsform weisen die Mikrokapsel-Dispersionen nach Lagerung im L*a*b*-Farbraum einen Farbort mit einem L*-Wert von mindestens 60 auf. Besonders bevorzugt liegt der Farbort bei mindestens 65.

Gemäß einer Ausführungsform beträgt die Lagerungszeit wenigstens vier Wochen, bevorzugt wenigstens sechs Wochen und insbesondere wenigstens acht Wochen.

### Beispiele

Die erfindungsgemäßem Mikrokapsel-Dispersionen in den folgenden Beispielen enthalten alle 5 Gew.-% Emulgator, aus der Gruppe der ethoxylierten, hydrierten Rizinusöle mit einem EO-Wert von 40 (Slurry = Suspension), nämlich Eumulgin^{®} HRE 40. Der Emulgator wurde nach vorherigem Aufschmelzen nach Herstellung der Mikrokapsel-Dispersionenen diesen hinzugefügt, insbesondere direkt vor dem Eindosieren in Weichspülerbase.

### Beispiel 1 - Herstellung von Referenz-Mikrokapseln mit Melamin-Formaldehyd Rezeptur

### 1.1 Materialien

Die eingesetzten Materialien zur Herstellung der Referenz-Mikrokapseln - Melamin-Formaldehyd sind in Tabelle 1 dargestellt.

**Tabelle 1: Liste der zur Herstellung von MK2 verwendeten Stoffe**

| | | | MK2 |
|---|---|---|---|
| Handelsname* | Stoffbeze ich n u ng | Konzentration / Gew.% | Einwaage / g |
| - | VE-Wasser | 100 | 187,5 |
| Dimension ^{™} SD, Fa. Solenis | 1,3,5-Triazin-2,4,6-triamin, Polymer mit Formaldehyd, methyliert (Gehalt (W/W): >= 60 % - <= 80 %), in Wasser | 67 | 42,5* |
| Dimension ^{™} PA 140, Fa. Solenis | Polymer auf Basis: Acrylamidosulfonat | 20 | 35,0* |
| Weiroclean, Fa. Kurt Kitzing GmbH | Kernmaterial (z.B. Duftöl, PCM, etc.) | 100 | 192,5* |
| - | Ameisensäure | 10 | 8,8 |
| Melafine^{®}, Fa. OCI Nitrogen B.V. | Melamin-Suspension ¹⁾ | 27 | 48,8 |
| - | Harnstoff-Lösung | 28,6 | 70,0 |

| | | | |
|---|---|---|---|
| 1) Konzentration bezogen auf die angesäuerte Suspension *Mengen der Komponenten beziehen sich auf die Handelsware und werden eingesetzt wie geliefert. | | | |

### 1.2 Herstellungsverfahren (basierend auf Patent BASF EP 1 246693 B1)

Dimension SD wurde in VE-Wasser eingerührt und danach Dimension PA140 zugegeben und gerührt bis eine klare Lösung entstand. Die Lösung wurde im Wasserbad auf 30-35 °C erwärmt. Unter Rühren mit einer Dissolverscheibe wurde das Parfümöl bei 1100 U/min zugegeben. Der pH-Wert der Öl-in-Wasser-Emulsion wurde mit einer 10 %-igen Ameisensäure auf 3,3 - 3,8 eingestellt. Danach wurde die Emulsion für 30 min mit 1100 U/min weiter gerührt bis eine Tropfengröße von 20 - 30 µm erreicht war oder entsprechend verlängert bis die gewünschte Teilchengröße von 20 - 30 µm (Peak-Max) erreicht ist. Die Teilchengröße wurde mittels eines Beckmann-Coulter Gerätes (Laserbeugung, Fraunhofer Methode) bestimmt. Die Drehzahl wurde in Abhängigkeit der Viskosität so verringert, dass eine gute Durchmischung gewährleistet war. Mit dieser Drehzahl wurde weitere 30 min bei 30 bis 40 °C gerührt. Anschließend wurde die Emulsion auf 60°C erwärmt und weiter gerührt. Die Melamin-Suspension wurde mit Ameisensäure (10%ig) auf einen pH-Wert von 4,5 eingestellt und zur Reaktionsmischung zudosiert. Der Ansatz wurde 60 min bei 60 °C gehalten und anschließend auf 80°C aufgeheizt. Nach 60 min Rühren bei 80°C wurde die Harnstoff-Lösung zugegeben.

Nach dem Abkühlen auf Raumtemperatur wurde die Mikrokapsel-Dispersion über ein 200-µm-Filtersieb filtriert.

### Beispiel 2 - Herstellung von erfindungsgemäßen Mikrokapsel-Dispersionen Slurrv 2 und Slurrv 5 sowie der nicht erfindungsgemäßen Referenzkapsel MK1

### 2.1 Materialien

Die eingesetzten Materialien zur Herstellung erfindungsgemäßer Mikrokapseln - Slurry 2 und Slurry 5 sind in Tabelle 2 dargestellt.

**Tabelle 2: Liste der zur Herstellung von Slurry 2 und 5 verwendeten Stoffe**

| | | | Slurry 2 | Slurry 5 |
|---|---|---|---|---|
| Handelsname | Stoffbeze ich n u ng | Konzent ration / Gew.% | Einwaage / g | Einwaage / g |
| | VE-Wasser Zugabe 1 | 100 | 35,2 | 39,6 |
| Dimension ^{™} SD, Fa. Solenis | 1,3,5-Triazin-2,4,6-triamin, Polymer mit Formaldehyd, methyliert (Gehalt (W/W): >= 60 % - <= 80 %), in Wasser | 67 | 2,1* | 1,7* |
| Dimension ^{™} PA 140, Fa. Solenis | Polymer auf Basis: Acrylamidosulfonat Zugabe 1 | 20 | 4,6* | 3,8* |
| Weiroclean, Fa. Kurt Kitzing GmbH | Kernmaterial (z.B. Duftöl, PCM, etc.) | 100 | 52,8* | 52,6* |
| - | Resorcin-Lösung | 3,2 | 12,7 | 11,9 |
| - | Ameisensäure Zugabe 1 | 10 | 0,7 | 0,4 |
| Melafine^{®}, Fa. OCI Nitrogen B.V. | Melamin-Suspension Zugabe 1 ¹⁾ | 27 | 2,6 | 2,0 |
| - | Harnstoff-Lösung | 41,9 | 2,6 | 2,2 |
| Dimension ^{™} PA 140, Fa. Solenis | Polymer auf Basis: Acrylamidosulfonat Zugabe 2 | 20 | 9,5* | 9,6* |
| - | Leitungswasser | 100 | 136,2 | 135,6 |
| - | Natriumsulfat | 100 | 0,6* | 0,6* |
| Scogin^{®} MV, Fa. DuPont Nutrition Ireland | Natriumalginat | 100 | 2,0* | 2,0* |
| Speisegelatinepulver, Fa. Ewald-Gelatine GmbH | Schweinehautgelatine | 100 | 8,4* | 8,4* |
| - | Ameisensäure Zugabe 2 | 20 | 2,5 | 2,5 |
| - | Natronlauge Zugabe 1 | 20 | 2,4 | 2,3 |
| Glutaraldehyd, 50% aq. soln. Fa. Alfa Aesar | Glutaraldehyd Lösung | 50 | 2,6* | 2,6* |
| Melafine^{®}, Fa. OCI Nitrogen B.V. | Melamin-Suspension Zugabe 2 ¹⁾ | 27 | 27,5 | 27,4 |
| - | Natronlauge Zugabe 2 | 20 | 4,7 | 4,7 |

| | | | | |
|---|---|---|---|---|
| 1) Konzentration bezogen auf die angesäuerte Suspension. 2) Bei den gegebenen Mengen für Säuren/Laugen handelt es sich um Richtwerte. Es wird auf den in der Versuchsdurchführung genannten pH-Bereich eingestellt. *Mengen der Komponenten beziehen sich auf die Handelsware und werden eingesetzt wie geliefert. | | | | |

Die eingesetzten Materialien zur Herstellung der Referenz-Mikrokapseln MK1 sind in Tabelle 3 dargestellt.

**Tabelle 3: Liste der zur Herstellung von MK1 verwendeten Stoffe**

| | | MK1 | |
|---|---|---|---|
| Handelsname | Stoffbeze ich n u ng | Konzentration / Gew.% | Einwaage / g |
| | VE-Wasser Zugabe 1 | 100 | 34,9 |
| Dimension ^{™} SD, Fa. Solenis | 1,3,5-Triazin-2,4,6-triamin, Polymer mit Formaldehyd, methyliert (Gehalt (W/W): >= 60 % - <= 80 %), in Wasser | 67 | 1,6* |
| Dimension ^{™} PA 140, Fa. Solenis | Polymer auf Basis: Acrylamidosulfonat | 20 | 3,4* |
| Weiroclean, Fa. Kurt Kitzing GmbH | Kernmaterial (z.B. Duftöl, PCM, etc.) | 100 | 38,8* |
| - | Resorcin-Lösung | 12,2 | 2,5 |
| - | Ameisensäure Zugabe 1 | 20 | 0,5 |
| Melafine^{®}, Fa. OCI Nitrogen B.V. | Melamin-Suspension Zugabe 1 ¹⁾ | 27 | 1,9 |
| - | Harnstoff-Lösung | 16,6 | 4,7 |
| - | Leitungswasser | 100 | 100,19 |
| - | Natriumsulfat | 100 | 0,5* |
| Speisegelatinepulver, Fa. Ewald-Gelatine GmbH | Schweinehautgelatine | 100 | 6,2* |
| Scogin^{®} MV, Fa. DuPont Nutrition Ireland | Natriumalginat | 100 | 1,4* |
| - | Ameisensäure Zugabe 2 | 20 | 1,4 |
| - | Natronlauge Zugabe 1 | 20 | 0,8 |
| - | Glutaraldehyd Lösung | 50 | 1,9 |
| Melafine^{®}, Fa. OCI Nitrogen B.V. | Melamin-Suspension Zugabe 2 ¹⁾ | 27 | 6,7 |
| - | Natronlauge Zugabe 2 | 20 | 2,2 |

| | | | |
|---|---|---|---|
| 1) Konzentration bezogen auf die angesäuerte Suspension. 2) Bei den gegebenen Mengen für Säuren/Laugen handelt es sich um Richtwerte. Es wird auf den in der Versuchsdurchführung genannten pH-Bereich eingestellt. *Mengen der Komponenten beziehen sich auf die Handelsware und werden eingesetzt wie geliefert. | | | |

### 2.2 Herstellungsverfahren für die nicht erfindungsgemäße Mikrokapsel MK1

Zur Herstellung der Reaktionsmischung 1 wurden Dimension PA140 und Dimension SD mit VE-Wasser Zugabe 1 in einem Becherglas eingewogen und mit einer 4 cm Dissolverscheibe vorgemischt. Das Becherglas wurde im Wasserbad fixiert, und mit der Dissolverscheibe bei 500 U/min bei 30°C verrührt bis eine klare Lösung entstand.

Sobald die Dimension SD / Dimension PA140 Lösung klar war und 30 - 40 °C erreicht hat, wurde die Parfümölmenge langsam zugegeben und dabei die Drehzahl so eingestellt (1100 U/min), dass damit die gewünschte Teilchengröße erzielt wird. Dann wurde der pH-Wert dieser Mischung durch Zugabe der Ameisensäure-Zugabe 1 angesäuert. Es wurde 20 - 30 min emulgiert oder entsprechend verlängert bis die gewünschte Teilchengröße von 20 - 30 µm (Peak-Max) erreicht ist. Die Teilchengröße wurde mittels eines Beckmann-Coulter Gerätes (Laserbeugung, Fraunhofer Methode) bestimmt. Nach Erreichen der Teilchengröße wurde die Drehzahl so reduziert, dass eine schonende Durchmischung gewährleistet war.

Anschließend wurde die Resorcin-Lösung eingerührt und unter schonendem Rühren für 30 - 40 min präformiert. Nach Ablauf der Präformierungszeit wurde die Emulsionstemperatur innerhalb von 15 min auf 50 °C erhöht. Bei Erreichen dieser Temperatur wurde die Mischung über einen Zeitraum von 15 min auf 60°C erhöht und diese Temperatur für weitere 30 min gehalten. Anschließend wurde mit Hilfe von 20%iger Ameisensäure die Melamin-Suspension Zugabe 1 auf einen pH-Wert von 4,5 eingestellt und über einen Zeitraum von 90 min zu der Reaktionsmischung zudosiert. Danach wurde die Temperatur für 30 min gehalten. Nach Ablauf der 30 min wurde innerhalb von 15 min die Temperatur zunächst auf 70 °C erhöht. Anschließend wurde die Temperatur innerhalb von 15 min auf 80 °C erhöht und für 120 min gehalten. Danach wurde die wässrige Harnstoff-Lösung zugegeben, die Wärmequelle abgeschaltet und die Reaktionsmischung 1 auf Raumtemperatur abgekühlt. In einem separaten Becherglas wurde Natriumsulfat in Leitungswasser unter Rühren mit einem Flügelrührer bei 40-50 °C gelöst. Natriumalginat und Schweinehautgelatine werden langsam in das erhitzte Wasser eingestreut. Nachdem alle Feststoffe gelöst waren, wurde Reaktionsmischung 1 unter Rühren zu der hergestellten Gelatine/Natriumalginat Lösung zugegeben. Bei Erreichen einer homogenen Mischung wurde mit der Ameisensäure-Zugabe 2 der pH-Wert durch langsames Zutropfen auf 3,9 eingestellt, danach wurde die Wärmequelle entfernt. Anschließend wurde der Ansatz auf Raumtemperatur abgekühlt. Nach dem Erreichen der Raumtemperatur wurde die Reaktionsmischung mit Eis gekühlt. Bei Erreichen einer Temperatur von 8 °C wurde das Eisbad entfernt und mit Natronlauge Zugabe 1 der pH-Wert auf 4,7 erhöht. Anschließend wurde Glutaraldehyd zugegeben. Dabei wurde darauf geachtet, dass die Temperatur bis zu der Zugabe des Glutaraldehyds 16-20 °C nicht überschreitet.

Im Anschluss wurde die, mittels 20% Ameisensäure auf einen pH-Wert von 4,5 angesäuerte Melamin-Suspension Zugabe 2 langsam zudosiert. Anschließend wurde die Reaktionsmischung auf 60 °C erwärmt und bei Erreichen der Temperatur für 60 min gehalten. Nach dieser Haltezeit wurde die Wärmequelle entfernt und die Mikrokapsel-Dispersion für 14 h schonend gerührt. Nach Ablauf der 14 h wurde die Mikrokapsel-Dispersion mittels Natronlauge Zugabe 2 auf einen pH-Wert von 10,5 eingestellt.

2.3 Herstellungsverfahren für die erfindungsgemäßen Mikrokapsel-Dispersionen Slurry 2 und Slurry 5 Zur Herstellung der Reaktionsmischung 1 wurden Dimension PA140-Zugabe 1 und Dimension SD mit VE-Wasser Zugabe 1 in einem Becherglas eingewogen und mit einer 4 cm Dissolverscheibe vorgemischt. Das Becherglas wurde im Wasserbad fixiert, und mit der Dissolverscheibe bei 500 U/min bei 30 °C verrührt bis eine klare Lösung entstand.

Sobald die Dimension SD/ Dimension PA140 Lösung klar war und 30 - 40 °C erreicht hat, wurde das Kernmaterial langsam zugegeben und dabei die Drehzahl so eingestellt (z.B. 1100 U/min), dass damit die gewünschte Teilchengröße erzielt wird. Anschließend wurde der pH-Wert dieser Mischung durch Zugabe der Ameisensäure-Zugabe 1 angesäuert (pH=3,3-3,5).

Es wurde 20 - 30 min emulgiert oder entsprechend verlängert bis die gewünschte Teilchengröße von 20 - 30 µm (Peak-Max) erreicht ist. Die Teilchengröße wurde mittels eines Beckmann-Coulter Gerätes (Laserbeugung, Fraunhofer Methode) bestimmt. Nach Erreichen der Teilchengröße wurde die Drehzahl so reduziert, dass eine schonende Durchmischung gewährleistet war und die Resorcin-Lösung zugegeben.

Unter schonendem Rühren wurde für 30 - 40 min präformiert. Nach Ablauf der Präformierungszeit wurde die Emulsionstemperatur innerhalb von 15 min auf 50 °C erhöht. Bei Erreichen dieser Temperatur wurde die Mischung über einen Zeitraum von 15 min auf 60°C erhöht und diese Temperatur für weitere 30 min gehalten. Anschließend wurde mit Hilfe von 20%iger Ameisensäure die Melamin-Suspension Zugabe 1 auf einen pH-Wert von 4,5 eingestellt und über einen Zeitraum von 90 min zu der Reaktionsmischung zudosiert.

Danach wurde die Temperatur für 30 min gehalten. Nach Ablauf der 30 min wurde innerhalb von 15 min die Temperatur zunächst auf 70 °C erhöht. Anschließend wurde die Temperatur innerhalb von 15 min auf 80 °C erhöht und für 90 min gehalten.

Danach wurde die wässrige Harnstoff-Lösung zugegeben, die Wärmequelle abgeschaltet und die Reaktionsmischung 1 auf Raumtemperatur abgekühlt. Nachdem Reaktionsmischung 1 Raumtemperatur erreicht hat, wird Dimension PA140-Zugabe 2 hinzugegeben.

In einem separaten Becherglas wurde Natriumsulfat in Leitungswasser unter Rühren mit einem Flügelrührer bei 40-50 °C gelöst. Natriumalginat und Schweinehautgelatine werden langsam in das erhitzte Leitungswasser eingestreut. Nachdem alle Feststoffe gelöst waren, wurde Reaktionsmischung 1 unter Rühren zu der hergestellten Gelatine/Natriumalginat Lösung zugegeben. Bei Erreichen einer homogenen Mischung wurde mit der Ameisensäure-Zugabe 2 der pH-Wert durch langsames Zutropfen auf 3,7 eingestellt, danach wurde die Wärmequelle entfernt und der Ansatz natürlich gegen Raumtemperatur abgekühlt.

Nach dem Erreichen der Raumtemperatur wurde die Reaktionsmischung mit Eis gekühlt. Bei Erreichen einer Temperatur von 8 °C wurde das Eisbad entfernt und mit Natronlauge Zugabe 1 der pH-Wert auf 4,7 erhöht. Anschließend wurde Glutaraldehyd 50% zugegeben. Dabei wurde darauf geachtet, dass die Temperatur bis zu der Zugabe des Glutaraldehyd 50% 16-20 °C nicht überschreitet.

Im Anschluss wurde die, mittels 20% Ameisensäure auf einen pH-Wert von 4,5 angesäuerte Melamin-Suspension Zugabe 2 in einem Zeitrahmen von ca. 2 min. zudosiert. Die Mikrokapsel-Dispersion wurde anschließend bei Raumtemperatur für 14 h schonend gerührt. Nach Ablauf der 14 h wurde die Mikrokapsel-Dispersion mittels Natronlauge Zugabe 2 in einem Zeitraum von ca. 15 min. auf einen pH-Wert von 10,5 eingestellt.

### Beispiel 3 - Stabilität

Die Stabilität der hierin beschriebenen Kapseln beim Einsatz in Kapselslurries sowie handelsüblichen Weichspülerformulierungen wurde untersucht. Als Vergleich wurden marktübliche Melamin-Formaldehyd-Kapseln (MF-Kapseln) sowie die Kapseln gemäß PCT/EP2020/085804 eingesetzt, die keinen Emulsionsstabilisator zwischen innerer und äußerer Schale aufweisen.

Für die Bewertung der Phasenstabilität der Mikrokapsel-Slurry und der Mikrokapseln in einem Endprodukt (Weichspülerformulierung) wurden entsprechende Formulierungen mit Zusatz der verschiedenen Parfümmikrokapseln (0,3 Gew.-% einer Kapselslurry mit gleichen Kapselmengen) formuliert und 4 Wochen bei Raumtemperatur (20-25°C) gelagert. Die Stabilität wurde auf der folgenden Skala bewertet: 1 = keine Phasentrennung, 2 = leichte Phasentrennung, 3 = mittlere Phasentrennung,

**Tabelle 4: Phasenstabilität der Kapseln nach 4 Wochen Lagerung bei Raumtemperatur**

| **Produkt** | **Phasenstabilität nach 4 Wochen bei Raumtemperatur** |
|---|---|
| | |

| **Weichspüler (jeweils 0,3 Gew.-% Kapselslurry)** | |
|---|---|
| MF Kapseln (Vergleich) | 1 |
| Kapseln gem. PCT/EP2020/085804 (Vergleich) | 3 |
| Kapseln mit Emulsionsstabilisator gemäß der Beschreibung (Vergleich) | 2 |
| Kapseln mit Emulsionsstabilisator gemäß der Beschreibung und Emulgator (erfindungsgemäß) | 1 |

| **Kapselslurry** | |
|---|---|
| MF Kapseln (Vergleich) | 3 |
| Kapseln gem. PCT/EP2020/085804 (Vergleich) | 3 |
| Kapseln mit Emulsionsstabilisator gemäß der Beschreibung (Vergleich) | 3 |
| Kapseln mit Emulsionsstabilisator gemäß der Beschreibung und Emulgator (erfindungsgemäß) | 2 |

Die Ergebnisse zeigen, dass sich durch den Einsatz des erfindungsgemäßen Emulgators sowohl in der Kapselslurry als auch dem Endprodukt eine verbesserte Phasenstabilität der Mikrokapseln erzielen lässt.

### Beispiel 4 - Stabilität alternativer Mikrokapselslurrv mit Vorverdünnung

In diesem Beispiel wird auf den Einsatz des Emulgators, also des ethoxylierten, hydrierten Rizinusöls verzichtet.

25 g Mikrokapselslurry wurden in einem 100 mL Becherglas vorgelegt und mit einem Rührfisch versehen. Unter Rühren bei 600 rpm wurde eine Verdünnung mit 25 g Leitungswasser (T = 60°C) durchgeführt. Das Wasser wurde in dünnem Strahl hinzugegeben und anschließend für ca. 1 min homogenisiert. Der verdünnte Ansatz wurde noch warm in die Weichspülerbase eingebracht.

Zum Einbringen der Slurry in die Weichspülerbase wurde jeweils 45,83 g Weichspülerbase in einem 100 mL Becherglas vorgelegt und mit einem Rührfisch versehen. Anschließend wurde eine Pipette mit dem entsprechenden Slurry vorab zur Reduktion des Wägefehlers benetzt und die entsprechende Masse an Slurry (insgesamt 4,17 g) langsam unter Rühren bei 550 rpm eingetropft.

Für die Bewertung der Phasenstabilität der Mikrokapsel-Slurry wurde die vorverdünnte Mikrokapselslurry und parallel eine Mikrokapsel-Slurry in eine Weichspülerformulierung eingebracht erfindungsgemäß formuliert und entweder mit Emulgator versehen, oder vorverdünnt und 4 Wochen bei Raumtemperatur (20-25°C) gelagert. Die Stabilität wurde auf der folgenden Skala bewertet: 1 = keine Phasentrennung, 2 = leichte Phasentrennung, 3 = mittlere Phasentrennung, 4 = starke Phasentrennung und 5 = sehr starke Phasentrennung. Die Ergebnisse sind in der folgenden Tabelle 5 dargestellt.

**Tabelle 5: Phasenstabilität der Kapseln nach Einbringung in das Endprodukt und nach 4 Wochen Lagerung bei Raumtemperatur**

| **Weichspüler (jeweils 1 Gew.% Kapselslurry, bezogen auf otro Slurry)** | **Phasenstabilität nach Einbringen der Kapselslurry** | **Phasenstabilität nach 4 Wochen bei Raumtemperatur** |
|---|---|---|
| Kapseln mit Emulsionsstabilisator gemäß der Beschreibung ohne Emulgator (Vergleich) | 2 | 2 |
| Kapseln mit Emulsionsstabilisator gemäß der Beschreibung ohne Emulgator, Vorverdünnung (1:1) mit 60 °C Wasser | 1 | 2 |
| Kapseln mit Emulsionsstabilisator gemäß der Beschreibung und Emulgator (erfindungsgemäß) | 1 | 1 |

Hinsichtlich weiterer vorteilhafter Ausgestaltungen der erfindungsgemäßen Mittel und Verwendungen wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Mittel und Verwendungen lediglich zur Erörterung der beanspruchten Lehre dienen, diese jedoch nicht auf die Ausführungsbeispiele einschränken.

## Patentansprüche

1. Mittel enthaltend
(1) bioabbaubare Mikrokapseln umfassend ein Kernmaterial und eine Schale, wobei die Schale aus mindestens einer Barriereschicht und einer Stabilitätsschicht besteht, wobei die Barriereschicht das Kernmaterial umgibt, wobei die Stabilitätsschicht mindestens ein Biopolymer umfasst, und auf der äußeren Oberfläche der Barriereschicht angeordnet ist, und wobei optional am Übergang von Barriereschicht zu Stabilitätsschicht ein Emulsionsstabilisator angeordnet ist, und
(2) mindestens einen Emulgator, wobei der Emulgator ausgewählt wird aus der Gruppe der ethoxylierten, hydrierten Rizinusöle, insbesondere solchen mit mittleren EO-Werten im Bereich von 20 bis 60, vorzugsweise 30 bis 50, wobei optional der mindestens eine Emulgator in 0,001 bis 0,25 Gew.-%, vorzugsweise 0,001 bis 0,15 Gew.-%, noch bevorzugter 0,001 bis 0,08 Gew.-%, bezogen auf das Gesamtgewicht des Mittels, enthalten ist, wobei der Emulgator bevorzugt in vorformulierter Form mit den bioabbaubaren Mikrokapseln eingesetzt wird, und wobei das Mittel ein Wasch- oder Reinigungsmittel oder kosmetisches Mittel ist.

2. Mittel nach Anspruch 1, wobei die Mikrokapseln mit dem Emulgator in Kontakt gebracht werden, um eine Vorformulierung enthaltend Mikrokapseln und Emulgator zu erhalten, welche dann zu dem Mittel zugegeben wird.

3. Mittel nach einem der vorherigen Ansprüche, wobei Emulgator und Mikrokapseln als Bestandteil einer Mikrokapsel-Dispersion (Slurry) eingesetzt werden, wobei die Dispersion die Mikrokapseln als feste Phase und Wasser als Hauptbestandteil einer kontinuierlichen Phase umfasst.

4. Mittel nach Anspruch 3, wobei der Emulgator Bestandteil der kontinuierlichen Phase ist, in welcher die Mikrokapseln dispergiert sind.

5. Mittel nach Anspruch 3 oder 4, wobei die kontinuierliche Phase zu mehr als 50 Gew.-%, vorzugsweise zu mehr als 60 Gew.-%, mehr als 70 Gew.-% oder mehr als 80 Gew.-% aus Wasser besteht.

6. Mittel nach einem der Ansprüche 3 bis 5, wobei die Mikrokapsel-Dispersion den mindestens einen Emulgator in einer Menge von bis zu 50 Gew.-%, vorzugsweise bis zu 40 Gew.-%, bevorzugter bis zu 30 Gew.-% oder bis zu 20 Gew.-%, noch bevorzugter bis maximal 10 Gew.-%, besonders bevorzugt in einer Menge von 2 bis 10 Gew.-% enthält.

7. Mittel nach einem der Ansprüche 3 bis 6, wobei der Anteil des Emulgators in der Mikrokapsel-Dispersion 0,5 Gew.-% bis 50 Gew.-%, bevorzugt 1,0 Gew.-% bis 30 Gew.-%, noch bevorzugter 2 Gew.-% bis 20 Gew.-%, besonders bevorzugt 4 Gew.-% bis 8 Gew.-%, bezogen auf das Gesamtgewicht der Mikrokapsel-Dispersion, beträgt.

8. Mittel nach einem der Ansprüche 3 bis 7, wobei die kontinuierliche Phase bezogen auf das Gesamtgewicht der kontinuierlichen Phase 60 bis 95 Gew.-%, vorzugsweise 70 bis 95 Gew.-%, Wasser und 2 bis 40 Gew.-%, vorzugsweise 2 bis 20 Gew.-%, des mindestens einen Emulgators enthält.

9. Mittel nach einem der Ansprüche 3 bis 8, wobei die Mikrokapsel-Dispersion die Mikrokapseln in einer Menge von 1 bis 60 Gew.-%, vorzugsweise 15 bis 50 Gew.-%, insbesondere 25 bis 35 Gew.-% enthält.

10. Mittel nach einem der Ansprüche 3 bis 9, wobei der Anteil der Mikrokapsel-Dispersion mindestens 0,1 Gew.-%, bevorzugt mindestens 0,5 Gew.-%, bezogen auf das Gesamtgewicht des Mittels ist, und vorzugsweise der Anteil des Emulgators im Mittel bezogen auf das Gesamtgewicht des Mittels 0,001 bis 0,25 Gew.-%, vorzugsweise 0,001 bis 0,15 Gew.-%, noch bevorzugter 0,001 bis 0,08 Gew.-% ist, wobei das Mittel bevorzugt flüssig ist.

11. Mittel nach einem der vorherigen Ansprüche, wobei der mindestens eine Emulgator PEG-40 hydrogenated Castor Oil (INCI) umfasst oder daraus besteht.

12. Mittel nach einem der vorherigen Ansprüche, wobei das Mittel einen pH-Wert von weniger als 11, bevorzugt von weniger als 10, bevorzugter weniger als 9, noch bevorzugter weniger als 5 und besonders bevorzugt weniger als 4 und/oder eine Leitfähigkeit von mindestens 0,1 mS/cm, vorzugsweise mindestens 0,2 mS/cm, und höchstens 100 mS/cm, vorzugsweise bis zu 60 mS/cm, besonders bevorzugt 34 mS/cm aufweist.

13. Mittel nach einem der vorherigen Ansprüche, wobei das Kernmaterial der bioabbaubaren Mikrokapseln mindestens eine Parfümzusammensetzung umfasst, wobei die Parfümzusammensetzung bezogen auf das Gesamtgewicht aller in der Parfümzusammensetzung enthaltenen Riechstoffe umfasst:
a) ≤10 Gew.-% an Riechstoffen mit einem CLogP von ≤2,5 und einem Siedepunkt von ≥200°C;
b) ≥15 Gew.-% von mindestens einem Riechstoff mit einem CLogP von ≥4,0 und einem Siedepunkt von ≤275°C; und
c) ≥30 Gew.-% von mindestens einem Riechstoff mit einem Dampfdruck von ≥5 Pa bei 20°C.

14. Mittel nach einem der vorherigen Ansprüche, wobei das Mittel ein flüssiges Mittel, insbesondere ein Weichspüler oder Flüssigwaschmittel ist.

15. Verwendung eines Mittels nach einem der Ansprüche 1 bis 14 als Wasch- oder Reinigungsmittel oder als kosmetisches Mittel, insbesondere als Weichspüler oder Flüssigwaschmittel.
